# EUROPEAN PATENT APPLICATION

(11) **EP 1 563 849 A2**
(43) Date of publication of application: **17.08.2005**
(21) Application number: 05010255.7
(22) Date of filing: 02.12.2002
(51) Int. Cl.: A61K 45/06, A61P 19/00, A61P 31/00, A61P 29/00, A61P 35/00

(54) **Methods and compositions for modulating the immune system and uses thereof**

(30) Priority: 30.11.2001 US 334121 P
(62) Divisional of application: 02794106.1
(71) Applicant: DANA-FARBER CANCER INSTITUTE, Boston, MA 02115 (US)
(72) Inventor: The designation of the inventor has not yet been filed
(74) Representative: Jones Day

(57) **Abstract**

The present invention provides methods of preventing, treating or ameliorating one or more symptoms of disorders in which modulation of a subject's immune system is beneficial utilizing a lymphoid tissue inducing agent and an immuno-modulatory agent. IN particular, the present invention provides methods of preventing, treating or ameliorating a porliferative disorder, an infectious disease, a cardiovascular disease, an autoimmune disorder, or an inflammatory disorder or one or more symptoms thereof comprising administering to a subject in need thereof one or more lymphoid tissue inducing agents and one or more immuno-modulatory agents. The present invention also provides compositions and articles of manufacture for use in preventing, treating or ameliorating one or more symptoms associated with disorders in which modulation of a subject's immune system is beneficial, including, but not limited to proliferative disorders, infectious diseases, cardiovascular diseases, automimmune disorders and inflammatory disorders. The present invention further provides methods for screening and identifying lymphoid tissue inducing agents and/or immunomodulatory agents.

## Description

This application is entitled to and claims the benefit of U.S. provisional patent application Serial No. 60/334,121, filed November 30,2001, which is incorporated herein by reference in its entirety.

### 1. FIELD OF THE INVENTION

The present invention provides methods of preventing, treating or ameliorating one or more symptoms of disorders in which modulation of a subject's immune system is beneficial utilizing a lymphoid tissue inducing agent and an immunomodulatory agent. In particular, the present invention provides methods of preventing, treating or ameliorating a proliferative disorder, an infectious disease, a cardiovascular disease, an autoimmune disorder, or an inflammatory disorder or one or more symptoms thereof comprising administering to a subject in need thereof one or more lymphoid tissue inducing agents and one or immunomodulatory agents. The present invention also provides compositions and articles of manufacture for use in preventing, treating or ameliorating one or more symptoms associated with disorders in which modulation of a subject's immune system is beneficial, including, but not limited to, proliferative disorders, infectious diseases, cardiovascular diseases, autoimmune disorders and inflammatory disorders. The present invention further provides methods for screening and identifying lymphoid tissue inducing agents and/or immunomodulatory agents.

### 2. BACKGROUND OF THE INVENTION

The immune system in higher vertebrates represents the first line of defense against various antigens that can enter the vertebrate body, including microorganisms such as bacteria, fungi and viruses that are the causative agents of a variety of diseases. Moreover, the immune system is also involved in a variety of other diseases or disorders, including autoimmune or immunopathologic diseases, immunodeficiency syndromes, atherosclerosis and various neoplastic diseases, including cancer, the second-leading cause of death in the United States. Although methods are available for treating these diseases, many current therapies provided less than adequate results.

### 2.1. CANCER

Approximately one in every three persons living in the United States will develop some form of cancer during their lifetimes, and nearly half of these patients will eventually die from their disease. It has been estimated that the total direct medical costs of cancer in the United States in the year 2000 were over $100 billion, with an additional $100 billion in indirect costs due to lost productivity - the largest such costs of any major disease. Current methods of cancer therapy can be divided into four categories: surgery, radiation therapy, chemotherapy and "biological" therapy (a broad category that includes gene-, protein- or cell-based treatments).

While surgery remains the most effective form of treatment for cancer, it has numerous shortcomings. Surgery is of limited use in patients whose cancer has metastasized to other areas of the body, and surgical procedures cannot be performed in patients whose tumors are inaccessible or are located in sensitive areas of the body, such as tumors deep in the brain, tumors near the heart, or tumors that are wrapped around major arteries. Radiation therapy can be more advantageous than surgery in such situations, especially when the tumor or metastases are located in the brain. Radiation therapy, however, is only curative in a small number of cancers and the heterogeneity of tumor cells that comprise most tumors typically results in various subpopulations of cells that are non-responsive to the effects of radiation or which develop resistance to ionizing radiation.

As such, chemotherapy is the standard method of treatment for most cancers. Cytotoxic anti-cancer agents primarily kill cancer cells by interfering with cell replication. However, chemotherapy is usually administered systemically, and can adversely affect normal cells since most chemotherapeutic agents are non-discriminatory between normal cells and abnormal cells. This lack of selectivity results in a variety of dose-limiting side effects, including nausea and vomiting, neurotoxicity, hematoxicity, nephrotoxicity, cardiotoxicity and hepatotoxicity. In addition, most cancer cell types eventually become chemo-resistant, thereby hampering the effectiveness of chemotherapy as a long-term method of treatment.

Despite the use of multi-drug regimens, drug resistance is still very difficult to overcome.

Biologic therapies include monoclonal antibodies, non-specific immune boosters that active the innate immune system (*e.g*., bacterial/fungal antigens such as Coley toxins; cytokines such as interferon-α and y), specific immune boosters that activate the acquired or "targeted" immune system (*e.g*., vaccines) and hormones.

The most direct immunotherapeutic approach is to treat patients with monoclonal antibodies against tumor antigens. Herceptin, for instance, targets a growth factor receptor over-expressed in approximately 25% of all breast cancer patients and has shown promising results in controlling tumor growth. Not only is Herceptin thought to block the function of the receptor, but the Herceptin-receptor complex can also serve to recruit natural killer (NK) cells to the tumor site. However, antibody-directed therapy is far from ideal. Although progress is being made to conjugate antibodies with chemotherapeutic agents or radioisotopes to enhance the efficacy of antibody-based immunotherapy, mutation of the tumor and insufficient penetration of the antibody into the tumor mass can lead to inefficient killing of tumor cells.

Cancer vaccines are another promising immunotherapeutic approach. Early attempts used a mixture of a patient's irradiated tumor cells and bacterial adjuvants. Increases in our understanding of the biology of the immune response, however, have led to more sophisticated vaccine treatments. One strategy exploits the use of heat-shock proteins (HSPs) to present antigenic peptides by antigen-presenting cells to effector cells. HSP peptide complexes have been harvested from the tumors of individual patients and used as vaccines. Another strategy attempts to activate T-cell responses by injecting dendritic cells that have been pulsed with tumor-derived antigens.

These recently developed immunotherapeutic approaches are being tested in experimental models and in some cases in human trials. Despite the advantages of these strategies over prior approaches, the adaptability and mutability of tumor cells can be a stumbling block for generation of sufficient immune responses to eliminate all tumor cells. There is therefore a large, unmet need for creative, new immunotherapeutic strategies to achieve complete tumor elimination.

### 2:2. INFECTIOUS DISEASES

Despite large immunization programs, viral infections, such as influenza virus, human immunodeficiency virus ("HIV"), herpes simplex virus ("HSV", type 1 or 2), human papilloma virus ("HPV", type 16 or 18), human cytomegalo virus ("CMV") or human hepatitis virus ("HCV", type C) infections, remain a serious source of morbidity and mortality throughout the world and a significant cause of illness and death among people with immune-deficiency associated with aging or different clinical conditions (see, *e.g.*, Hughes-Fulford et al., 1992, Antimicrob. Agents Chemother. 36: 2253-2258). Although antiviral chemotherapy with compounds such as amantadine and rimantadine have been shown to reduce the duration of symptoms of clinical infections (*i.e.*, influenza infection), major side effects and the emergence of drug-resistant variants have been described (see, *e.g.,* Couch et al., 1997, N. Engl. J. Med. 337: 927-928 and Hughes-Fulford et al., 1992, *supra*). New classes of antiviral agents designed to target particular viral proteins such as influenza neuraminidase are being developed. However, the ability of viruses to mutate the target proteins represents an obstacle for effective treatment with molecules which selectively inhibit the function of specific viral polypeptides. Thus, there is need for new therapeutic strategies to prevent and treat viral infections.

Additionally, there is a need for new therapies for the prevention and treatment of bacterial infections, especially bacterial infections caused by multiple drug resistant bacteria. Currently, bacterial infections are treated with various antibiotics. Although antibiotics have and can be effective in the treatment of various bacterial infections, there are a number of limitations to the effectiveness and safety of antibiotics. For example, some individuals have an allergic reaction to certain antibiotics and other individuals suffer from serious side effects. Moreover, continued use of antibiotics for the treatment of bacterial infections contributes to formation of antibiotic-resistant strains of bacteria.

### 2.3. AUTOIMMUNE DISEASES

Autoimmune diseases are caused when the body's immune system, which is meant to defend the body against bacteria, viruses, and any other foreign product, malfunctions and produces antibodies against healthy tissue, cells and organs. Antibodies, T cells and macrophages provide beneficial protection, but can also produce harmful or deadly immunological responses.

The principle mechanisms by which auto-antibodies can produce an autoimmune disease are complement-dependent lytic destruction of the target cell, opsonization, formation of immune complexes, blockade of receptor sites for physiological ligands, and stimulation of cell surface receptors. The auto-antibody can bind to cell surface receptors and either inhibit or stimulate the specialized function of the cell (Paul, W.E. Ed., 1989, Fundamental Immunology, Raven Press, New York, Chapter 31, p. 839).

Autoimmune diseases can be organ specific or systemic and are provoked by different pathogenic mechanisms. Organ specific autoimmunization is characterized by tolerance and suppression within the T cell compartment, aberrant expression of major-histocompatibility complex (MHC) antigens, antigenic mimicry and allelic variations in MHC genes. Systemic autoimmune diseases involve polyclonal B cell activation and abnormalities of immunoregulatory T cells, T cell receptors and MHC genes. Examples of organ specific autoimmune diseases are diabetes, hyperthyroidism, autoimmune adrenal insufficiency, pure red cell anemia, multiple sclerosis and rheumatic carditis. Representative systemic autoimmune diseases are systemic lupus erythematosus, rheumatoid arthritis, chronic inflammation, Sjogren's syndrome polymyositis, dermatomyositis and scleroderma.

Current treatment of autoimmune diseases involves administering immunosuppressive agents such as cortisone, aspirin derivatives, hydroxychloroquine, methotrexate, azathioprine and cyclophsophamide or combinations thereof. The dilemma faced when administering immunosuppressive agents, however, is the more effectively the autoimmune disease is treated, the more defenseless the patient is left to attack from infections. Thus, there is a need for new therapies for the treatment of autoimmune diseases.

### 2.4. INFLAMMATORY DISORDERS

Inflammation is a process by which the body's white blood cells and chemicals protect our bodies from infection by foreign substances, such as bacteria and viruses. It is usually characterized by pain, swelling, warmth and redness of the affected area. Chemicals known as cytokines and prostaglandins control this process, and are released in an ordered and self-limiting cascade into the blood or affected tissues. This release of chemicals increases the blood flow to the area of injury or infection, and may result in the redness and warmth. Some of the chemicals cause a leak of fluid into the tissues, resulting in swelling. This protective process may stimulate nerves and cause pain. These changes, when occurring for a limited period in the relevant area, work to the benefit of the body.

Rheumatoid arthritis (RA) and juvenile rheumatoid arthritis are types of inflammatory arthritis. Arthritis is a general term that describes inflammation in joints. Some, but not all, types of arthritis are the result of misdirected inflammation. Besides rheumatoid arthritis, other types of arthritis associated with inflammation include the following: psoriatic arthritis, Reiter's syndrome, ankylosing spondylitis arthritis, and gouty arthritis. Rheumatoid arthritis is a type of chronic arthritis that occurs in joints on both sides of the body (such as both hands, wrists or knees). This symmetry helps distinguish rheumatoid arthritis from other types of arthritis. In addition to affecting the joints, rheumatoid arthritis may occasionally affect the skin, eyes, lungs, heart, blood or nerves.

Rheumatoid arthritis affects about 1% of the world's population and essentially disabling. There are approximately 2.9 million incidences of rheumatoid arthritis in the United States. Two to three times more women are affected than men. The typical age that rheumatoid arthritis occurs is between 25 and 50. Juvenile rheumatoid arthritis affects 71,000 young Americans (aged eighteen and under), affecting six times as many girls as boys.

Rheumatoid arthritis is an autoimmune disorder where the body's immune system improperly identifies the synovial membranes that secrete the lubricating fluid in the joints as foreign. Inflammation results, and the cartilage and tissues in and around the joints are damaged or destroyed. In severe cases, this inflammation extends to other joint tissues and surrounding cartilage, where it may erode or destroy bone and cartilage and lead to joint deformities. The body replaces damaged tissue with scar tissue, causing the normal spaces within the joints to become narrow and the bones to fuse together. Rheumatoid arthritis creates stiffness, swelling, fatigue, anemia, weight loss, fever, and often, crippling pain. Some common symptoms of rheumatoid arthritis include joint stiffness upon awakening that lasts an hour or longer; swelling in a specific finger or wrist joints; swelling in the soft tissue around the joints; and swelling on both sides of the joint. Swelling can occur with or without pain, and can worsen progressively or remain the same for years before progressing.

The diagnosis of rheumatoid arthritis is based on a combination of factors, including: the specific location and symmetry of painful joints, the presence of joint stiffness in the morning, the presence of bumps and nodules under the skin (rheumatoid nodules), results of X-ray tests that suggest rheumatoid arthritis, and/or positive results of a blood test called the rheumatoid factor. Many, but not all, people with rheumatoid arthritis have the rheumatoid-factor antibody in their blood. The rheumatoid factor may be present in people who do not have rheumatoid arthritis. Other diseases can also cause the rheumatoid factor to be produced in the blood. That is why the diagnosis of rheumatoid arthritis is based on a combination of several factors and not just the presence of the rheumatoid factor in the blood.

The typical course of the disease is one of persistent but fluctuating joint symptoms, and after about 10 years, 90% of sufferers will show structural damage to bone and cartilage. A small percentage will have a short illness that clears up completely, and another small percentage will have very severe disease with many joint deformities, and occasionally other manifestations of the disease. The inflammatory process causes erosion or destruction of bone and cartilage in the joints. In rheumatoid arthritis, there is an autoimmune cycle of persistent antigen presentation, T-cell stimulation, cytokine secretion, synovial cell activation, and joint destruction. The disease has a major impact on both the individual and society, causing significant pain, impaired function and disability, as well as costing millions of dollars in healthcare expenses and lost wages. (*See,* for example, the NIH website and the NIAID website).

Currently available therapy for arthritis focuses on reducing inflammation of the joints with anti-inflammatory or immunosuppressive medications. The first line of treatment of any arthritis is usually anti-inflammatories, such as aspirin, ibuprofen and Cox-2 inhibitors such as celecoxib and rofecoxib. "Second line drugs" include gold, methotrexate and steroids. Although these are well-established treatments for arthritis, very few patients remit on these lines of treatment alone. Recent advances in the understanding of the pathogenesis of rheumatoid arthritis have led to the use of methotrexate in combination with antibodies to cytokines or recombinant soluble receptors. For example, recombinant soluble receptors for tumor necrosis factor ("TNF")-α have been used in combination with methotrexate in the treatment of arthritis. However, only about 50% of the patients treated with a combination of methotrexate and anti-TNF-α agents such as recombinant soluble receptors for TNF-α show clinically significant improvement. Many patients remain refractory despite treatment. Difficult treatment issues still remain for patients with rheumatoid arthritis. Many current treatments have a high incidence of side effects or cannot completely prevent disease progression. So far, no treatment is ideal, and there is no cure. Thus, there is a need for new therapies for the treatment of inflammatory disorders.

Citation or discussion of a reference herein shall not be construed as an admission that the reference is prior art to the present invention.

### 3. SUMMARY OF THE INVENTION

The present invention encompasses treatment protocols that provide better prophylactic and therapeutic profiles than current single agent therapies for disorders in which modulation of a subject's immune response is beneficial including, but not limited to, proliferative disorders, infectious diseases, cardiovascular diseases, inflammatory disorders and autoimmune disorders. The invention provides combination therapies for the prevention, treatment or amelioration of one or more symptoms associated with a proliferative disorder, an infectious disease, a cardiovascular disease, an inflammatory disorder or an autoimmune disorder in a subject, said combination therapies comprising administering to said subject one or more lymphoid tissue inducers and one or more immunomodulatory agents.

In a specific embodiment, the invention provides a method of preventing, treating or ameliorating one or more symptoms of a proliferative disorder, an infectious disease, a cardiovascular disease, an autoimmune disorder or an inflammatory disorder, said method comprising administering to a subject in need of such treatment a dose of a prophylactically or therapeutically effective amount of one or more lymphoid tissue inducers and a dose of a prophylactically or therapeutically effective amount of one or more immunomodulatory agents. In accordance with this embodiment, the lymphoid tissue inducers may be microtubule stabilizing agents, TNF-inducing agents or small molecules, and the immunomodulating agents may be HSP-inducing agents, ICAM-inducing agents, chemokine receptor-inducing agents, antibodies or bacterial agents. In accordance with the invention, the lymphoid tissue inducers and the immunomodulatory agents utilized in the combination therapies of the invention are different.

In another embodiment, the invention provides a method of preventing, treating or ameliorating one or more symptoms of a proliferative disorder, an infectious disease, a cardiovascular disease, an autoimmune disorder, or an inflammatory disorder, said method comprising administering to a subject in need of such treatment a dose of a prophylactically or therapeutically effective amount of one or more microtubule stabilizing agents and a dose of a prophylactically or therapeutically effective amount of one or more immunomodulatory agents. In accordance with this embodiment, the microtubule stabilizing agents may be a taxane (e.g., taxotere or paclitaxel), an epothilone, a discodermolide, an eleutherobin, a taccalonide, or a sarcodictyin. In a specific embodiment, the immunomodulatory agents administered to a subject with a proliferative disorder in conjunction with the microtubule stabilizing agent taxol do not include one or more of the following immunomodulatory agents: 5-FU, acisplatin, leucovorin, mitoxantrone, doxorubicin, cyclophosphamide, carboplatin, an anthracyline, gemcitabine, epirubicin, capecitabine, isofamide, edatrexate, vinorelbine, verapramil, etoposide, hydroxyurea, folinic acid, taxotere, estramustine, GM-CSF, TNF-alpha induction, raltitrexid, phrazoloacridine, amifostine, PS-341, vinfluinine, squalamine, melphalan, cryptophycens, polyamines, herceptin, TFN-alpha, glutamine, geldenamycin, PDGF antagonists, oreotide, EGF, herbimycin A, genistein, sodium azide, dexamethascne, diphenhydramene, ranitidini, and non-steriodal anti-inflammatory agents. In another embodiment, the immunomodulatory agents administered to a subject with a proliferative disorder in conjunction with the microtubule stabilizing agent taxotere, do not include one or more of the following immunomodulatory agents: 5-FU, doxorubicin, capecitabine, and cyt P450 Cyp1 inhibitor.

In another embodiment, the invention provides a method of preventing, treating or ameliorating one or more symptoms of a proliferative disorder, an infectious disease, a cardiovascular disease, an autoimmune disorder or an inflammatory disorder, said method comprising administering to a subject in need of such treatment a dose of a prophylactically or therapeutically effective amount of one or more small molecules or one or more TNF-inducing agents as lymphoid tissue inducing agents and a dose of a prophylactically or therapeutically amount of one or more immunomodulatory agents.

The combination therapies of the invention enable lower dosages of lymphoid tissue inducers to be utilized in conjunction with immunomodulatory agents for the prevention or treatment of a disorder described herein and/or less frequent administration of such agents to a subject with a disorder described herein to achieve a prophylactic or therapeutic effect. The combination therapies of the invention reduce or avoid unwanted or adverse side effects associated with the administration of current single agent therapies and/or existing combination therapies for the disorders described herein, which in turn improve patient compliance with the treatment protocol. Further, the combination therapies of the invention reduce the frequency of administration of dosages of one or more lymphoid tissue inducers, or the frequency of administration of dosages of one or more immunomodulatory agents to a subject with a disorder described herein to improve the quality of life of said subject.

The lymphoid tissue inducers and immunomodulatory agents of the combination therapies of the present invention can be administered concomitantly or sequentially to a subject. The lymphoid tissue inducers and immunomodulatory agents of the combination therapies of the present invention can also be cyclically administered. Cycling therapy involves the administration of a first prophylactic or therapeutic agent (*e.g.*, a lymphoid tissue inducer) for a period of time, followed by the administration of a second prophylactic or therapeutic agent (*e.g.*, an immunomodulatory agent) for a period of time and repeating this sequential administration, *i.e.,* the cycle, in order to reduce the development of resistance to one of the agents, to avoid or reduce the side effects of one of the agents, and/or to improve the efficacy of the treatment.

The lymphoid tissue inducers and immunomodulatory agents of the combination therapies of the invention can be administered to a subject concurrently. The term "concurrently" is not limited to the administration of lymphoid tissue inducers and immunomodulatory agents at exactly the same time, but rather it is meant that a lymphoid tissue inducer and an immunomodulatory agent are administered to a subject in a sequence and within a time interval such that the lymphoid tissue inducer can act together with the immunomodulatory agent to provide an increased benefit than if they were administered otherwise. For example, a lymphoid tissue inducer and an immunomodulatory agent may be administered at the same time or sequentially in any order at different points in time; however, if not administered at the same time, they should be administered sufficiently close in time so as to provide the desired therapeutic or prophylactic effect. A lymphoid tissue inducer and an immunomodulatory agent can be administered separately, in any appropriate form and by any suitable route. In various embodiments, a lymphoid tissue inducer and an immunomodulatory agent are administered less than 15 minutes, less than 30 minutes, less than 1 hour apart, at about 1 hour apart, at about 1 hour to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, 24 hours apart, 48 hours apart, 72 hours apart, or 1 week apart. In a preferred embodiment, a lymphoid tissue inducer and two or more immunomodulatory agents are administered within the same patient visit. In another preferred embodiment, two or more lymphoid tissue inducers and one, two or more immunomodulatory agents are administered to a patient within the same patient visit.

The lymphoid tissue inducers and immunomodulatory agents of the combination therapies can be administered to a subject in the same pharmaceutical composition. Alternatively, the lymphoid tissue inducers and immunomodulatory agents of the combination therapies can be administered concurrently to a subject in separate pharmaceutical compositions. The lymphoid tissue inducers and immunomodulatory agents may be administered to a subject by the same or different routes of administration.

The present invention provides pharmaceutical compositions comprising one or more lymphoid tissue inducers and a pharmaceutically acceptable carrier. The present invention also provides pharmaceutical compositions comprising one or more immunomodulatory agents and a pharmaceutically acceptable carrier. The present invention further provides pharmaceutical compositions comprising one or more lymphoid tissue inducers, one or more immunomodulatory agents, and a pharmaceutically acceptable carrier. In a specific embodiment, the invention provides a pharmaceutical composition comprising a therapeutically effective amount of one or more microtubule stabilizing agents, a therapeutically effective amount of one or more HSP-inducing agents, and a pharmaceutically acceptable carrier. In another embodiment, the invention provides a pharmaceutical composition comprising a therapeutically effective amount of one or more of microtubule stabilizing agents, a therapeutically effective amount of one or more chemokine receptor-inducing agents, and a pharmaceutically acceptable carrier. In yet another embodiment, the invention provides a pharmaceutical composition comprising a therapeutically effective amount of one or more microtubule stabilizing agents, a therapeutically effective amount of one or more ICAM-inducing agents, and a pharmaceutical acceptable carrier.

The pharmaceutical compositions of the invention may be used in accordance with the methods of the invention for the prevention, treatment or amelioration of a disorder described herein or one or more symptoms thereof. Preferably, the pharmaceutical compositions of the invention are sterile and in suitable form for a particular method of administration to a subject with a proliferative disorder, an infectious disease, a cardiovascular disease, an autoimmune disorder, or an inflammatory disorder.

The invention encompasses sustained release formulations for the administration of one or more lymphoid tissue inducers and/or one or more immunomodulatory agents to a subject. The sustained release formulations reduce the dosage and/or frequency of administration of such agents to a subject.

The compositions and methods described herein are useful for the prevention, treatment or amelioration of proliferative disorders including, but not limited to, lung cancer, including small cell and non-small cell lung cancer; gastrointestinal cancer, including esophogeal cancer, gastric cancer, pancreatic cancer, hepatocellular cancer, colorectal cancer and anal carcinoma; genitourinary cancer, including prostate cancer, testicular cancer, bladder cancer, renal cell cancer, ovarian cancer, endometrial cancer and cervical cancer; breast cancer, neoplasms of endocrine organs, including the thyroid and parathyroid, tumors of adrenal medulla, such as pheochromocytoma and neuroblastoma; and multiple endocrine neoplasia (such as Types 1-3); hematologic cancers, including leukemia, multiple myeloma, Hodgkins disease and non-Hodgkins lymphoma; brain cancers, including central nervous system cancers such as craniopharyngeoma, pituitary neoplasms, astrocytomas, meningiomas, and spinal cord tumors; and peripheral nervous system cancers, including schwannomas and acoustic neuromas; skin cancer, including melanoma, basal cell carcinoma and squamous cell carcinoma; cardiac tumors, such as atrial myxomas; and psoriasis. The compositions and methods described herein are useful for the prevention, treatment or amelioration of infectious diseases including, but not limited to, viral, bacterial, fungal and parasitic diseases.

The compositions and methods described herein are useful for the prevention, treatment or amelioration of cardiovascular diseases including, but not limited to, athlerosclerosis, stroke, cerebral infarction, endothelium dysfunctions (in particular, those dysfunctions affecting blood vessel elasticity) ischemic heart disease (*e.g.,* angina pectoris, myocardial infarction, and chronic ischemic heart disease), hypertensive heart disease, pulmonary heart disease, coronary heart disease, valvular heart disease (*e.g.*, rheumatic fever and rheumatic heart disease, endocarditis, mitral valve prolapse, restenosis and aortic valve stenosis), congenital heart disease (*e.g*., valvular and vascular obstructive lesions, atrial or ventricular septal defect, and patent ductus arteriosus), and myocardial disease (*e.g.,* myocarditis, congestive cardiomyopathy, and hypertrophic cariomyopathy).

The compositions and methods described herein are useful for the prevention, treatment or amelioration of autoimmune disorders including, but not limited to, alopecia areata, ankylosing spondylitis, antiphospholipid syndrome, autoimmune Addison's disease, autoimmune diseases of the adrenal gland, autoimmune hemolytic anemia, autoimmune hepatitis, autoimmune oophoritis and orchitis, autoimmune thrombocytopenia, Behcet's disease, bullous pemphigoid, cardiomyopathy, celiac sprue-dermatitis, chronic fatigue immune dysfunction syndrome (CFIDS), chronic inflammatory demyelinating polyneuropathy, Churg-Strauss syndrome, cicatrical pemphigoid, CREST syndrome, cold agglutinin disease, Crohn's disease, discoid lupus, essential mixed cryoglobulinemia, fibromyalgia-fibromyositis, glomerulonephritis, Graves' disease, Guillain-Barre, Hashimoto's thyroiditis, idiopathic pulmonary fibrosis, idiopathic thrombocytopenia purpura (ITP), IgA neuropathy, juvenile arthritis, lichen planus, Ménière's disease, mixed connective tissue disease, multiple sclerosis, type 1 or immune-mediated diabetes mellitus, myasthenia gravis, pemphigus vulgaris, pernicious anemia, polyarteritis nodosa, polychrondritis, polyglandular syndromes, polymyalgia rheumatica, polymyositis and dermatomyositis, primary agammaglobulinemia, primary biliary cirrhosis, psoriasis, psoriatic arthritis, Raynauld's phenomenon, Reiter's syndrome, Rheumatoid arthritis, sarcoidosis, scleroderma, progressive systemic sclerosis, Sjögren's syndrome, Good pasture's syndrome, stiff-man syndrome, systemic lupus erythematosus, lupus erythematosus, takayasu arteritis, temporal arteristis/giant cell arteritis, ulcerative colitis, uveitis, vasculitides such as dermatitis herpetiformis vasculitis, vitiligo, and Wegener's granulomatosis.

The compositions and methods described herein are useful for the prevention, treatment or amelioration of inflammatory disorders including, but are not limited to, asthma, encephilitis, inflammatory bowel disease (*e.g.*, Crohn's disease and ulcerative colitis), chronic obstructive pulmonary disease (COPD), inflammatory osteolysis, allergic disorders, septic shock, pulmonary fibrosis (*e.g.*, idiopathic pulmonary fibrosis), inflammatory vaculitides (*e.g.*, polyarteritis nodosa, Wegner's granulomatosis, Takayasu's arteritis, temporal arteritis, and lymphomatoid granulomatosus), post-traumatic vascular angioplasty (*e.g.*, restenosis after angioplasty), undifferentitated spondyloarthropathy, undifferentiated arthropathy, arthritis, inflammatory osteolysis, chronic hepatitis, and chronic inflammation resulting from chronic viral or bacteria infections. In particular, the composition and methods described herein are useful for the prevention, treatment or amelioration of inflammatory disorders characterized by increased T cell activation and/or abnormal antigen presentation. The compositions and methods described herein can also be applied to the prevention, treatment or amelioration of one or more symptoms associated with inflammatory osteolysis, other disorders characterized by abnormal bone reabsorption, or disorder characterized by bone loss (*e.g.*, osteoporosis).

The present invention provides article of manufactures comprising packaging material and a pharmaceutical composition of the invention in suitable form for administration to a subject contained within said packaging material. In particular, the present invention provides article of manufactures comprising packaging material and a pharmaceutical composition of the invention in suitable form for administration to a subject contained within said packaging material, wherein said pharmaceutical composition comprises one or more lymphoid tissue inducers, one or more immunodmodulatory agents, and a pharmaceutically acceptable carrier. The articles of manufacture of the invention may include instructions regarding the use or administration of a pharmaceutical composition, or other informational material that advises the physician, technician or patient on how to appropriately prevent or treat the disease or disorder in question.

### 3.1. TERMINOLOGY

As used herein, the term "activated immune cells" and analogous terms refer to immune cells, including, but not limited to lymphoid cells (*e.g.*, T-cells, NK cells and B cells), myeloid cells (*e.g.*, macrophages, monocytes, eosinophils, neutrophils, basophils, mast cells, granulocytes and platelets), dendritic cells, and antigen-presenting cells that express specific markers (antigens) or produce specific cytokines. The expression of activation markers and cytokines can be determined by a variety of methods known to those of skill in the art, including, but not limited to, immunofluorescence, fluorescence activated cell sorter ("FACS"), Western blot analysis, Northern blot analysis and RT-PCR.

As used herein, the terms "adjunctive" and "conjunction" are used interchangeably with "in combination" or "combinatorial".

As used herein, the terms "agonistic antibody", "agonistic antibodies" and analogous terms refer to antibodies that immunospecifically bind to an antigen expressed by an immune cell (*e.g.,* a cytokine receptor or a co-stimulatory molecule) and induce the activation of a signal transduction pathway associated with said antigen. Preferably, agonistic antibodies immunospecifically bind to an antigen selectively expressed by activated immune cells, and augment the activation of the immune cells.

As used herein, the term "aliphatic group" is a straight chain, branched chain or cyclic non aromatic hydrocarbon which is completely saturated or which contains one or more units of unsaturation. Typically, a straight chain or branched chain aliphatic group has from 1 to about 20 carbon atoms, preferably from 1 to about 10 carbon atoms, and a cyclic aliphatic group has from 3 to about 10 carbon atoms, preferably from 3 to about 8 carbon atoms. An aliphatic group is preferably a straight chained or branched alkyl group, e.g, methyl, ethyl, *n*-propyl, *iso*-propyl, *n*-butyl, *sec*-butyl, *tert-butyl,* pentyl, hexyl, pentyl or octyl, or a cycloalkyl group with 3 to about 8 carbon atoms. AC1-C20 straight chained or branched alkyl group or a C3-C8 cyclic alkyl group is also referred to as a "lower alkyl" group.

As used herein, the term "analog" in the context of a proteinaceous agent (*e.g.*, a peptide, a polypeptide, a protein, a fusion protein and an antibody) refers to a proteinaceous agent that possesses a similar or identical function as a second proteinaceous agent but does not necessarily comprise a similar or identical amino acid sequence of the second proteinaceous agent, or possesses a similar or identical structure of the second proteinaceous agent. A proteinaceous agent that has a similar amino acid sequence refers to a second proteinaceous agent that satisfies at least one of the following: (a) a proteinaceous agent having an amino acid sequence that is at least 30%, at least 35%, at least 40%, at least 45%, at least 50%, at least 55%, at least 60%, at least 65%, at least 70%, at least 75%, at least 80%, at least 85%, at least 90%, at least 95% or at least 99% identical to the amino acid sequence of a second proteinaceous agent; (b) a proteinaceous agent encoded by a nucleotide sequence that hybridizes under stringent conditions to a nucleotide sequence encoding a second proteinaceous agent of at least 5 contiguous amino acid residues, at least 10 contiguous amino acid residues, at least 15 contiguous amino acid residues, at least 20 contiguous amino acid residues, at least 25 contiguous amino acid residues, at least 40 contiguous amino acid residues, at least 50 contiguous amino acid residues, at least 60 contiguous amino residues, at least 70 contiguous amino acid residues, at least 80 contiguous amino acid residues, at least 90 contiguous amino acid residues, at least 100 contiguous amino acid residues, at least 125 contiguous amino acid residues, or at least 150 contiguous amino acid residues; and (c) a proteinaceous agent encoded by a nucleotide sequence that is at least 30%, at least 35%, at least 40%, at least 45%, at least 50%, at least 55%, at least 60%, at least 65%, at least 70%, at least 75%, at least 80%, at least 85%, at least 90%, at least 95% or at least 99% identical to the nucleotide sequence encoding a second proteinaceous agent. A proteinaceous agent with similar structure to a second proteinaceous agent refers to a proteinaceous agent that has a similar secondary, tertiary or quaternary structure to the second proteinaceous agent. The structure of a proteinaceous agent can be determined by methods known to those skilled in the art, including but not limited to, peptide sequencing, X-ray crystallography, nuclear magnetic resonance, circular dichroism, and crystallographic electron microscopy.

To determine the percent identity of two amino acid sequences or of two nucleic acid sequences, the sequences are aligned for optimal comparison purposes (*e.g.*, gaps can be introduced in the sequence of a first amino acid or nucleic acid sequence for optimal alignment with a second amino acid or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences (*i.e.,* % identity = number of identical overlapping positions/total number of positions x 100%). In one embodiment, the two sequences are the same length.

The determination of percent identity between two sequences can also be accomplished using a mathematical algorithm. A preferred, non-limiting example of a mathematical algorithm utilized for the comparison of two sequences is the algorithm of Karlin and Altschul, 1990, Proc. Natl. Acad. Sci. U.S.A. 87:2264-2268, modified as in Karlin and Altschul, 1993, Proc. Natl. Acad. Sci. U.S.A. 90:5873-5877. Such an algorithm is incorporated into the NBLAST and XBLAST programs of Altschul et al., 1990, J. Mol. Biol. 215:403. BLAST nucleotide searches can be performed with the NBLAST nucleotide program parameters set, *e.g.*, for score=100, wordlength=12 to obtain nucleotide sequences homologous to a nucleic acid molecules of the present invention. BLAST protein searches can be performed with the XBLAST program parameters set, *e.g.,* to score-50, wordlength=3 to obtain amino acid sequences homologous to a protein molecule of the present invention. To obtain gapped alignments for comparison purposes, Gapped BLAST can be utilized as described in Altschul et al., 1997, Nucleic Acids Res. 25:3389-3402. Alternatively, PSI-BLAST can be used to perform an iterated search which detects distant relationships between molecules (*Id*.). When utilizing BLAST, Gapped BLAST, and PSI-Blast programs, the default parameters of the respective programs (*e.g.*, of XBLAST and NBLAST) can be used (see, *e.g.,* the NCBI website). Another preferred, non-limiting example of a mathematical algorithm utilized for the comparison of sequences is the algorithm of Myers and Miller, 1988, CABIOS 4:11-17. Such an algorithm is incorporated in the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used.

The percent identity between two sequences can be determined using techniques similar to those described above, with or without allowing gaps. In calculating percent identity, typically only exact matches are counted.

As used herein, the term "analog" in the context of a non-proteinaceous analog refers to a second organic or inorganic molecule which possess a similar or identical function as a first organic or inorganic molecule and is structurally similar to the first organic or inorganic molecule.

As used herein, the terms "antibody" and "antibodies" refer to monoclonal antibodies, multispecific antibodies, human antibodies, humanized antibodies, camelised antibodies, chimeric antibodies, single domain antibodies, single-chain Fvs (scFv), single chain antibodies, Fab fragments, F(ab¹) fragments, disulfide-linked Fvs (sdFv), and anti-idiotypic (anti-Id) antibodies, and epitope-binding fragments of any of the above. In particular, antibodies include immunoglobulin molecules and immunologically active fragments of immunoglobulin molecules, *i.e.*, molecules that contain an antigen binding site. Immunoglobulin molecules can be of any type (*e.g.,* IgG, IgE, IgM, IgD, IgA and IgY), class (*e.g.*, IgG₁, IgG₂, IgG₃, IgG₄, IgA₁ and IgA₂) or subclass.

As used herein the term "aromatic group" refers to carbocyclic aromatic groups such as phenyl, naphthyl, and anthracyl, and heteroaryl groups such as imidazolyl, thienyl, furanyl, pyridyl, pyrimidy, pyranyl, pyrazolyl, pyrroyl, pyrazinyl, thiazole, oxazolyl, and tetrazole.

Aromatic groups also include fused polycyclic aromatic ring systems in which a carbocyclic aromatic ring or heteroaryl ring is fused to one or more other heteroaryl rings. Examples include benzothienyl, benzofuranyl, indolyl, quinolinyl, benzothiazole, benzooxazole, benzimidazole, quinolinyl, isoquinolinyl and isoindolyl.

The term "aryl" as used herein, refers to an aromatic carbocycle having from 5 to 10 ring carbon atoms or an aromatic heterocyclic ring. An aromatic heterocyclic ring is to an aromatic carbocycle having from 5 to 10 ring carbon atoms in which one to four of the carbon ring atoms are replaced by N, O or S atoms.

As used herein, the term "benzyl" refers to a -CH₂-phenyl group.

As used herein, the term "cytokine receptor modulator" refers to an agent which modulates the phosphorylation of a cytokine receptor, the activation of a signal transduction pathway associated with a cytokine receptor, and/or the expression of a particular protein such as a cytokine induced by the activation of a signal transduction pathway assoicated with a cytokine receptor. Such an agent may directly or indirectly modulate the phosphorylation of a cytokine receptor, the activation of a signal transduction pathway associated with a cytokine receptor, and/or the expression of a particular protein such as a cytokine induced by the activation of a signal transduction pathway assoicated with a cytokine receptor. Examples of cytokine receptor modulators include, but are not limited to, proteinaneous agents (*e.g.*, cytokines, peptide mimetics, and antibodies), small molecules, organic compounds, inorganic compounds, and nucleic acid molecules *(e.g.,* nucleic acid molecules encoding proteins, polypeptides, peptides, and antibodies) that have these activities. In certain embodiments, the cytokine receptor modulator is a protein, polypeptide, or peptide which immunospecifically binds to or associates with one or more subunits of a cytokine receptor and induces the activation of a signal transduction pathway associated with the cytokine receptor. In other embodiments, the cytokine receptor modulator is a nucleic acid molecule comprising a nucleotide sequence encoding a protein, polypeptide or peptide that immunospecifically binds to or associates with one or more subunits of a cytokine receptor and induces the activation of a signal transduction pathway associated with the cytokine receptor. In certain other embodiments, the cytokine receptor modulator is a fusion protein or a nucleic acid molecule comprising a nucleotide sequence encoding a fusion protein, said fusion protein comprising a protein, polypeptide or peptide that immunospecifically binds to or associates with one or more subunits of a cytokine receptor and induces the activation of a signal transduction pathway associated with the cytokine receptor fused to a heterologous protein, polypeptide or peptide.

In a preferred embodiment, the cytokine receptor modulator is a cytokine, a nucleic acid molecule comprising a nucleotide sequence encoding a cytokine, an agonistic antibody which immunospecifically binds to one or more subunits of a cytokine receptor, or a nucleic acid molecule comprising a nucleotide sequence encoding an agonistic antibody that immunospecifically binds to one or more subunits of a cytokine receptor. Examples of cytokines include, but are not limited to, interferon ("IFN")-alpha, IFN-beta, IFN-gamma, tumor necrosis factor ("TNF")-alpha, Fit3 ligand, interleukin ("IL")-1, IL-2, IL-3, IL-4, IL-5, IL-6, IL-7, IL-8, IL-9, IL-10, IL-12, IL-15, IL-18, colony-stimulating factor ("CSF")-1, granulocyte colony-stimulating factor ("G-CSF"), macrophage colony-stimulating factor ("M-CSF"), granulocyte macrophage colony-stimulating factor ("GM-CSF") and chemokines such as macrophage inflammatory protein ("MIP")-1, gamma interferon inducible protein ("IP-10") and monokine induced by IFN-γ ("MIG").

As used herein, the term "derivative" in the context of a proteinaceous agent refers to a proteinaceous agent that comprises an amino acid sequence which has been altered by the introduction of amino acid residue substitutions, deletions or additions. The term "derivative" as used herein also refers to a proteinaceous agent which has been modified, *i.e,* by the covalent attachment of any type of molecule to the proteinaceous agent. For example, but not by way of limitation, an antibody may be modified, *e.g.*, by glycosylation, acetylation, pegylation, phosphorylation, amidation, derivatization by known protecting/blocking groups, proteolytic cleavage, linkage to a cellular ligand or other protein, etc. A derivative of a proteinaceous agent may be produced by chemical modifications using techniques known to those of skill in the art, including, but not limited to specific chemical cleavage, acetylation, formylation, metabolic synthesis of tunicamycin, etc. Further, a derivative of a proteinaceous agent may contain one or more non-classical amino acids. In a preferred embodiment, a proteinaceous derivative possesses a similar or identical function as the proteinaceous agent from which it was derived.

As used herein, the term "derivative" in the context of a non-proteinaceous agent refers to a second organic or inorganic molecule that is formed based upon the structure of a first organic or inorganic molecule. A derivative of an organic molecule includes, but is not limited to, a molecule modified, *e.g.*, by the addition or deletion of a hydroxyl, methyl, ethyl, carboxyl or amine group. An organic molecule may also be esterified, alkylated and/or phosphorylated. In a preferred embodiment, a non-proteinaceous derivative possesses a similar or identical function as the organic or inorganic molecule from which it was derived.

As used herein, the terms "disorder" and "disease" are used interchangeably to refer to a condition in a subject. In particular, the term "autoimmune disease" is used interchangeably with the term "autoimmune disorder" to refer to a condition in a subject characterized by cellular, tissue and/or organ injury caused by an immunologic reaction of the subject to its own cells, tissues and/or organs. The term "inflammatory disease" is used interchangeably with the term "inflammatory disorder" to refer to a condition in a subject characterized by inflammation, preferably chronic inflammation. Autoimmune disorders may or may not be associated with inflammation. Moreover, inflammation may or may not be caused by an autoimmune disorder. Thus, certain disorders may be characterized as both autoimmune and inflammatory disorders.

As used herein the term "docetaxel" refers to the taxane commonly referred to as "taxotere." As such, the terms "docetaxel" and "taxotere" may be used interchangeably.

As used herein, the term "epitopes" refers to fragments of a polypeptide or protein having antigenic or immunogenic activity in an animal, preferably in a mammal, and most preferably in a human. An epitope having immunogenic activity is a fragment of a polypeptide or protein that elicits an antibody response in an animal. An epitope having antigenic activity is a fragment of a polypeptide or protein to which an antibody immunospecifically binds as determined by any method well-known to one of skill in the art, for example by immunoassays. Antigenic epitopes need not necessarily be immunogenic.

As used herein, the term "fragment" refers to a peptide or polypeptide comprising an amino acid sequence of at least 5 contiguous amino acid residues, at least 10 contiguous amino acid residues, at least 15 contiguous amino acid residues, at least 20 contiguous amino acid residues, at least 25 contiguous amino acid residues, at least 40 contiguous amino acid residues, at least 50 contiguous amino acid residues, at least 60 contiguous amino residues, at least 70 contiguous amino acid residues, at least contiguous 80 amino acid residues, at least contiguous 90 amino acid residues, at least contiguous 100 amino acid residues, at least contiguous 125 amino acid residues, at least 150 contiguous amino acid residues, at least contiguous 175 amino acid residues, at least contiguous 200 amino acid residues, or at least contiguous 250 amino acid residues of the amino acid sequence of another polypeptide. In a specific embodiment, a fragment of a polypeptide retains at least one function of the polypeptide.

As used herein, the term "functional fragment" refers to a peptide or polypeptide comprising an amino acid sequence of at least 5 contiguous amino acid residues, at least 10 contiguous amino acid residues, at least 15 contiguous amino acid residues, at least 20 contiguous amino acid residues, at least 25 contiguous amino acid residues, at least 40 contiguous amino acid residues, at least 50 contiguous amino acid residues, at least 60 contiguous amino residues, at least 70 contiguous amino acid residues, at least contiguous 80 amino acid residues, at least contiguous 90 amino acid residues, at least contiguous 100 amino acid residues, at least contiguous 125 amino acid residues, at least 150 contiguous amino acid residues, at least contiguous 175 amino acid residues, at least contiguous 200 amino acid residues, or at least contiguous 250 amino acid residues of the amino acid sequence of second, different polypeptide, wherein said peptide or polypeptide retains at least one function of the second, different polypeptide.

As used herein, the term "fusion protein" refers to a polypeptide that comprises an amino acid sequence of a first protein or functional fragment, analog or derivative thereof, and an amino acid sequence of a heterologous protein (*i.e.,* a second protein or functional fragment, analog or derivative thereof different than the first protein or functional fragment, analog or derivative thereof). In one embodiment, a fusion protein comprises a prophylactic or therapeutic agent fused to a heterologous protein, polypeptide or peptide. In accordance with this embodiment, the heterologous protein, polypeptide or peptide may or may not be a different type of prophylactic or therapeutic agent. For example, two different proteins, polypeptides or peptides with immunomodulatory activity may be fused together to form a fusion protein.

As used herein, the term "host cell" refers to the particular subject cell transfected with a nucleic acid molecule and the progeny or potential progeny of such a cell. Progeny of such a cell may not be identical to the parent cell transfected with the nucleic acid molecule due to mutations or environmental influences that may occur in succeeding generations or integration of the nucleic acid molecule into the host cell genome.

As used herein, the term "hybridizes under stringent conditions" describes conditions for hybridization and washing under which nucleotide sequences at least 30% (preferably, at least 35%, at least 40%, at least 45%, at least 50%, at least 55%, at least 60%, at least 65%, at least 70%, at least 75%, at least 80%, at least 85%, at least 90% or at least 95%) identical to each other typically remain hybridized to each other. Such stringent conditions are known to those skilled in the art and can be found in *Current Protocols in Molecular Biology,* John Wiley & Sons, N.Y. (1989), 6.3.1-6.3.6. In one, non-limiting example stringent hybridization conditions are hybridization at 6X sodium chloride/sodium citrate (SSC) at about 45° C, followed by one or more washes in 0.1 XSSC, 0.2% SDS at about 68 °C. In a preferred, non-limiting example stringent hybridization conditions are hybridization in 6XSSC at about 45° C, followed by one or more washes in 0.2 X SSC, 0.1 % SDS at 50-65 °C (*i.e.,* one or more washes at 50°C, 55°C, 60°C or 65°C). It is understood that the nucleic acids of the invention do not include nucleic acid molecules that hybridize under these conditions solely to a nucleotide sequence consisting of only A or T nucleotides.

As used herein, the terms "immunomodulatory agent," 'immune system modulator" and variations thereof including, but not limited to, immunomodulatory agents, immunomodulants or immunomodulatory drugs, refer to an agent that modulates one or more aspects of a subject's immune response. Aspects of the immune response include, but are not limited to, the inflammatory response, the complement cascade, the humoral immune response, and the cellular immune response. In a specific embodiment, an immunomodulatory agent is an agent that shifts one aspect of a subject's immune response, *e.g.*, the agent shifts the immune response from a Th1 to a Th2 response. In other embodiments, an immunomodulatory agent modulates more than one aspect of the immune response. In one embodiment, an immunomodulatory agent is an immunosuppressant agent. In a preferred embodiment, an immunomodulatory agent is an immunostimulatory agent or immune system enhancer. In certain circumstances, a lymphoid tissue inducing agent may also be classified as an immunomodulatory agent. Thus, in accordance with the invention; the immunomodulatory agent(s) used in a combination therapy of the invention is a different agent than the lymphoid tissue inducing agent used in that combination therapy.

As used herein, the terms "immunostimulatory agent" and "immune system enhancer" refer to an agent that enhances, activates or otherwise increases a subject's immune response. In particular, an immune system enhancer is an agent that enhances, activates or otherwise increases a subject's humoral and/or cellular immune response. An immune system enhancer is an agent that activates one or more biological activities *(e.g.,* the proliferation, differentiation, priming, effector function, production of cytokines or expression of antigens) of one or more immune cells (*e.g.,* T cells (*e.g.,* T helper cells and cytotoxic T-cells), natural killer ("NK") cells, and antigen-presenting cells (*e.g.*, macrophages, dendritic cells, and B cells)). For example, an immunostimulatory molecule may stimulate or increase the synthesis of antibody molecule, or increase the expression or release of cytokines (*e.g.*, IL-2), or increase the expression of cytokine receptors, or increase the proliferation of an immune cell. In a specific embodiment, an immunostimulatory agent activates a biological activity of an immune cell 1-5 fold, 5-10 fold, 10-20 fold or more than 20 fold as compared to the biological activity of the immune cells in the absence of the immunostimulatory agent. The immune cells activated by an immunostimulatory agent may also migrate to a particular site of a disease or to the lymphoid tissue induced by a lymphoid tissue inducing agent.

As used herein, the term "immunosuppressant agent" refers to an agent that inhibits or reduces one or more biological activities of the immune system. An immunosuppressant agent is an agent that inhibits or reduces one or more biological activities (*e.g.,* the proliferation, differentiation, priming, effector function, production of cytokines or expression of antigens) of one or more immune cells (*e.g.,* T cells (*e.g.,* T helper cells and cytotoxic T-cells), natural killer ("NK") cells, and antigen-presenting cells (*e.g.*, macrophages, dendritic cells, and B cells).

As used herein, the term "immunospecifically binds to an antigen" and analogous terms refer to peptides, polypeptides, fusion proteins and antibodies or fragments thereof that specifically bind to an antigen or a fragment and do not specifically bind to other antigens. A peptide or polypeptide that immunospecifically binds to an antigen may bind to other peptides or polypeptides with lower affinity as determined by, *e.g.,* immunoassays (*e.g.*, competitive ELISAs and radioimmunoassays), BIAcore, or other assays known in the art. Antibodies or fragments that immunospecifically bind to an antigen may cross-reactive with related antigens. Preferably, antibodies or fragments that immunospecifically bind to an antigen do not cross-react with other antigens. In certain embodiments, the antigen to which a peptide, polypeptide, or antibody immunospecifically binds is a cytokine, a cytokine receptor, a costimulatory molecule or a T cell receptor.

As used herein, the term "in combination" refers to the use of more than one prophylactic and/or therapeutic agents. The use of the term "in combination" does not restrict the order in which prophylactic and/or therapeutic agents are administered to a subject with a disorder such as proliferative disorder, an infectious disease, a cardiovascular disease, an inflammatory disorder or an autoimmune disorder. A first prophylactic or therapeutic agent (*e.g.*, lymphoid tissue inducing agent) can be administered prior to (*e.g.,* 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (*e.g.*, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second prophylactic or therapeutic agent (*e.g.*, an immunomodulatory agent) to a subject with a proliferative disorder, an infectious disease, a cardiovascular disease, an inflammatory disorder or an autoimmune disorder.

As used herein, the term "isolated" in the context of a proteinaceous agent (*i.e.*, a peptide, polypeptide, fusion protein or antibody refers to a peptide, polypeptide, fusion protein or antibody) which is substantially free of cellular material or contaminating proteins from the cell or tissue source from which it is derived, or substantially free of chemical precursors or other chemicals when chemically synthesized. The language "substantially free of cellular material" includes preparations of a peptide, polypeptide, fusion protein or antibody in which the peptide, polypeptide, fusion protein or antibody is separated from cellular components of the cells from which it is isolated or recombinantly produced. Thus, a peptide, polypeptide, fusion protein or antibody that is substantially free of cellular material includes preparations of a peptide, polypeptide, fusion protein or antibody having less than about 30%, 20%, 10%, or 5% (by dry weight) of heterologous protein (also referred to herein as a "contaminating protein"). When the peptide, polypeptide, fusion protein or antibody is recombinantly produced, it is also preferably substantially free of culture medium, *i.e.*, culture medium represents less than about 20%, 10%, or 5% of the volume of the protein preparation. When the peptide, polypeptide, fusion protein or antibody is produced by chemical synthesis, it is preferably substantially free of chemical precursors or other chemicals, *i.e.*, it is separated from chemical precursors or other chemicals which are involved in the synthesis of the peptide, polypeptide, fusion protein or antibody. Accordingly such preparations of a peptide, polypeptide, fusion protein or antibody have less than about 30%, 20%, 10%, 5% (by dry weight) of chemical precursors or compounds other than the peptide, polypeptide, fusion protein or antibody of interest.

As used herein, the term "isolated" in the context of a nucleic acid molecule refers to a nucleic acid molecule which is separated from other nucleic acid molecules which are present in the natural source of the nucleic acid molecule. Moreover, an "isolated" nucleic acid molecule, such as a cDNA molecule, can be substantially free of other cellular material, or culture medium when produced by recombinant techniques, or substantially free of chemical precursors or other chemicals when chemically synthesized.

As used herein, the term "isolated" in the context of a non-proteinaceous compound that is not a nucleic acid such as, *e.g.,* a lymphoid tissue inducing agent or an immunomodulatory agent, refers to a compound that is substantially free of chemical precursors or other chemicals when chemically synthesized. In a specific embodiment, the compound is 60%, 65%, 75%, 80%, 85%, 90%, 95%, or 99% free of other, different compounds.

The terms "lower alkoxy", "lower acyl", "(lower alkoxy)methyl" and "(lower alkyl)thiomethyl" as used herein refer to -O-(lower alkyl), -C(O)-(lower alkyl), -CH₂-O-(lower alkyl) and -CH₂-S-( lower alkyl) groups, respectively.

The terms "substituted lower alkoxy" and "substituted lower acyl" as used herein refer to -O-(substituted lower alkyl) and -C(O)-(substituted lower alkyl) groups, respectively.

Suitable substituents on a "substituted lower alkyl," "substituted lower alkoxy," "substituted phenyl," "substituted aryl," "substituted acyl" group, or substituted non-aromatic heterocyclic ring, include, but are not limited to, -OH, halogen (-Br, -Cl, -I and -F), -OR^{a}, -O-COR^{a}, -COR^{a}, -CN, -NO₂, -COOH, -SO₃H, -NH₂, -NHR^{a}, -N(R^{a}R^{b}), -COOR^{a}, - CHO, -CONH₂, -CONHR^{a}, -CON(R^{a}R^{b}), -NHCOR^{a}, -NRCOR^{a}, -NHCONH₂, -NHCONR^{a}H, -NHCON(R^{a}R^{b}), -NR^{c}CONH₂ -NR^{c}CONR^{a}H, -NR^{c}CON(R^{a}R^{b}), -C(=NH)-NH₂, -C(=NH)-NHR^{a}, -C(=NH)-N(R^{a}R^{b}), -C(=NR^{c})-NH₂, -C(=NR^{c})-NHR^{a}, -C(=NR^{c})-N(R^{a}R^{b}), -NH-C(=NH)-NH₂, NH-C(=NH)-NHR^{a}, -NH-C(=NH)-N(R^{a}R^{b}), -NH-C(=NR^{c})-NH₂, -NH-C(=NR^{c})-NHR^{a}, -NH-C(=NR^{c})-N(R^{a}R^{b}), -NR^{d}H-C(=NH)-NH₂, -NR^{d}-C(=NH)-NHR^{a}, -NR^{d}-C(=NH)-N(R^{a}R^{b}), -NR^{d}=C(=NR^{c})-NH₂, -NR^{d}-C(=NR^{c})-NHR^{a}, NR^{d}-C(=NR^{c})-N(R^{a}R^{b}) - NHNH₂, -NHNHR^{a}, -NHR^{a}R^{b}, SO₂NH₂, -SO₂NHR^{a}, -SO₂NR^{a}R^{b}, -CH=CHR^{a}, -CH=CR^{a}R^{b}, CR^{c}=CR^{a}R^{b}, -CR^{c}=CHR^{a}, -CR^{c}=CR^{a}R^{b}, -C≡CR^{a}, -SH, -SOₖR^{a} (k is 0, 1 or 2) and -NH-C(=NH)-NH₂. R^{a}-R^{d} are each independently an aliphatic, substituted aliphatic, benzyl, substituted benzyl, aromatic or substituted aromatic group, preferably an alkyl, benzylic or aryl group. In addition. -NR^{a}R^{d}, taken together, can also form a substituted or unsubstituted nonaromatic heterocyclic group. A non-aromatic heterocyclic group, benzylic group or aryl group can also have an aliphatic or substituted aliphatic group as a substituent. A substituted aliphatic group can also have a non-aromatic heterocyclic ring, a substituted a non-aromatic heterocyclic ring, benzyl, substituted benzyl, aryl or substituted aryl group as a substituent. A substituted lower alkyl group, non-aromatic heterocyclic group, substituted aryl, substituted acyl or substituted phenyl group can have more than one substituent.

As used herein, the terms "lymphoid tissue inducer" and "lymphoid tissue inducing agent" refer to an agent that increases expression of one or more genes involved in producing lymphoid tissue, increases the activity of one or more proteins encoded by such genes sufficiently to induce production of lymphoid tissue, or recruits lymphoid cells to a site (*e.g.*, a tumor site). In a preferred embodiment, lymphoid tissue inducers include agents that induce histologically discernable lymphoid tissue, such as secondary lymphoid tissue (*e.g.*, lymph nodes, spleen, tonsils, and mucosa-associated lymphoid tissue) or lymphoid tissue having various characteristics of, or similarities to, secondary lymphoid tissue. For example, the tissue produced may have discrete regions wherein one or more types of immune cells, such as, *e.g.*, T-cells (*e.g.*, T helper cells or cytotoxic T lymphoyctes ("CTLs")), NK cells, and antigen-presenting cells (*e.g.*, macrophages, dendritic cells and B cells)) aggregate or are otherwise located, housed or sequestered. These discrete regions may resemble, *e.g.*, germinal centers, white pulp or other secondary lymphoid tissue structures. Other hallmarks of lymphoid tissue that may be found include high endothelial venules ("HEV").

As used herein, the term "microtubule stabilizing agent" refers to a compound that induces the polymerization of tubulin and/or stabilizes a microtubule against depolymerization. In a preferred embodiment, the microtubule stabilizing agent stabilizes a microtubule against depolymerization during mitosis. Examples of microtubule stabilizing agents include, but are not limited to, taxanes, including taxol and taxotere; epothilones, including epothilones A-D; discodermolides; eleutherobins; taccalonolides, including taccalonolide A; laulimalides; and sarcodyctins.

As used herein the term "non-aromatic heterocyclic ring" refers to a non-aromatic carbocyclic ring which include one or more heteroatoms such as nitrogen, oxygen or sulfur in the ring. The ring can be five, six, seven or eight-membered. Examples include tetrahydrofuranyl, tetrahyrothiophenyl, morpholino, thiomorpholino, pyrrolidinyl, piperazinyl, piperidinyl, and thiazolidinyl.

As used herein, the terms "non-responsive" and refractory" describe patients treated with a currently available prophylactic or therapeutic agent for a disorder such as a proliferative disorder, an infectious disease, a cardiovascular disease, an inflammatory disorder, or an autoimmune disorder, which is not clinically adequate to relieve one or more symptoms associated with such disorder or disease. Typically, such patients suffer from severe, persistently active disease and require additional therapy to ameliorate the symptoms associated with their disorder.

As used herein, the terms "nucleic acids" and "nucleotide sequences" include DNA molecules (*e.g.,* cDNA or genomic DNA), RNA molecules (*e.g.*, mRNA), combinations of DNA and RNA molecules or hybrid DNA/RNA molecules, and analogs of DNA or RNA molecules. Such analogs can be generated using, for example, nucleotide analogs, which include, but are not limited to, inosine or tritylated bases. Such analogs can also comprise DNA or RNA molecules comprising modified backbones that lend beneficial attributes to the molecules such as, for example, nuclease resistance or an increased ability to cross cellular membranes. The nucleic acids or nucleotide sequences can be single-stranded, double-stranded, may contain both single-stranded and double-stranded portions, and may contain triple-stranded portions, but preferably is double-stranded DNA.

As used herein the term "paclitaxel" refers to the taxane commonly referred to as "taxol." As such, the terms "paclitaxel" and "taxol" may be used interchangeably.

As used herein, the phrase "pharmaceutically acceptable salt" refers to pharmaceutically acceptable organic or inorganic salts of a prophylactic or therapeutic agent. Pharmaceutically acceptable salts of prophylactic or therapeutic agents containing at least one amino group can be formed by acid addition of salts with this amino group. Preferred salts include, but are not limited, to sulfate, citrate, acetate, oxalate, chloride, bromide, iodide, nitrate, bisulfate, phosphate, acid phosphate, isonicotinate, lactate, salicylate, acid citrate; tartrate, oleate, tannate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate, and pamoate (*i.e.,* 1,1'-methylene-bis-(2-hydroxy-3- naphthoate)) salts. A pharmaceutically acceptable salt may involve the inclusion of another molecule such as an acetate ion, a succinate ion or other counterion. The counterion may be any organic or inorganic moiety that stabilizes the charge on the parent compound. Furthermore, a pharmaceutically acceptable salt may have more than one charged atom in its structure. Instances where multiple charged atoms are part of the pharmaceutically acceptable salt can have multiple counterions. Hence, a pharmaceutically acceptable salt can have one or more charged atoms and/or one or more counterions.

As used herein, the term "pharmaceutically acceptable solvate" refers to an association of one or more solvent molecules and a prophylactic or therapeutic agent. Examples of solvents that form pharmaceutically acceptable solvates include, but are not limited to, water, isopropanol, ethanol, methanol, DMSO, ethyl acetate, acetic acid, and ethanolamine.

As used herein, the term "phenyl" refers to a monovalent benzene group. A phenyl group can be unsubstituted or optionally substituted .

As used herein, the terms "prophylactic agent" and "prophylactic agents" refer to any agent(s) which can be used in the prevention of a disorder in which modulation of the immune system is beneficial, including, but not limited to a proliferative disorder, an infectious disease, a cardiovascular disease, an inflammatory disorder, or an autoimmune disorder. In certain embodiments, the term "prophylactic agent" refers to a lymphoid tissue inducing agent and/or an immunomodulatory agent. In certain other embodiments, the term "prophylactic agent" does not refer a lymphoid tissue inducing agent and/or an immunomodulatory agent.

As used herein, the terms "prevent", "preventing" and "prevention" refer to the prevention of the recurrence or onset of one or more symptoms of a disorder in which modulation of the immune system is beneficial, including, but not limited to, a proliferative disorder, an infectious disease, a cardiovascular disease, an inflammatory disorder, or an autoimmune disorder, in a subject resulting from the administration of a combination of prophylactic or therapeutic agents.

As used herein, the term "prophylactically effective amount" refers to that amount of the prophylactic agent sufficient to enhance or improve the prophylactic effect(s) of another prophylactic agent, or to result in the prevention of the recurrence or onset of one or more symptoms of a disorder in which modulation of the immune system is beneficial, including, but not limited to, a proliferative disorder, a cardiovascular disease, an infectious disease, an inflammatory disorder and an autoimmune disorder.

As used herein, a "prophylactic protocol" refers to a regimen for dosing and timing the administration of one or more prophylactic agents.

A used herein, a "protocol" includes dosing schedules and dosing regimens. The protocols herein are methods of use and include prophylactic and therapeutic protocols.

As used herein, the phrase "side effects" encompasses unwanted and adverse effects of a prophylactic or therapeutic agent. Adverse effects are always unwanted, but unwanted effects are not necessarily adverse. An adverse effect from a prophylactic or therapeutic agent might be harmful or uncomfortable or risky. For example, the side effects that may arise with the administration of a lymphoid tissue inducing agent or an immunomodulatory agent include, but are not limited to, weakness, headache, somnolence, nausea, vomiting, dry mouth, muscle pain, bone pain, neutropenia, mucositis, anemia, thrombocytopenia, bradycardia, diarrhea, metallic taste, polydyspsia, polyuria, constipation, weight loss, pancreatitis, photophobia, pruritis, renal dysfunction, aminotransferase elevation, hypertension, psychosis, and a variety of neurologic symptoms.

As used herein, the term "small molecules" and analogous terms include, but are not limited to, peptides, peptidomimetics, amino acids, amino acid analogs, polynucleotides, polynucleotide analogs, nucleotides, nucleotide analogs, organic or inorganic compounds (*i.e,.* including heteroorganic and organometallic compounds) having a molecular weight less than about 10,000 grams per mole, organic or inorganic compounds having a molecular weight less than about 5,000 grams per mole, organic or inorganic compounds having a molecular weight less than about 1,000 grams per mole, organic or inorganic compounds having a molecular weight less than about 500 grams per mole, organic or inorganic compounds having a molecular weight less than about 100 grams per mole, and salts, esters, and other pharmaceutically acceptable forms of such compounds. Salts, esters, and other pharmaceutically acceptable forms of such compounds are also encompassed.

As used herein, the terms "subject" and "patient" are used interchangeably. As used herein, the terms "subject" and "subjects" refer to an animal, preferably a mammal including a non-primate (*e.g.*, a cow, pig, horse, cat, dog, rat, and mouse) and a primate (*e.g.*, a monkey, such as a cynomolgous monkey, and a human), and more preferably a human. In one embodiment, the subject is not an immunocompromised or immunosuppressed mammal, preferably a human (*e.g.,* an HIV patient). In another embodiment, the subject is a farm animal (*e.g.,* a horse, a cow, a pig, etc.) or a pet (*e.g.*, a dog or a cat). In a preferred embodiment, the subject is a human.

As used herein, the term "synergistic" refers to a combination of a lymphoid tissue inducing agent(s) and an immunomodulatory agent(s) which is more effective than the additive effects of the agents. A synergistic effect of a combination of lymphoid tissue inducing agents and immunomodulatory agents permits the use of lower dosages of one or more of the agents and/or less frequent administration of said agents to a subject with a disorder in which modulation of a subject's immune system is beneficial such as a proliferative disorder, a cardiovascular disease, an inflammatory disorder, an autoimmune disorder, or an infectious disease. The ability to utilize lower dosages of a lymphoid tissue inducing agent and/or an immunomodulatory agent, and/or to administer said agents less frequently reduces the toxicity associated with the administration of said agents to a subject without reducing the efficacy of said agents in the prevention or treatment of a disorder in which modulation of a subject's immune system is beneficial such as a proliferative disorder, an inflammatory disorder, a cardiovascular disease, an autoimmune disorder, or an infectious disease. In addition, a synergistic effect can result in improved efficacy of agents in the prevention or treatment of a proliferative disorder, a cardiovascular disease, an inflammatory disorder, an autoimmune disorder, or an infectious disease. Finally, a synergistic effect of a combination of a lymphoid tissue inducing agent and an immunomodulatory agent may avoid or reduce adverse or unwanted side effects associated with the use of either agent alone.

As used herein, the term "T cell receptor modulator" refers to an agent which modulates the phosphorylation of a T cell receptor, the activation of a signal transduction pathway associated with a T cell receptor, and/or the expression of a particular protein such as a cytokine induced in response to the activation of a signal transduction pathway associated with a T cell receptor. Such an agent may directly or indirectly modulate the phosphorylation of a T cell receptor, the activation of a signal transduction pathway associated with a T cell receptor, and/or the expression of a particular protein such as a cytokine. Examples of T cell receptor modulators include, but are not limited to, proteinaneous agents (*e.g.*, cytokines, peptide mimetics, and antibodies), small molecules, organic compounds, inorganic compounds, and nucleic acid molecules encoding proteins, polypeptides, or peptides (*e.g.,* cytokines, peptide mimetics, and antibodies). In certain embodiments, the T cell receptor modulator is a peptide, polypeptide, fusion protein or antibody which immunospecifically bind to a T cell receptor or a fragment thereof. In other embodiments, the T cell receptor modulator is a peptide, polypeptide (*e.g.*, soluble T cell receptor), fusion protein or antibody that immunospecifically binds to a ligand for a T cell receptor or a fragment thereof.

As used herein, the terms "therapeutic agent" and "therapeutic agents" refer to any agent(s) which can be used in the prevention, treatment, management or amelioration of one or more symptoms of a disorder in which modulation of a subject's immune system is beneficial including, but not limited to, a proliferative disorder, a cardiovascular disease, an inflammatory disorder, an autoimmune disorder, and an infectious disease. In certain embodiments, the term "therapeutic agent" refers to a lymphoid tissue inducing agent and/or an immunomodulatory agent. In other embodiments, the term "therapeutic agent" does not refer to a lymphoid tissue inducing agent and/or an immunomodulatory agent.

As used herein, the term "therapeutically effective amount" refers to that amount of the therapeutic agent sufficient to result in the amelioration of one or more symptoms of a disorder, or prevent advancement of a disorder, cause regression of the disorder, or to enhance or improve the therapeutic effect(s) of another therapeutic agent. For example, with respect to the treatment of cancer, a therapeutically effective amount refers to the amount of a therapeutic agent that inhibits or reduces the proliferation of cancerous cells, inhibits or reduces the spread of tumor cells (metastasis), inhibits or reduces the onset, development or progression of one or more symptoms associated with cancer, or reduces the size of a tumor. Preferably, a therapeutically effective of a therapeutic agent reduces the proliferation of cancerous cells or the size of a tumor by at least 5%, preferably at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100%. With respect to the treatment of infectious diseases, a therapeutically effective amount refers to the amount of a therapeutic agent sufficient to reduce or inhibit the replication of an infectious agent (*e.g.*, bacteria, viruses, or fungi), kill the infectious agent, inhibit or reduce the spread of the infectious agent to other tissues or subjects, or ameliorate one or more symptoms associated with the infectious disease. Preferably, a therapeutically effective amount of a therapeutic agent reduces the replication or spread of an infectious agent by at least 5%, preferably at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100%. With respect to the treatment of psoriasis, a therapeutically effective amount refers to the amount of a therapeutic agent that reduces a human's Psoriasis Area and Severity Index (PASI) score by at least 20%, at least 35%, at least 30%, at least 40%, at least 45%, at least 50%, at least 55%, at least 60%, at least 65%, at least 70%, at least 75%, at least 80%, or at least 85%. Alternatively, with respect to the treatment of psoriasis, a therapeutically effective amount preferably refers to the amount of a therapeutic agent that improves a human's global assessment score by at least 25%, at least 35%, at least 30%, at least 40%, at least 45%, at least 50%, at least 55%, at least 60%, at least 65%, at least 70%, at least 75%, at least 80%, at least 85%, at least 90%, or at least 95%. With respect to the treatment of an inflammatory disorder or an autoimmune disorder characterized by inflammation, a therapeutically effective amount refers to the amount of a therapeutic agent that reduces the inflammation of a joint, organ or tissue by at least 5%, preferably at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100%.

As used herein, the term "therapeutic protocol" refers to a regimen for dosing and timing the administration of one or more therapeutic agents.

As used herein, the terms "treat", "treatment" and "treating" refer to the amelioration of one or more symptoms associated with a disorder such as a proliferative disorder, a cardiovascular disease, an inflammatory disorder, an autoimmune disorder, or an infectious disease that results from the administration of a combination of prophylactic or therapeutic agents. In certain embodiments, such terms refer to the inhibition or reduction in the proliferation of cancerous cells, the inhibition or reduction the spread of tumor cells (metastasis), the inhibition or reduction in the onset, development or progression of one or more symptoms associated with cancer, or the reduction in the size of a tumor. In other embodiments, such terms refer to the reduction or inhibition of the replication of an infectious agent (*e.g.*, bacteria, viruses, or fungi), the killing of an infectious agent, the inhibition or reduction in the spread of an infectious agent to other tissues or subjects, or the amelioration of one or more symptoms associated with an infectious disease. In other embodiments, such terms refer to a reduction in the swelling of one or more joints, or a reduction in the pain associated with an inflammatory disorder resulting from the administration of one or more lymphoid tissue inducing agents and one or more immunomodulatory agents to a subject with such a disorder. In other embodiments, such terms refer to a reduction in a human's PASI score. In other embodiments, such terms refer to an improvement in a human's global assessment score.

### 4. BRIEF DESCRIPTION OF THE DRAWINGS

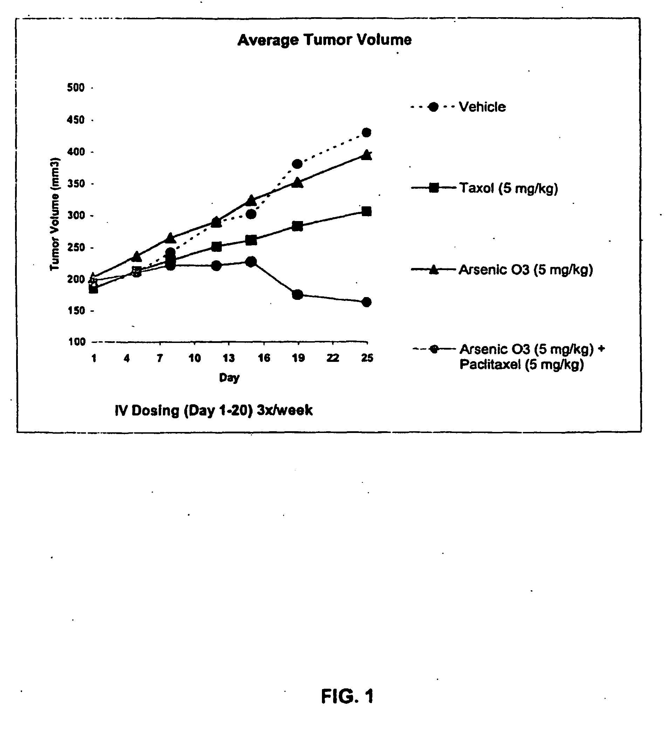

FIG 1. Anti-tumor efficacy of paclitaxel and As₂O₃ treatment in the MDA-435 human xenograph nude mouse model.

### 5. DETAILED DESCRIPTION OF THE INVENTION

The present invention encompasses treatment protocols that provide better prophylactic or therapeutic profiles than current single agent therapies or combination therapies for disorders in which the modulation of a subject's immune system is beneficial including, but not limited to, proliferative disorders, infectious diseases, cardiovascular diseases, autoimmune disorders, and inflammatory disorders. The invention provides lymphoid tissue inducer and immunomodulator-based therapies for the prevention, treatment or amelioration of proliferative disorders, infectious diseases, cardiovascular diseases, autoimmune disorders, and inflammatory disorders or one or more symptoms thereof. In particular, the invention provides prophylactic and therapeutic protocols for the prevention, treatment or amelioration of a proliferative disorder, an infectious disease, a cardiovascular disease, an autoimmune disorder, or an inflammatory disorder or one or more symptoms thereof, comprising administering to a subject in need thereof a prophylactically or therapeutically effective amount of one or more lymphoid tissue inducers and a prophylactically or therapeutically effective amount of one or more immunostimulatory agents.

The present invention provides pharmaceutical compositions and articles of manufacture comprising one or more lymphoid tissue inducers and one or more immunomodulatory agents (preferably, immunostimulatory agents) for use in the prevention, treatment or amelioration of a proliferative disorder, an infectious disease, a cardiovascular disease, an autoimmune disorder, or an inflammatory disorder or one or more symptoms thereof. The present invention also provides methods for screening and identifying one or more lymphoid tissue inducers and one or more immunomodulatory agents (preferably, immunostimulatory agents) for use in the prevention, treatment or amelioration of a proliferative disorder, a cardiovascular disease, an infectious disease, an autoimmune disorder, or an inflammatory disorder or one or more symptoms thereof.

### 5.1. LYMPHOID TISSUE INDUCERS

Lymphoid tissue inducers include, but are not limited to, small molecules, synthetic drugs, proteinaceous agents (*e.g.*, peptides, polypeptides, proteins, and antibodies), nucleic acids (*e.g.*, DNA and RNA nucleotides including, but not limited to, antisense nucleotide sequences, triple helices and nucleotide sequences encoding biologically active proteins, polypeptides or peptides), synthetic or natural inorganic molecules, mimetic agents, and synthetic or natural organic molecules that induce or increase the expression of one or more genes involved in producing lymphoid tissue, or increase one or more of the biological activities of one or more proteins encoded by such genes sufficiently to induce production of lymphoid tissue. In a specific embodiment, a lymphoid tissue inducer increases the expression or one or more of the biological activities of one or more of the following proteins: tumor necrosis factor ("TNF"; including TNF-α, also known as cachectin), lymphotoxin (*e.g.*, lymphotoxin alpha and lymphotoxin beta) NIK, NF-κβ, B lymphocyte chemokine ("BLC"; also known as CXCR5), IL-2, IL-7, IL-12, LT-β, VCAM-1, ICAM-1, and secondary lymphoid organ chemokine ("SLC"; also known as CCR7). In accordance with this embodiment, preferably, the lymphoid tissue inducer increases the expression or one or more of the biological activities of one or more of said proteins 2 fold, 3 fold, 4 fold, 5 fold, 6 fold, 7 fold, 8 fold, 9 fold, 10 fold, 15 fold, 20 fold or more relative to the expression or biological activity in the absence of the lymphoid tissue inducer. In a preferred embodiment, a lymphoid tissue inducer increases the expression of one or more of the following proteins: TNF-α, lymphotoxin-α, and lymphotoxin-β.

In specific embodiment, lymphoid tissue inducers include, but are not limited to, small molecules, synthetic drugs, proteinaceous agents (*e.g.,* peptides, polypeptides, proteins, and antibodies), nucleic acids (*e.g.*, DNA and RNA nucleotides including, but not limited to, antisense nucleotide sequences, triple helices and nucleotide sequences encoding biologically active proteins, polypeptides or peptides), synthetic or natural inorganic molecules, mimetic agents, and synthetic or natural organic molecules that induce or increase the aggregation one or more types of immune cells, such as, *e.g.,* T-cells (*e.g.,* T helper cells or cytotoxic T lymphoyctes ("CTLs")), NK cells, and antigen-presenting cells (*e.g.*, macrophages, dendritic cells and B cells)) in discrete regions, preferably at the site of a disease, *e.g.*, in a tumor. In accordance with this embodiment, the lymphoid tissue inducer increases or induces the aggregation of approximately 10 immune cells/mm³ to approximately 100,000 immune cells/mm³, preferably approximately 1,000 immune cells/mm³ to approximately 100,000 immune cells/mm³, more preferably approximately 10,000 immune cells/mm³ to approximately 100,000 immune cells/mm³ at the site of a disease. In a specific embodiment, the lymphoid tissue inducer increases or induces the aggregation of at least approximately 10 immune cells/mm³, preferably at least approximately 50 immune cells/mm³, at least approximately 100 immune cells/mm³, at least approximately 500 immune cells/mm³, at least approximately 1,000 immune cells/mm³, at least approximately 5,000 immune cells/mm³, at least approximately 10,000 immune cells/mm³, at least approximately 25,000 immune cells/mm³, at least approximately 50,000 immune cells/mm³, at least approximately 75,000 immune cells/mm³, or at least approximately 100,000 immune cells/mm³.

In a preferred embodiment, lymphoid tissue inducers include, but are not limited to, small molecules, synthetic drugs, proteinaceous agents (*e.g.*, peptides, polypeptides, proteins and antibodies), nucleic acids (*e.g.,* DNA and RNA nucleotides including, but not limited to, antisense nucleotide sequences, triple helices and nucleotide sequences encoding biologically active proteins, polypeptides or peptides), synthetic or natural inorganic molecules, mimetic agents, and synthetic or natural organic molecules that induce histologically discemable lymphoid tissue, such as secondary lymphoid tissue (*e.g.*, lymph nodes, spleen, tonsils, and mucosa-associated lymphoid tissue) or lymphoid tissue having various characteristics of, or similarities to, secondary lymphoid tissue. In accordance with this embodiment, the lymphoid tissue inducer induces lymphoid tissue that is approximately 0.05 mm to approximately 10 mm, preferably approximately 0.5 mm to approximately 10 mm, and more preferably approximately 1 mm to approximately 10 mm. In a specific embodiment, the lymphoid tissue inducer induces lymphoid tissue that is approximately 0.05 mm, preferably approximately 0.5 mm, approximately 1 mm, approximately 1.5 mm, approximately 2 mm, approximately 2.5 mm, approximately 3 mm, approximately 3.5 mm, approximately 4 mm, approximately 4.5 mm, approximately 5 mm, approximately 5.5 mm, approximately 6 mm, approximately 7 mm, approximately 8 mm, approximately 9 mm or approximately 10 mm. Preferably, the lymphoid tissue inducer induces lymphoid tissue at the site of the disease.

In accordance with the invention, a lymphoid tissue inducer can be further characterized into groups such as microtubule stabilizing agents, TNF-inducing agents and small molecules. As a result of such characterization of lymphoid tissue inducers, a particular lymphoid tissue inducer may be considered, *e.g.,* both a TNF-inducing agent and a small molecule. In accordance with invention and under such circumstances, the lymphoid tissue inducers administered to a subject with a disorder described herein are different when, *e.g.,* the lymphoid tissue inducers are considered both a TNF-inducing agent and small molecule.

In another embodiment, the lymphoid tissue inducer is a microtubule stabilizing agent. In an alternative embodiment, the lymphoid tissue inducer is not a microtubule stabilizing agent. In another embodiment, the lymphoid tissue inducer is taxol, paclitaxel, an epothilone, a discodermolide, a eleutherobin, a laulimalide, a taccalonolide, or a sarcodictyin or taxotere. In an alternative embodiment, the lymphoid tissue inducer is not one or more of the following agents: taxol, paclitaxel, an epothilone, a discodermolide, a eleutherobin, a laulimalide, a taccalonolide, or a sarcodictyin or taxotere. In another embodiment, a lymphoid tissue inducer is a recombinant TNF-alpha or a TNF-inducing agent, in particular a TNF-alpha-inducing agent or a TNF-beta-inducing agent. In an alternative embodiment, a lymphoid tissue inducer is not a recombinant TNF-alpha or a TNF-inducing agent. In a specific embodiment, the lymphoid tissue inducer is a small molecule.

### 5.1.1. MICROTUBLE STABILIZING AGENTS

In accordance with the invention, a microtubule stabilizing agent may used as a lymphoid tissue inducer. Microtubule stabilizing agents useful in the present invention include, but are not limited to, the agents listed in Table 1, derivatives or analogs thereof, and pharmaceutically acceptable salts, solvates or hydrates thereof.

**Table 1**

| |
|---|
| Taxanes |
| Taxol |
| Taxotere |
| Paclitaxel |
| Epothilones |
| Epothilone A |
| Epothilone B |
| Epothilone C |
| Epothilone D |
| Epothilone E |
| Epothilone F |
| Desoxyepothilone B |
| Desoxyepothilone F |
| aza-Epothilone B |
| BMS-247550 |
| Discodermolides |
| Eleutherobins |
| Eluthoside A |
| Eluthoside B |
| Taccalonolides |
| Taccalonolide A |
| Laulimalides |
| Sarcodictyins |
| Sarcodictyin A |
| Sarcodictyin B |

In a specific embodiment, the microtubule stabilizing agent is a taxane of formula (I) or (II). 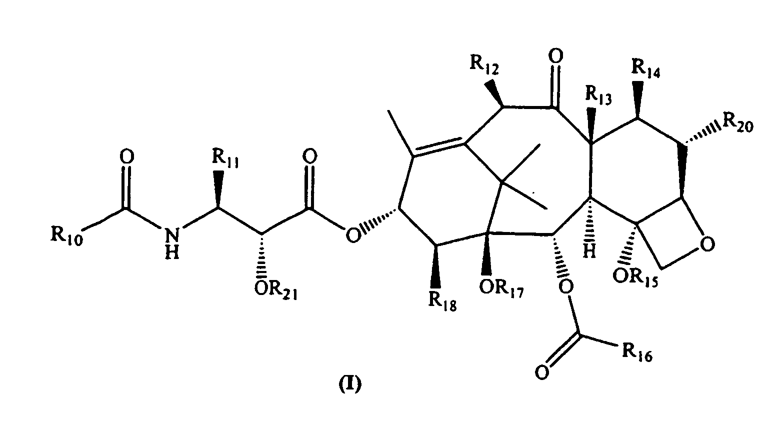 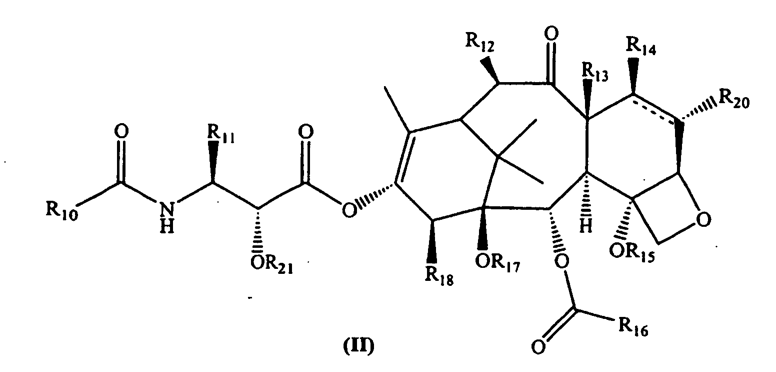 wherein
R₁₀ is a lower alkyl group, a substituted lower alkyl group, a phenyl group, a substituted phenyl group, -SR₁₉, -NHR₁₉ or -OR₁₉;
R₁₁ is a lower alkyl group, a substituted lower alkyl group, an aryl group or a substituted aryl group;.
R₁₂ is -H, -OH, lower alkyl, substituted lower alkyl, lower alkoxy, substituted lower alkoxy, -O-C(O)-(lower alkyl), -O-C(O)-(substituted lower alkyl), -O-CH₂-O-(lower alkyl) - S-CH₂-O-(lower alkyl);
R₁₃ is -H or -CH₃, or R₁₃ taken together with R₁₄ and the carbon atoms to which they are attached, form a cyclopropyl group;
R₁₄ is -H, -OH, lower alkoxy, -O-C(O)-(lower alkyl), substituted lower alkoxy, -O-C(O)-(substituted lower alkyl), -O-CH₂-O-P(O)(OH)₂, -O-CH₂-O-(lower alkyl) or -O-CH₂-S-(lower alkyl), or R₁₄ is taken together with R₂₀ to form a double bond;
R₁₅ is -H, lower acyl, lower alkyl, substituted lower alkyl, alkoxymethyl, alkthiomethyl, -OC(O)-O(lower alkyl), -OC(O)-O(substituted lower alkyl), -OC(O)NH(lower alkyl) or -OC(O)-NH(substituted lower alkyl);
R₁₆ is phenyl or substituted phenyl;
R₁₇ is -H, lower acyl, substituted lower acyl, lower alkyl, substituted lower alkyl, (lower alkoxy)methyl or (lower alkyl)thiomethyl, or R₁₇ and R₁₈, taken together with the atoms to which they are attached, form a five- or six-membered non-aromatic heterocyclic ring;
R₁₈ is -H or -CH₃;
R₁₉ is a lower alkyl group, a substituted lower alkyl group, a phenyl group or a substituted phenyl group;
R₂₀ is -H, -F, -Cl, -Br or -I; and
R₂₁ is -H, lower alkyl, substituted lower alkyl, lower acyl or substituted lower acyl.

Preferably, the variables in Structural Formulas (I) and (II) are defined as follows: R₁₀ is phenyl, *tert*-butoxy, -S-CH₂-CH-(CH₃)₂, -S-CH(CH₃)₃, -S-(CH₂)₃CH₃, -O-CH(CH₃)₃, -NH-CH(CH₃)₃, -CH=C(CH₃)₂ or *para*-chlorophenyl; R₁₁ is phenyl, (CH₃)₂CHCH₂-, -2-furanyl, cyclopropyl or *para*-toluyl; R₁₂ is -H, -OH. CH₃CO- or -(CH₂)₂-*N*-morpholino; R₁₃ is methyl, or, R₁₃ and R₁₄, taken together, are -CH₂-,
R₁₄ is -H, -CH₂SCH₃ or -CH₂-O-P(O)(OH)₂ R₁₅ is CH₃CO-;
R₁₆ is phenyl; R₁₇ -H, or, R₁₇ and R₁₈, taken together, are -O-CO-O-;
R₁₈ is -H; R₂₀ is -H or -F; and R₂₁ is -H, -C(O)-CHBr-(CH₂)₁₃-CH₃ or -(C(O)-CH₂)₁₄-CH₃; -C(O)-CH₂-CH(OH)-COOH, -C(O)-CH₂-O-C(O)-CH₂CH(NH₂)-CONH₂, -C(O)-CH₂-O--CH₂CH₂OCH₃ or -C(O)-O-C(O)-CH₂CH₃.

In one embodiment, the microtubule stabilizing agent is taxol or taxotere. In an alterative embodiment, the microtubule stabilizing agent is not taxol or taxotere.

A "taxane" as used and defined herein also includes compounds of formulas (I) and (II) which are attached to a pharmaceutically acceptable polymer including, but not limited to, a polyacrimide.

Microtubule stabilizing agents can be obtained commercially, isolated from natural sources or can be synthesized by methods known to the skilled artisan. For example, taxol can be isolated from the Pacific Yew or synthesized as described in Holton, R.A. et al., 1994, J. Am. Chem. Soc. 116:1597-1599. Epothilones can be isolated from Myxobacteria of the genus *Sorangium* by methods known to the skilled artisan. Alternatively, epothilones and analogs thereof, can be synthesized as described in Chou, T.C. et al., 2001, PNAS 98(14):8113-8118; U.S. Patent No. 6,300,355 to Danishefsky, et al.; and Lee, F.Y.F. et al., 2001, Clin. Cancer Res. 7(5):1429-1437. Discodermolides suitable for use in the invention can be isolated from marine sponges by known methods or alternatively, can be synthesized as described in U.S. Patent Nos. 4,939,168 and 5,010,099 to Gunasekera et al.; U.S. Patent Nos. 5,681,847 and 5,840,750 to Longley et al.; and Hung, D.T., 1996, J. Am. Chem. Soc. 118:11054-11080. Laulimalides can be obtained from marine sponges and are further described in Corley, D.J. et al., 1988, J. Org. Chem. 53(15):3644-3646.

Eleutherobins and sarcodictyins can be isolated from soft coral by methods described in Nicolaou, K.C. et al., 1997, J. Am. Chem. Soc. 119:11353-11354. Methods of synthesizing various eleutherobins, can be found in Nicolaou, et al., 1997, J. Am. Chem. Soc. 120 (34):8674-8680. Sarcodictyins, and analogs thereof, can be prepared as described in U.S. Patent No. 5,965,718 to Nicolaou et al.

### 5.1.2. TNF-INDUCING AGENTS

In accordance with the invention, a recombinant TNF-alpha or a TNF-inducing agent may be used as a lymphoid tissue inducer. A TNF-inducing agent is an agent that induces or increases the expression of a TNF-encoding gene (*e.g.*, TNF-alpha encoding gene and TNF-beta encoding gene), or increases the activity of TNF (*e.g.*, TNF-alpha and TNF-beta). Examples of TNF-inducing agents include, but are not limited to, cytokines (*e.g.*, IL-19) fragments cytokines, flavone acetic acid ("FAA"), OK-432, PSK, glycyrrhizin, prostaglandin E2 and J2, romurtide, cimetidine, LPS, peptidoglycan, lipoteichoic acid, and derivatives and analogs thereof. In a specific embodiment, the TNF-inducing agent increases the expression or one or more of the biological activities of TNF-alpha or TNF-beta 2 fold, 3 fold, 4 fold, 5 fold, 6 fold, 7 fold, 8 fold, 9 fold, 10 fold, 15 fold, 20 fold or more relative to the expression or biological activity in the absence of said agent.

### 5.1.3. SMALL MOLECULES

In accordance with the invention, a small molecule that induces or increases the expression of one or more genes involved in producing lymphoid tissue, or increases one or more of the biological activities of one or more proteins encoded by such genes sufficiently to induce production of lymphoid tissue may be used as a lymphoid tissue inducer. In a specific embodiment, a small molecule increases the expression or one or more of the biological activities of one or more of the following proteins: TNF, lymphotoxin, NIK, NF-κβ, B lymphocyte chemokine ("BLC"; also known as CXCR5), IL-2, IL-7, IL-12, LT-β, VCAM-1, ICAM-1, and secondary lymphoid organ chemokine ("SLC"; also known as CCR7). In accordance with this embodiment, preferably, the small molecule increases the expression or one or more of the biological activities of one or more of said proteins 2 fold, 3 fold, 4 fold, 5 fold, 6 fold, 7 fold, 8 fold, 9 fold, 10 fold, 15 fold, 20 fold or more relative to the expression or biological activity in the absence of said small molecule. Examples such small molecules include, but are not limited to, pentoxifylline, which induces lymphotoxin expression; and camptothecin, which activates NF-kB.

### 5.2: IMMUNOMODULATORY AGENTS

Any immunomodulatory agent known to one of skill in the art may be used in the methods and compositions of the invention. Immunomodulatory agents can affect one or more or all aspects of the immune response in a subject. Aspects of the immune response include, but are not limited to, the inflammatory response, the complement cascade, leukocyte and lymphocyte differentiation, proliferation and/or effector function, monocyte and/or basophil counts, and the cellular communication among cells of the immune system. In certain embodiments of the invention, an immunomodulatory agent modulates one aspect of the immune response. In other embodiments, an immunomodulatory agent modulates more than one aspect of the immune response.

An immunomodulatory agent may be selected to alter (*e.g.*, increase) the proliferation, differentiation, activity and/or function of CD4⁺ and/or CD8⁺ T cells. For example, antibodies specific for T cells can be used as immunomodulatory agents to induce the proliferation, differentiation, activity and/or function of CD4⁺ and/or CD8⁺ T cells. In a specific embodiment, an immunomodulatory agent alters Th1 and/or Th2 proliferation, differentiation and/or effector function.

Examples of immunomodulatory agents include, but are not limited to, proteinaceous agents (*e.g.*, cytokines, peptide mimetics, and antibodies (*e.g.,* human antibodies, humanized antibodies, camelised antibodies, chimeric antibodies, monoclonal antibodies, polyclonal antibodies, single domain antibodies, Fvs, ScFvs, Fab or F(ab)2 fragments or epitope binding fragments)}, nucleic acid molecules (*e.g.*, antisense nucleic acid molecules, triple helices and nucleic acid molecules encoding peptides, polypeptides, proteins and antibodies), small molecules, organic compounds, and inorganic compounds. In particular, immunomodulatory agents include, but are not limited to, methothrexate, leflunomide, cyclophosphamide (Cytoxan), azathioprine (Immuran), cyclosporine, minocycline, antibiotics, tacrolimus (FK506), methylprednisolone (MP), corticosteroids, steriods, mycophenolate mofetil (CellCept), rapamycin (sirolimus), chlorambucil, mizoribine, deoxyspergualin, brequinar, malononitriloamindes (*e.g.*, leflunamide), T cell receptor modulators, cytokine receptor modulators and bis[thio-hydrazide amide] compounds. For clarification regarding T cell receptor modulators and cytokine receptor modulators see Section 3.1. Examples of T cell receptor modulators include, but are not limited to, anti-T cell receptor antibodies (*e.g.*, anti-CD4 antibodies (*e.g.*, cM-T412 (Boeringer), IDEC-CE9.1® (IDEC and SKB), mAB 4162W94, Orthoclone and OKTcdr4a (Janssen-Cilag)), anti-CD3 antibodies (*e.g.,* Nuvion (Product Design Labs), OKT3 (Johnson & Johnson), or Rituxan (IDEC)), anti-CD5 antibodies (*e.g.*, an anti-CD5 ricin-linked immunoconjugate), anti-CD7 antibodies (*e.g.*, CHH-380 (Novartis)), anti-CD8 antibodies, anti-CD40 ligand monoclonal antibodies (*e.g.*, IDEC-131 (IDEC)), anti-CD52 antibodies (*e.g.*, CAMPATH 1H (Ilex)), anti-CD2 antibodies, anti-CD11a antibodies (*e.g.*, Xanelim (Genentech)), and anti-B7 antibodies (*e.g.,* IDEC-114) (IDEC))) and CTLA4-immunoglobulin ("CTLA4-Ig").

Examples of cytokine receptor modulators include, but are not limited to, soluble cytokine receptors (*e.g.*, the extracellular domain of a TIVF-alpha receptor or a fragment thereof, the extracellular domain of an interleukin ("IL")-1α receptor or a fragment thereof, and the extracellular domain of an IL-6 receptor or a fragment thereof), cytokines or fragments thereof (*e.g.*, IL-2, IL-3, IL-4, IL-5, IL-6, IL-7, IL-8, IL-9, IL-10, IL-11, IL-12, IL-15, TNF-alpha, TNF-beta, interferon (IFN)-alpha, IFN-beta, IFN-gamma, and GM-CSF), anti-cytokine receptor antibodies (*e.g.*, anti-IFN receptor antibodies, anti-IL-2 receptor antibodies (*e.g.,* Zenapax (Protein Design Labs)), anti-IL-4 receptor antibodies, anti-IL-6 receptor antibodies, anti-IL-10 receptor antibodies, and anti-IL-12 receptor antibodies), anti-cytokine antibodies (*e.g.*, anti-IFN antibodies, anti-TNF-α antibodies, anti-IL-1α antibodies, anti-IL-6 antibodies, anti-IL-8 antibodies (*e.g.*, ABX-IL-8 (Abgenix)), anti-IL-9 antibodies and anti-IL-12 antibodies). In a specific embodiment, a cytokine receptor modulator is IL-4, IL-10, or a fragment thereof. In another embodiment, a cytokine receptor modulator is an anti-IL-1α antibody, anti-IL-6 antibody, anti-IL-12 receptor antibody, or anti-TNF-α antibody. In another embodiment, a cytokine receptor modulator is the extracellular domain of a TNF-alpha receptor or a fragment thereof. In certain embodiments, a cytokine receptor modulator is not the extracellular domain of a TNF-alpha receptor or a fragment thereof.

In certain embodiments, immunomodulatory agents include chemotherapeutic agents such as methotrexate, cyclosporine A, leflunomide, cisplatin, ifosfamide, paclitaxol, taxanes, topoisomerase I inhibitors (*e.g.,* CPT- 11, topotecan, 9-AC, and GG-211), gemcitabine, vinorelbine, oxaliplatin, 5-fluorouracil (5-FU), leucovorin, vinorelbine, temodal, taxol, cytochalasin B, gramicidin D, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, melphalan, glucocorticoids, procaine, tetracaine, lidocaine, propranolol, puromycin homologs, and cytoxan. In other embodiments, immunomodulatory agents do not include one or more chemotherapeutic agents such as methotrexate, cyclosporine A, leflunomide, cisplatin, ifosfamide, paclitaxol, taxanes, topoisomerase I inhibitors (e.g., CPT-11, topotecan, 9-AC, and GG-211), gemcitabine, vinorelbine, oxaliplatin, 5-fluorouracil (5-FU), leucovorin, vinorelbine, temodal, taxol, cytochalasin B, gramicidin D, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, melphalan, glucocorticoids, procaine, tetracaine, lidocaine, propranolol, puromycin homologs, and cytoxan.

In a preferred embodiment of the invention, the immunomodulatory agent is an immune system enhancer. Any immune system enhancer known to one of skill in the art may be used in the methods and compositions of the invention.

An immune system enhancer may be selected to increase the proliferation, differentiation, activity and/or function of CD4⁺ and/or CD8⁺ T cells. For example, antibodies specific for T cells can be used as immune system enhancer to induce the proliferation, differentiation, activity and/or function of CD4⁺ and/or CD8⁺ T cells. In a specific embodiment, an immune system enhancer induces or increases the expression or one or more of the biological activities of one or more of the following proteins: a heat shock protein ("HSPs"; *e.g.,* HSP70 and HSP90), ICAM (*e.g.,* ICAM-1), CCR7, BLC and SLC. In a preferred embodiment, an immune system enhancer induces or increases the expression or one or more biological activities of one or more heat shock proteins, such as HSP70, that can, *e.g.*, induce cytotoxic T cells, dendritic cells and natural killer (NK) cells. In accordance with these embodiments, preferably, the immune system enhancers increase the expression or one or more of the biological activities of one or more of said proteins 2 fold, 3 fold, 4 fold, 5 fold, 6 fold, 7 fold, 8 fold, 9 fold, 10 fold, 15 fold, 20 fold or more relative to the expression or biological activity in the absence of said immune system enhancer.

Examples of immune system enhancers include, but are not limited to, proteinaceous agents (*e.g.*, cytokines, peptide mimetics, and antibodies (*e.g.*, human antibodies, humanized antibodies, camelised antibodies, chimeric antibodies, monoclonal antibodies, polyclonal antibodies, single domain antibodies, Fvs, ScFvs, Fab or F(ab)2 fragments or epitope binding fragments), nucleic acid molecules (*e.g.*, antisense nucleic acid molecules, triple helices and nucleic acid molecules encoding peptides, polypeptides, and antibodies), small molecules, organic compounds, and inorganic compounds. In particular, immune system enhancers include, but are not limited to, ICAM-inducing agents (*e.g.,* tributyrin, OK-432, retinoic acid/vitamin A, sodium butyrate, lymphotoxin-α and cisplatin), HSP-inducing agents (*e.g.,* curcumin, arsenite, prostaglandins, prostaglandin-like molecules, geranyl-gemayl-acetone, cyclosporine A, glutamine, aspirin, herbimycin A and bis[thio-hydrazide amide] compounds), BLC-inducing agents (*e.g.*, CpG oligonucleotides and lymphotoxin-α), SLC-inducing agents (*e.g.*, lymphotoxin-α), Coley's toxins, T cell receptor modulators, and cytokine receptor modulators. For clarification regarding T cell receptor modulators and cytokine receptor modulators see Section 3.1.

Certain immune system enhancers may be favored in certain circumstances. Preferred immune system enhancers for treating breast cancer, for example, can be selected based on their ability to facilitate antibody-based cancer cell killings, including facilitating such killings by complement fixation, antibody-dependent cellular toxicity (ADCC), C5a neutrophil killing, and opsonin-macrophage killing. Preferred enhancers in the treatment of breast cancer include prostaglandin J2, which induces HSP70, monoclonal antibodies for various oncogenic products, such as Her-2-neu, monoclonal antibody against epidermal growth factor (EGF) receptor, epithelial cell adhesion molecule (EpCAM) and OK-432.

In certain circumstances an immunomodulatory agent (*e.g.*, an immune system enhancer) may also be characterized as a lymphoid tissue inducer. Thus, in accordance with the invention, the immunomodulatory agent used in accordance with the invention is different than the lymphoid tissue inducer.

In accordance with the invention, an immunomodulatory agent can be further characterized into groups such as HSP-inducing agents, antibodies, chemokine receptor-inducing agents and ICAM-inducing agents. As a result of such characterization a particular immunomodulatory agent may be considered, *e.g.*, both an HSP-inducing agent and an ICAM-inducing agent. In accordance with the invention and under such circumstances, the immunomodulatory agents administered to a subject with a disorder described herein are different when, *e.g.*, the immunomodulatory agents are considered both an HSP-inducing agent and an ICAM-inducing agent.

In a specific embodiment, the immunomodulatory agent is an antibody. In another embodiment, the immunomodulatory agent is not an antibody. In another embodiment, the immunomodulatory agent is an HSP-inducing agent. In another embodiments, the immunomodulatory agent is not an HSP-inducing agent. In another embodiment, the immunomodulatory agent is an ICAM-inducing agent. In another embodiment, the immunomodulatory agent is not an ICAM-inducing agent. In another embodiment, the immunomodulatory agent is a chemokine receptor-inducing agent. In yet another embodiment, the immunomodulatory agent is not a chemokine-receptor-inducing agent.

In another embodiment, an immunomodulatory agent does not have the chemical structure of compounds I to VI depicted in the following applications by Koya et al., entitled "Taxol Enhancer Compounds" or "Synthesis of Taxol Enhancer": U.S. patent application Serial Nos. 10/193,075, 10/193,639, and 10/193,076; and PCT Application Nos. PCT/US02/21717, PCT/US02/21714, and PCT/US02/21716, each of which is incorporated herein by reference in its entirety.

### 5.2.1. ANTIBODIES AS IMMUNOMODULATORY AGENTS

Antibodies that may be used as immunomodulatory agents (preferably, immune system enhancing agents) can be selected based on the nature of the disease being treated. For example, when treating a particular neoplastic condition, the required antibodies will depend on the nature of the neoplasm. Antibodies can be targeted against known antigens, including tumor-specific cell surface receptors present on the surface of tumor cells, and can thus be utilized to more effectively allow recognition of the tumor cells by the immune system. As mentioned above, such antibodies will vary depending on the nature of the neoplasm and include, for example, antibodies directed against the following antigens: class 1-restricted antigens recognized by CD8⁺ lymphocytes, such as melanoma-melanocyte differentiation antigens and including Melan-A, tyrosinase, melanocyte-stimulating hormone receptor; mutated antigens such as MUM-1, CDK-4, caspase-8, KIA 0205, cancer testes antigens, including MACE-1, MACE-2, MACE -3, MACE-12, BAGE, GAGE and non-mutated shared antigens such as α-fetoprotein, telomerase catalytic protein, G-250, MUC-1, p53, and Her-2/neu. Other antigens can include class II-restricted antigens recognized by CD4⁺ lymphocytes, such as gp100, MACE-1, CDC-27 and NY-LSO-1, as well as others known to the art and discussed in, for example, Rosenberg, S.A., 2001, Nature 111:380-384. Other suitable antigens include carcinoembryonic antigen and vascular endothelial cell adhesion molecules ("VCAM"), including VCAM-1, intracellular adhesion molecules ("ICAM"), such as ICAM-1; adhesion molecules such as epithelial cell adhesion molecule (EpCAM), periostin, integrin, and cadherin; endoglin; cell surface receptors, including growth factor receptors, such as epidermal growth factor receptors, insulin-like growth factor receptors, platelet-derived endothelial cell growth factor receptor, and transforming growth factor receptors; APO-1; proto-oncogene product receptors, such as c-*kit*; folate receptors; intratumoral vasculature cell surface receptors, including vascular endothelial growth factor/vascular permeability factor (VEGF/VPF) receptor, and fibroblast growth factor (FGF) receptor; and prostate-specific membrane antigen (PSMA).

In a specific embodiment, known antibodies for the treatment or prevention of cancer are used in accordance with the compositions and methods of the invention. Examples of antibodies available for the treatment of cancer include, but are not limited to, HERCEPTIN (Trastuzumab; Genentech, CA) which is a humanized anti-HER2 monoclonal antibody for the treatment of patients with metastatic breast cancer (Stebbing et al., 2000, Cancer Treat Rev. 26:287-90); RITUXAN (rituximab; Genentech) which is a chimeric anti-CD20 monoclonal antibody for the treatment of patients with non-Hodgkin's lymphoma; OvaRex (AltaRex Corporation, MA) which is a murine antibody for the treatment of ovarian cancer; Panorex (Glaxo Wellcome, NC) which is a murine IgG₂ₐ antibody for the treatment of colorectal cancer; BEC2 (ImClone Systems Inc., NY) which is murine IgG antibody for the treatment of lung cancer; IMC-C225 (Imclone Systems Inc., NY) which is a chimeric IgG antibody for the treatment of head and neck cancer; Campath I/H (Leukosite, MA) which is a humanized IgG, antibody for the treatment of chronic lymphocytic leukemia (CLL); Smart MI95 (Protein Design Labs, Inc., CA) which is a humanized IgG antibody for the treatment of acute myeloid leukemia (AML); LymphoCide (Immunomedics, Inc., NJ) which is a humanized IgG antibody for the treatment of non-Hodgkin's lymphoma; Smart ID10 (Protein Design Labs, Inc., CA) which is a humanized antibody for the treatment of non-Hodgkin's lymphoma; Oncolym (Techniclone, Inc., CA) which is a murine antibody for the treatment of non-Hodgkin's lymphoma; Allomune (BioTransplant, CA) which is a humanized anti-CD2 mAb for the treatment of Hodgkin's Disease or non-Hodgkin's lymphoma; anti-VEGF (Genentech, Inc., CA) which is humanized antibody for the treatment of lung and colorectal cancers; CEAcide (Immunomedics, NJ) which is a humanized anti-CEA antibody for the treatment of colorectal cancer, IMC-1C11 (ImClone Systems, NJ) which is an anti-KDR chimeric antibody for the treatment of colorectal cancer, lung cancers, and melanoma; and Cetuximab (ImClone, NJ) which is an anti-EGFR chimeric antibody for the treatment of epidermal growth factor positive cancers.

Other antibodies useful in the treatment of cancer include, but are not limited to, antibodies against the following antigens: CA125 (ovarian), CA15-3 (carcinomas), CA19-9 (carcinomas), L6 (carcinomas), Lewis Y (carcinomas), Lewis X (carcinomas), alpha fetoprotein (carcinomas), CA 242 (colorectal), placental alkaline phosphatase (carcinomas), prostate specific antigen (prostate), prostatic acid phosphatase (prostate), epidermal growth factor (carcinomas), MAGE-1 (carcinomas), MAGE-2 (carcinomas), MAGE-3 (carcinomas), MAGE -4 (carcinomas), anti-transferrin receptor (carcinomas), p97 (melanoma), MUC1-KLH (breast cancer), CEA (colorectal), gp100 (melanoma), MART1 (melanoma), PSA (prostate), IL-2 receptor (T-cell leukemia and lymphomas), CD20 (non-Hodgkin's lymphoma), CD52 (leukemia), CD33 (leukemia), CD22 (lymphoma), human chorionic gonadotropin (carcinoma), CD38 (multiple myeloma), CD40 (lymphoma), mucin (carcinomas), P21 (carcinomas), MPG (melanoma), and Neu oncogene product (carcinomas). Some specific useful antibodies include, but are not limited to, BR96 mAb (Trail et al., 1993, Science 261:212-215), BR64 (Greenfieldet al., 1997, Cancer Research 57:100-105), mAbs against the CD 40 antigen, such as S2C6 mAb (Francisco et al., 2000, Cancer Res. 60:3225-3231), and mAbs against the CD30 antigen, such as AC10 (Bowen et al., 1993, J. Immunol. 151:5896-5906). Many other internalizing antibodies that bind to tumor-associated antigens can be used in this invention (for reviews see, *e.g.,* Franke et al., 2000, Cancer Biother Radiopharm. 15:459-76; Murray, J. L., 2000, Semin Oncol. 27:64-70; and Breitling, F., and Dubel, S., *Recombinant Antibodies,* John Wiley, and Sons, New York, 1998 ).

In the case of infectious diseases, antibodies that can be utilized as immunomodulatory agents include those that immunospecifically bind to a microbial antigen. As used herein, the term "microbial antigen" includes, but is not limited to, any microbial peptide, polypeptide, protein, saccharide, polysaccharide, or lipid molecule (*e.g.*, a bacterial, fungi, pathogenic protozoa, or yeast polypeptide including, *e.g.*, LPS and capsular polysaccharide 5/8) that is capable of eliciting an immune response.

In the case of bacterial infectious diseases, antibodies that can be utilized as immunomodulatory agents include those directed against a wide variety of bacterial cell wall and cell surface components including, for example, lipid A, peptidylglycan, teichoic acid, lipoteichoic acid, D-leucine-containing moieties and various bacterial-specific polysaccharides, glycoconjugates, glycolipids, lipopolysaccharides, and polypeptides known to the art. Other antibodies useful in the invention for the treatment of bacterial infectious diseases include, but are not limited to, antibodies against the antigens from the following pathogenic strains of bacteria: *Streptococcus pyogenes, Streptococcus pneumoniae, Neisseria* *gonorrheae, Neisseria meningitidis, Corynebacterium diphtheriae, Clostridium botulinum, Clostridium perfringens, Clostridium tetani, Hemophilus influenzae, Klebsiella pneumoniae, Klebsiella ozaenas, Klebsiella rhinoscleromotis, Staphylococcus aureus, Vibrio colerae, Escherichia coli, Pseudomonas aeruginosa, Campylobacter (Vibrio)fetus, Aeromonas hydrophila, Bacillus aereus, Edwardsiella tarda, Yersinia enterocolitica, Yersinia pestis, Yersinia pseudotuberculosis, Shigella dysenteriae, Shigella flexneri, Shigella sonnei, Salmonella typhimurium, Treponema pallidum, Treponema pertenue, Treponema carateneum, Borrelia vincentii, Borrelia burgdorferi, Leptospira icterohemorrhagiae, Mycobacterium tuberculosis, Pneumocystis carinii, Francisella tularensis, Brucella abortus, Brucella suis, Brucella melitensis, Mycoplasma spp., Rickettsia prowazeki, Rickettsia tsutsugumushi,* and *Chlamydia spp..*

In the case of fungal or parasitic infectious diseases, antibodies that can be utilized as immunomodulatory agents include those known in the art that are directed against a wide variety of fungal/parasitic components of the fungi/parasite. Antibodies useful in the treatment of fungal or parasitic infectious diseases include, but are not limited to, antibodies against the antigens from pathogenic fungi (*e.g., Coccidioides immitis, Aspergillus fumigatus, Candida albicans, Blastomyces dermatitidis, Cryptococcus neoformans,* and *Histoplasma capsulatum);* protozoa (*e.g., Entomoeba histolytica, Toxoplasma gondii, Trichomonas tenas, Trichomonas hominis, Trichomonas vaginalis, Tryoanosoma gambiense, Trypanosoma rhodesiense, Trypanosoma cruzi, Leishmania donovani, Leishmania tropica, Leishmania braziliensis, Pneumocystis pneumonia, Plasmodium vivax, Plasmodium falciparum,* and *Plasmodium malaria*); or Helminiths (*e.g., Enterobius vermicularis, Trichuris trichiura, Ascaris lumbricoides, Trichinella spiralis, Strongyloides stercoralis, Schistosoma japonicum, Schistosoma mansoni, Schistosoma haematobium,* and hookworms).

In the case of viral infectious diseases, antibodies that can be utilized as immunomodulatory agents include those known in the art that are immunospecific for a viral antigen. As used herein, the term "viral antigen" includes, but is not limited to, any viral peptide, polypeptide and protein (*e.g.*, HIV gp120, HIV nef, RSV F glycoprotein, influenza virus neuraminidase, influenza virus hemagglutinin, HTLV tax, herpes simplex virus glycoprotein (*e.g.*, gB, gC, gD, and gE) and hepatitis B surface antigen) that is capable of eliciting an immune response. Antibodies useful in this invention for treatment of a viral infectious disease include, but are not limited to, antibodies against antigens of pathogenic viruses, including as examples and not by limitation: Poxviridae, Herpesviridae, Herpes Simplex virus 1, Herpes Simplex virus 2, Adenoviridae, Papovaviridae, Enteroviridae, Picomaviridae, Parvoviridae, Reoviridae, Retroviridae, influenza viruses, parainfluenza viruses, mumps, measles, respiratory syncytial virus, rubella, Arboviridae, Rhabdoviridae, Arenaviridae, Hepatitis A virus, Hepatitis B virus, Hepatitis C virus, Hepatitis E virus, Non-A/Non-B Hepatitis virus, Rhinoviridae, Coronaviridae, Rotoviridae, and Human Immunodeficiency Virus.

Specific examples of antibodies available useful for the treatment of a viral infectious disease include, but are not limited to, PRO542 (Progenics) which is a CD4 fusion antibody useful for the treatment of HIV infection; OSTAVIR (Protein Design Labs, Inc., CA) which is a human antibody useful for the treatment of hepatitis B virus; and PROTOVIR (Protein Design Labs, Inc., CA) which is a humanized IgG₁ antibody useful for the treatment of cytomegalovirus (CMV).

### 5.2.2. HEAT SHOCK PROTEIN INDUCERS

An HSP-inducing agent may be used in the treatment of the disorders disclosed herein as an immunomodulatory agent (preferably, an immune system enhancer). The HSP-inducing agents (otherwise referred to herein as HSP inducers) induce or increase the expression or one or more biological activities of one or more heat shock proteins known to the art, including HSP60, HSP70, HSP72, HSP80 and HSP90. HSP70 inducers useful in the present invention include, but are not limited to, prostaglandin J2, geranyl-geranyl-acetone, geldanamycin, 5-fluorouracil, cyclosporine A, sodium butyrate, glutamine, aspirin, herbimycin A, and various forms of arsenic, including arsenite and arsenic trioxide (Trisenox™). In a specific embodiment, an HSP-inducing agent induces or increases the expression or one or more of the biological activities of one or more HSPs (preferably, HSP70) 2 fold, 3 fold, 4 fold, 5 fold, 6 fold, 7 fold, 8 fold, 9 fold, 10 fold, 15 fold, 20 fold or more relative to the expression or biological activity in the absence of said agent.

In certain embodiments, one or more of the following HSP70 inducers are not used as immunomodulatory agents: prostaglandin J2, geranyl-geranyl-acetone, geldanamycin, 5-fluorouracil, cyclosporine A, sodium butyrate, glutamine, aspirin, herbimycin A, arsenite and arsenic trioxide (Trisenox™)

In another embodiment, an HSP-inducing agent does not have the chemical structure of compounds I to VI depicted in the following applications by Koya et al., entitled "Taxol Enhancer Compounds" or "Synthesis of Taxol Enhancer": U.S. patent application Serial Nos. 10/193,075, 10/193,639, and 10/193,076; and PCT Application Nos. PCT/US02/21717, PCT/US02/21714, and PCT/US02/21716, each of which is incorporated herein by reference in its entirety.

### 5.2.3. ICAM-INDUCING AGENTS

An ICAM-inducing agent may be used in the treatment of the disorders disclosed herein as an immunomodulatory agent (preferably, an immune system enhancer). The ICAM-inducing agents induce or increase the expression or one or more biological activities of one or more ICAMs known to the art, including ICAM-1. ICAM-1-inducing agents useful in the present invention include, but are not limited to, tributyrin, OK-432, retinoic acid/vitamin A, sodium butyrate, lymphotoxin-alpha and cisplatin. In a specific embodiment, an ICAM-inducing agent induces or increases the expression or one or more of the biological activities of one or more ICAMs (preferably, ICAM-1) 2 fold, 3 fold, 4 fold, 5 fold, 6 fold, 7 fold, 8 fold, 9 fold, 10 fold, 15 fold, 20 fold or more relative to the expression or biological activity in the absence of said agent.

In certain embodiments, one or more of the following ICAM-inducing agents are not used as immunomodulatory agents: tributyrin, OK-432, retinoic acid/vitamin A, sodium butyrate, lymphotoxin-alpha and cisplatin.

### 5.2.4. CHEMOKINE RECEPTOR-INDUCING AGENTS

A chemokine receptor-inducing agent (*e.g.,* CCR-7-inducing agents and CXCR5-inducing agents) may be used in the treatment of the disorders disclosed herein as an immunomodulatory agent (preferably, an immune system enhancer). The chemokine receptor-inducing agents induce or increase the expression or one or more biological activities of one or more chemokine receptors (*e.g.*, BCL-inducing agents and SCL-inducing agents) known to the art, including SCL (otherwise referred to as CCR7) and BCL (otherwise referred to as CXCR5). Examples of BCL-inducing agents include, are not limited to, lymphotoxin-alpha and CpG-containing oligonucleotides, including, but not limited to CpG 7909, CpG 8916 and CpG 8954 (Coley Pharmaceutical Group; Wellesley, MA). Examples of SCL-inducing agents include, but are not limited to, lymphotoxin-alpha In a specific embodiment, a chemokine receptor-inducing agent induces or increases the expression or one or more of the biological activities of one or more chemokine receptors (preferably, SCL or BCL) 2 fold, 3 fold, 4 fold, 5 fold, 6 fold, 7 fold, 8 fold, 9 fold, 10 fold, 15 fold, 20 fold or more relative to the expression or biological activity in the absence of said agent.

In certain embodiments, one or more of the following chemokine receptor-inducing agents are not used as immunomodulatory agents: lymphotoxin-alpha, CpG 7909, CpG 8916 and CpG 8954.

### 5.2.5. BACTERIAL AGENTS

Several bacteria or bacterial products can be utilized in the invention as immunomodulatory agents. Representative examples include, but are not limited to, lipoteichoic acid, OK-432 (a streptococcal preparation) and Bacillus Calmete-Guerin ("BCG") vaccine. In certain embodiments, the immunomodulatory agent is not OK-432 or BCG vaccine.

### 5.3. SCREENING AND IDENTIFICATION OF LYMPHOID TISSUE INDUCERS AND IMMUNOMODULATORY AGENTS

Other potential lymphoid tissue inducers or immunomodulatory agents that can advantageously be included in the compositions and methods described herein can be identified using *in vivo* and *in vitro* screening methods. Accordingly, in another aspect of the invention, methods for screening for lymphoid tissue inducers and immunomodulatory agents are provided.

In one embodiment, the invention provides a method for screening for lymphoid tissue inducers includes treating the cells or tissue in culture with the test compounds. The cells and/or tissue can first be isolated or otherwise obtained as desired. The method can include assaying by known methods for induction of the genes encoding proteins, such as those described herein, involved in inducing lymphoid tissue. For example, enzyme-linked immunosorbent assays (ELISA) can be utilized to detect the proteins produced by such genes. Moreover, RNA encoding such genes can be detected or otherwise assayed for by Northern blotting techniques known to the art. Additionally, RNA encoding such genes, or nucleic acids derived therefrom, can be detected utilizing biochip technology as known in the art and as described, for example, in U.S. Patent No. 6,040,138 to Lockhart et al. Methods of manufacturing such chips are also known in the art and are described, for example, in U.S. Patent No. 6,309,831 to Goldberg et al. Such methods for screening can be automated and otherwise adapted for high throughput screening of potential inducers.

In another embodiment, the invention provides an *in vivo* method for screening or otherwise identifying lymphoid tissue inducers includes administering a test compound to a subject and assaying for production, or the presence, of lymphoid tissue or inducers thereof as described above. Lymphoid tissue can be detected or otherwise assayed for by various methods known to the art, including observing such tissue histologically utilizing, for example, appropriate stains and suitable microscopes known to the art.

In another emobdiment, the invention provides a method for screening for immunomodulatory agents, such as immune system enhancers, comprising administering a test compound to a subject and assaying for activation of the immune system. A wide variety of assays can be utilized to determine whether the immune system has been activated, including differential white blood cell counting and quantitation of antibody titers.

A wide variety of cells and/or tissues can be utilized in the *in vitro* screening methods described herein. For example, in the methods that include assaying for induction of genes encoding proteins involved in the formation of lymphoid tissue, suitable cells include, for example, cancer cell lines, especially human cancer cell lines. Exemplary cancer cell lines include MCF-7, MDA-435, DU-145, CX-1, MX-1, LX-1, U937, EJ, CRL-1420 or other suitable cancer cell line. Other suitable cells include those that have other abnormal phenotypes, such as those from uterine fibroids and endometrial cells from individuals with endometriosis.

### 5.4. THERAPEUTIC USES

The present invention provides methods of preventing, treating, or ameliorating one or more symptoms associated with a disorder, said methods comprising administering to a subject in need of such treatment a dose of a prophylactically or therapeutically effective amount of one or more lymphoid tissue inducers and a dose of a prophylactically or therapeutically effective amount of one or more immunomodulatory agents. A wide variety of diseases can be treated according to the methods described herein. The diseases are typically those whose symptoms can be improved by, alleviated by, or whose prognostic outcome can be improved by the action of immune system components in a patient. They include diseases that can further propagate in the presence of an ineffective immune response in the patient. Such diseases include proliferative disorders, infectious diseases, cardiovascular diseases, inflammatory disorders, and autoimmune disorders.

### 5.4.1. TREATMENT OF CANCER

The present invention provides methods of preventing, treating, or ameliorating one or more symptoms associated with a proliferative disorder, said method comprising administering to a subject in need of such treatment a prophylactically or therapeutically effective amount of one or more lymphoid tissue inducers and a prophylactically or therapeutically effective amount of one or more immunomodulatory agents. In particular, the present invention provides methods of preventing, treating, or ameliorating one or more symptoms associated with a proliferative disorder, said method comprising administering to a subject in need of such treatment a prophylactically or therapeutically effective amount of one or more microtubule stabilizing agents and a prophylactically or therapeutically effective amount of one or more immunomodulatory agents. The present invention also provides methods of preventing, treating, or ameliorating one or more symptoms associated with a proliferative disorder, said method comprising administering to a subject in need of such treatment a prophylactically or therapeutically effective amount of one or more small molecules or one or more TNF-inducing agents and a prophylactically or therapeutically effective amount of one or more immunomodulatory agents.

In a specific embodiment, the present invention provides methods of preventing, treating, or ameliorating one or more symptoms associated with a proliferative disorder, said method comprising administering to a subject in need of such treatment a prophylactically or therapeutically effective amount of a taxane (*e.g.,* taxol) and a prophylactically or therapeutically effective amount of one or more immunomodulatory agents. In certain embodiments, the present invention does not include methods of preventing, treating, or ameliorating one or more symptoms associated with a proliferative disorder, said method comprising administering to a subject in need of such treatment a prophylactically or therapeutically effective amount of taxol and a prophylatically or therapeutically effective amount of one or more of the following immunomodulatory agents: 5-fluorouracil ("5-FU") or analogs thereof, cisplatin, leucovorin, mitoxantrone, doxorubicin, cyclophosphamide, carboplatin, an anthracycline, gemcitabine, epirubicin, capecitabine, isofamide, edatrexate, vinorelbine, verapramil, etoposide, hydroxyurea, folinic acid, taxotere, estramustine, GM-CSF, TNF-alpha induction, raltitrexid, pyrazoloacridine, amifostine, PS-341 (proteasome inhibitor), vinfluinine, squalamine, melphalan, cryptophycins, polyamines, herceptin, IFN-alpha, glutamine, geldenamycin or analogs thereof, PDGF antagonists, ocreotide, EGF, herbimycin A, genistein, sodium azide, dexamethasone, diphenhydramine, ranitidine, and non-steriodal anti-inflammatory drugs. In other embodiments, the present invention does not include methods of preventing, treating, or ameliorating one or more symptoms associated with a proliferative disorder, said method comprising administering to a subject in need of such treatment a prophylactically or therapeutically effective amount of taxotere and a prophylatically or therapeutically effective amount of one or more of the following immunomodulatory agents: 5-FU, doxorubicin, capecitabine and Cyt P450 Cyp1 inhibitor.

In a preferred embodiment, the present invention provides methods of preventing, treating, or ameliorating one or more symptoms associated with a proliferative disorder, said method comprising administering to a subject in need of such treatment a prophylactically or therapeutically effective amount of one or more microtubule stablizing agents, a prophylactically or therapeutically effective amount of arsenic trioxide and optionally, one or more immunomodulatory agents other than arsenic trioxide.

Examples of proliferative disorders (*i.e.,* diseases associated with abnormal, or otherwise uncontrolled, cellular proliferation) which can be treated in accordance with the methods of the invention include, but are not limited to, lung cancer, including small cell and non-small cell lung cancer; gastrointestinal cancer, including esophogeal cancer, gastric cancer, pancreatic cancer, hepatocellular cancer, colorectal cancer and anal carcinoma; genitourinary cancer, including prostate cancer, testicular cancer, bladder cancer, renal cell cancer, ovarian cancer, endometrial cancer and cervical cancer; breast cancer, neoplasms of endocrine organs, including the thyroid and parathyroid, tumors of adrenal medulla, such as pheochromocytoma and neuroblastoma; and multiple endocrine neoplasia (such as Types 1-3); hematologic cancers, including leukemia, multiple myeloma, Hodgkins disease and non-Hodgkins lymphoma; brain cancers, including central nervous system cancers such as craniopharyngeoma, pituitary neoplasms, astrocytomas, meningiomas, and spinal cord tumors; and peripheral nervous system cancers, including schwannomas and acoustic neuromas; skin cancer, including melanoma, basal cell carcinoma and squamous cell carcinoma; and cardiac tumors, such as atrial myxomas. Additional examples of proliferative disorders are listed in Table 2 below. The methods of the invention are applicable to the treatment of benign and malignant forms of the tumors described herein, as well as metastases thereof. The methods of the invention are also applicable to the treatment of proliferative diseases such as psoriasis, uterine fibroids, endometriosis, and benign prostate hyperplasia.

**TABLE 2**

| Solid tumors, including but not limited to: |
|---|
| fibrosarcoma |
| myxosarcoma |
| liposarcoma |
| chondrosarcoma |
| osteogenic sarcoma |
| chordoma |
| angiosarcoma |
| endotheliosarcoma |
| lymphangiosarcoma |
| lymphangioendotheliosarcoma |
| synovioma |
| mesothelioma |
| Ewing's tumor |
| leiomyosarcoma |
| rhabdomyosarcoma |
| colon cancer |
| colorectal cancer |
| kidney cancer |
| pancreatic cancer |
| bone cancer |
| breast cancer |
| ovarian cancer |
| prostate cancer |
| esophogeal cancer |
| stomach cancer |
| oral cancer |
| nasal cancer |
| throat cancer |
| squamous cell carcinoma |
| basal cell carcinoma |
| adenocarcinoma |
| sweat gland carcinoma |
| sebaceous gland carcinoma |
| papillary carcinoma |
| papillary adenocarcinomas |
| cystadenocarcinoma |
| medullary carcinoma |
| bronchogenic carcinoma |
| renal cell carcinoma |
| hepatoma |
| bile duct carcinoma |
| choriocarcinoma |
| seminoriza |
| embryonal carcinoma |
| Wilms' tumor |
| cervical cancer |
| uterine cancer |
| testicular cancer |
| small cell lung carcinoma |
| bladder carcinoma |
| lung cancer |
| epithelial carcinoma |
| glioma |
| glioblastoma multiforme |
| astrocytoma |
| medulloblastoma |
| craniopharyngioma |
| ependymoma |
| pinealoma |
| hemangioblastoma |
| acoustic neuroma |
| oligodendroglioma |
| meningioma |
| skin cancer |
| melanoma |
| neuroblastoma |
| retinoblastoma |
| blood-borne cancers, including but not limited to: |
| acute lymphoblastic leukemia "ALL" |
| acute lymphoblastic B-cell leukemia |
| acute lymphoblastic T-cell leukemia |
| acute myeloblastic leukemia "AML" |
| acute promyelocytic leukemia "APL" |
| acute monoblastic leukemia |
| acute erythroleukemic leukemia |
| acute megakaryoblastic leukemia |
| acute myelomonocytic leukemia |
| acute nonlymphocyctic leukemia |
| acute undifferentiated leukemia |
| chronic myelocytic leukemia "CML" |
| chronic lymphocytic leukemia "CLL" |
| hairy cell leukemia |
| multiple myeloma |
| acute and chronic leukemias: |
| lymphoblastic |
| myelogenous |
| lymphocytic |
| myelocytic leukemias |
| Lymphomas: |
| Hodgkin's disease |
| non-Hodgkin's Lymphoma |
| |
| Multiple myeloma |
| Waldenström's macroglobulinemia |
| Heavy chain disease |
| Polycythemia vera |

In a specific embodiment, the combination therapies of the invention are administered to a subject with a proliferative disorder such as cancer that is refractory to one or more chemotherapeutic agents or radiation therapy. In another embodiment, the combination therapies of the invention are used in conjunction with other types of cancer therapies including, but not limited to, surgery and radiation therapy (*e.g.*, x-ray radiation can be administered; in particular, high-energy megavoltage (radiation of greater that 1 MeV energy) can be used for deep tumors, and electron beam and orthovoltage x-ray radiation can be used for skin cancers). In accordance with this embodiment, the combination therapies of the invention can be used prior to, concurrently or subsequent to the administration of other cancer therapies such as radiation therapy and surgery.

### 5.4.2. TREATMENT OF VIRAL DISEASES

The present invention provides methods of preventing, treating, or ameliorating one or more symptoms associated with a viral disease, said method comprising administering to a subject in need of such treatment a prophylactically or therapeutically effective amount of one or more lymphoid tissue inducers and a prophylactically or therapeutically effective amount of one or more immunomodulatory agents. In particular, the present invention provides methods of preventing, treating, or ameliorating one or more symptoms associated with a viral disease, said method comprising administering to a subject in need of such treatment a prophylactically or therapeutically effective amount of one or more microtubule stabilizing agents and a prophylactically or therapeutically effective amount of one or more immunomodulatory agents. The present invention also provides methods of preventing, treating, or ameliorating one or more symptoms associated with a viral disease, said method comprising administering to a subject in need of such treatment a prophylactically or therapeutically effective amount of one or more small molecules or one or more TNF-inducing agents and a prophylactically or therapeutically effective amount of one or more immunomodulatory agents. In a specific embodiment, the immunomodulatory agents used in accordance with the methods of the invention to treat a viral disease shift the Th1 and/or Th2 response.

In a specific embodiment, the present invention provides methods of preventing, treating, or ameliorating one or more symptoms associated with a viral disease, said method comprising administering to a subject in need of such treatment a prophylactically or therapeutically effective amount of a taxane (*e.g.,* taxol) and a prophylactically or therapeutically effective amount of one or more immunomodulatory agents. In another embodiment, the present invention provides methods of preventing, treating, or ameliorating one or more symptoms associated with a viral disease, said method comprising administering to a subject in need of such treatment a prophylactically or therapeutically effective amount of one or microtubule stabilizing agents, a prophylatically or therapeutically effective amount arsenic trioxide, and optionally, a prophylactically or therapeutically effective amount of one or more immunomodulatory agents other than arsenic trioxide.

Viral diseases caused by any virus (*e.g.,* DNA-or RNA-containing virus) can be treated in accordance with the methods of the invention. Examples of viruses that can cause viral diseases include, but are not limited to, picomaviruses, including rhinoviruses, echoviruses and coxsackieviruses; orthomyxoviruses; paramyxoviruses; adenoviruses; bunyaviruses; togaviruses; rhabdoviruses; coronaviruses; herpes virus; varicella-zoster virus; cytomegalovirus; retroviruses; papovaviruses; arborviruses; arenaviruses; flavivirus; hantavirus; marburg virus; and ebola virus. Exemplary viral diseases caused by such viruses include, but are not limited to, respiratory viral diseases (*e.g.*, the common cold, influenza, and acute febrile respiratory disease), rubella, mumps, measles, rabies, conjunctivitis, herpes, chicken pox, hepatitis, central nervous system viral diseases (*e.g.,* rabies, progressive multifocal leukoencephalopathy, tropical spastic paraparesis, and prion diseases which include spongiform encephalopathies such as Creutzfeldt-Jakob disease, kuru, Gerstmann-Straussler-Scheinker disease, and fatal familial insomnia), human-T-lymphotrophic virus (HTLV), including type 1 and 2, encephalitis, yellow fever, dengue, and lymphocytic choriomeningitis.

In a specific embodiment, the combination therapies of the invention are administered to a subject with a viral disease that is refractory to one or more antiviral agents. In another embodiment, the combination therapies of the invention are used in conjunction with other types of antiviral therapies including, but not limited to, acyclovir, AZT, interferon, and amantadine. In accordance with this embodiment, the combination therapies of the invention can be used prior to, concurrently or subsequent to the administration of other antiviral therapies such as acyclovir, AZT, interferon, and amantadine. Further, in accordance with this embodiment, such other antiviral therapies do not encompass agents characterized herein as lymphoid tissue inducers and/or immunomodulatory agents.

### 5.4.3. TREATMENT OF BACTERIAL DISEASES

The present invention provides methods of preventing, treating, or ameliorating one or more symptoms associated with a bacterial disease, said method comprising administering to a subject in need of such treatment a prophylactically or therapeutically effective amount of one or more lymphoid tissue inducers and a prophylactically or therapeutically effective amount of one or more immunomodulatory agents. In particular, the present invention provides methods of preventing, treating, or ameliorating one or more symptoms associated with a bacterial disease, said method comprising administering to a subject in need of such treatment a prophylactically or therapeutically effective amount of one or more microtubule stabilizing agents and a prophylactically or therapeutically effective amount of one or more immunomodulatory agents. The present invention also provides methods of preventing, treating, or ameliorating one or more symptoms associated with a bacterial disease, said method comprising administering to a subject in need of such treatment a prophylactically or therapeutically effective amount of one or more small molecules or one or more TNF-inducing agents and a prophylactically or therapeutically effective amount of one or more immunomodulatory agents. In a specific embodiment, the immunomodulatory agents used in accordance with the methods of the invention to treat a bacterial disease shift the Th1 and/or Th2 response.

In a specific embodiment, the present invention provides methods of preventing, treating, or ameliorating one or more symptoms associated with a bacterial disease, said method comprising administering to a subject in need of such treatment a prophylactically or therapeutically effective amount of a taxane (*e.g.,* taxol) and a prophylactically or therapeutically effective amount of one or more immunomodulatory agents. In another embodiment, the present invention provides methods of preventing, treating, or ameliorating one or more symptoms associated with a bacterial disease, said method comprising administering to a subject in need of such treatment a prophylactically or therapeutically effective amount of one or more microtubule stabilizing agents, a prophylactically or therapeutically effective amount of arsenic trioxide, and optionally, a prophylactically or therapeutically effective amount of one or more immunomodulatory agents other than arsenic trioxide.

Bacterial diseases caused by any bacteria, including, but not limited to, gram positive cocci, gram negative cocci, gram positive bacilli, gram negative bacilli, spirochetes or mycobacteria can be treated in accordance with the methods of the invention. Such bacteria include, but are not limited to, those in the genus *Staphylococcus, Streptococcus, Neisseria, Bacillus, Nocardia Salmonella, Shigella, Pseudomonas, Actinomyces, Escherichia, Klebsiella, Enterobacter, Serratia, Proteus, Morganella; Providencia, Yersinia, Clostridium, Brucella, Francisella, Treponema, Streptobacillus, Mycobacterium, Mycoplasma, Chlamydia, Coxiella, Listeria, Rickettsia,* and *Erysipelothrix.* Exemplary bacteria include, but are not limited to, *Neisseria meningitidis, Neisseria gonorrhoeae, Staphylococcus aureus, Streptococcus pyogenes, Streptococcus pneumoniae, Escherichia coli, Klebsiella pneumoniae, Salmonella typhi, Salmonella typhimurium, Shigella dysenteriae, Haemophilus* *influenzae, Brucella abortus, Francisella tularensis, Pseudomonas aeruginosa, Clostridium perfringens, Clostridium tetani, Actinomyces israelii, Borrelia burgdorferi, Mycobacterium tuberculosis, Mycobacterium leprae, Bacillus anthracis, Chlamydia trachomatis, Coxiella burnetti, Rickettsia rickettsii, Mycoplasma pneuomoniae. Listeria monocytogenes* and *Erysipelothrix rhusiopathiae.* Exemplary bacterial diseases caused by such bacteria include, but are not limited to, sexually transmitted diseases (*e.g.*, gonorrhea, syphilis, cervicitis, and pelvic inflammatory disease), pneumonia, endocarditis, Q fever, rickets, osteomyelitis, toxic shock syndrome, scarlet fever, meningitis, bacteremia, peritonitis, gastroenteritis and food poisoning generally, bacterial dysentery, brucellosis, tularemia, cholera, bubonic plague, urinary tract infections, including urethritis; tetanus, actinomycosis, Rock Mountain Spotted Fever, Lyme disease, tuberculosis, anthrax, leprosy, and erysipelothricosis.

In a specific embodiment, the combination therapies of the invention are administered to a subject with a bacterial disease that is refractory to one or more antibacterial agents. In another embodiment, the combination therapies of the invention are used in conjunction with other types of antibacterial therapies including, but not limited to, penicillin, cephalosporin, imipenem, axtreonam, vancomycin, cycloserine, bacitracin, chloramphenicol, erythromycin, clindamycin, tetracycline, streptomycin, tobramycin, gentamicin, amikacin, kanamycin, neomycin, spectinomycin, trimethoprim, norfloxacin, rifampin, polymyxin, amphotericin B, nystatin, ketocanazole, isoniazid, metronidazole, and pentamidine. In accordance with this embodiment, the combination therapies of the invention can be used prior to, concurrently or subsequent to the administration of other antibacterial therapies. Further, in accordance with this embodiment, such other antibacterial therapies do not encompass agents characterized herein as lymphoid tissue inducers and/or immunomodulatory agents.

### 5.4.4. TREATMENT OF FUNGAL DISEASES

The present invention provides methods of preventing, treating, or ameliorating one or more symptoms associated with a fungal disease, said method comprising administering to a subject in need of such treatment a prophylactically or therapeutically effective amount of one or more lymphoid tissue inducers and a prophylactically or therapeutically effective amount of one or more immunomodulatory agents. In particular, the present invention provides methods of preventing, treating, or ameliorating one or more symptoms associated with a fungal disease, said method comprising administering to a subject in need of such treatment a prophylactically or therapeutically effective amount of one or more microtubule stabilizing agents and a prophylactically or therapeutically effective amount of one or more immunomodulatory agents. The present invention also provides methods of preventing, treating, or ameliorating one or more symptoms associated with a fungal disease, said method comprising administering to a subject in need of such treatment a prophylactically or therapeutically effective amount of one or more small molecules or one or more TNF-inducing agents and a prophylactically or therapeutically effective amount of one or more immunomodulatory agents. In a specific embodiment, the immunomodulatory agents used in accordance with the methods of the invention to treat a fungal disease shift the Th1 and/or the Th2 response.

In a specific embodiment, the present invention provides methods of preventing, treating, or ameliorating one or more symptoms associated with a fungal disease, said method comprising administering to a subject in need of such treatment a prophylactically or therapeutically effective amount of a taxane (*e.g.*, taxol) and a prophylactically or therapeutically effective amount of one or more immunomodulatory agents. In another embodiment, the present invention provides methods of preventing, treating, or ameliorating one or more symptoms associated with a fungal disease, said method comprising administering to a subject in need of such treatment a prophylactically or therapeutically effective amount of one or more microtubule stabilzing agents, a prophylactically or therapeutically effective amount of arsenic trioxide, and optionally, a prophylactically or therapeutically effective amount of one or more immunomodulatory agents other than arsenic trioxide.

Fungal diseases caused by any fungus can be treated in accordance with the methods of the invention. Such diseases include, but are not limited to, Coccidioidomycosis, Blastomycosis, Sporotrichosis, Cryptococcosis, Aspergillosis, Chromomycosis, Phaeohyhomycosis, Mycetoma and Mucormycosis. The causative agent of such diseases include, but are not limited to, fungi in the genus *Blastomyces,* including *Blastomyces dermatitidis; Paracoccidiodes,* including *Paracoccidioides brasiliensis;* Sporothrix, including *Sporothrix schenckii; Cryptococcus, Candida,* including *Candida albicans, Candida tropicalis* and *Candida glabrala; Aspergillus,* including *Aspergillus fumigarus* and *Aspergillus flavus; Histoplasma,* including *Histoplasma capsulatum; Cryptococcus,* including *Cryptococcus neoformans; Bipolaris, Cladophialophora, Cladosporium, Drechslera, Exophiala, Fonsecaea, Phialophora, Xylohypha, Ochroconis, Rhinocladiella, Scolecobasidium,* and *Wangiella.*

In a specific embodiment, the combination therapies of the invention are administered to a subject with a fungal disease that is refractory to one or more antifungal agents. In another embodiment, the combination therapies of the invention are used in conjunction with other types of antifungal therapies including, but not limited to, amphotericin B, nystatin, ketoconazole, fluconazole, and miconazole. In accordance with this embodiment, the combination therapies of the invention can be used prior to, concurrently or subsequent to the administration of other antifungal therapies.

Further, in accordance with this embodiment, such antifungal therapies do not encompass agents characterized herein as lymphoid tissue inducers and/or immunomodulatory agents.

### 5.4.5. TREATMENT OF CARDIOVASCULAR DISEASES

The present invention provides methods of preventing, treating, or ameliorating one or more symptoms associated with a cardiovascular disease, said method comprising administering to a subject in need of such treatment a prophylactically or therapeutically effective amount of one or more lymphoid tissue inducers and a prophylactically or therapeutically effective amount of one or more immunomodulatory agents. In particular, the present invention provides methods of preventing, treating, or ameliorating one or more symptoms associated with a cardiovascular disease, said method comprising administering to a subject in need of such treatment a prophylactically or therapeutically effective amount of one or more microtubule stabilizing agents and a prophylactically or therapeutically effective amount of one or more immunomodulatory agents. The present invention also provides methods of preventing, treating, or ameliorating one or more symptoms associated with a cardiovascular disease, said method comprising administering to a subject in need of such treatment a prophylactically or therapeutically effective amount of one or more small molecules or one or more TNF-inducing agents and a prophylactically or therapeutically effective amount of one or more immunomodulatory agents.

In a specific embodiment, the present invention provides methods of preventing, treating, or ameliorating one or more symptoms associated with a cardiovascular disease, said method comprising administering to a subject in need of such treatment a prophylactically or therapeutically effective amount of a taxane (*e.g.,* taxol) and a prophylactically or therapeutically effective amount of one or more immunomodulatory agents. In another embodiment, the present invention provides methods of preventing, treating, or ameliorating one or more symptoms associated with a cardiovascular disease, said method comprising administering to a subject in need of such treatment a prophylactically or therapeutically effective amount of one or more microtubule stabilizing agents, a prophylactically or therapeutically effective amount of arsenic trioxide, and optionally, a prophylactically or therapeutically effective amount of one or more immunomodulatory agents other than arsenic trioxide.

Any cardiovascular disease can be treated in accordance with the methods of the invention. Examples of cardiovascular diseases include, but not limited to, athlerosclerosis, stroke, cerebral infarction, endothelium dysfunctions (in particular, those dysfunctions affecting blood vessel elasticity) ischemic heart disease (*e.g.,* angina pectoris, myocardial infarction, and chronic ischemic heart disease), hypertensive heart disease, pulmonary heart disease, coronary heart disease, valvular heart disease (*e*.*g*., rheumatic fever and rheumatic heart disease, endocarditis, mitral valve prolapse, restenosis and aortic valve stenosis), congenital heart disease (e.g., valvular and vascular obstructive lesions, atrial or ventricular septal defect, and patent ductus arteriosus), and myocardial disease (*e.g.*, myocarditis, congestive cardiomyopathy, and hypertrophic cariomyopathy).

In a specific embodiment, the combination therapies of the invention are administered to a subject with a cardiovascular disease that is refractory to one or more cardiovascular drugs. In another embodiment, the combination therapies of the invention are used in conjunction with other types of cardiovascular drugs including, but not limited to, peripheral antiadrenergic drugs, centrally acting antihypertensive drugs (*e.g.,* methyldopa, methyldopa HCl), antihypertensive direct vasodilators (*e.g.*, diazoxide, hydralazine HCl), drugs affecting renin-angiotensin system, peripheral vasodilators, phentolamine, antianginal drugs, cardiac glycosides, inodilators (*e.g*.; amrinone, milrinone, enoximone, fenoximone, imazodan, sulmazole), antidysrhythmic drugs, calcium entry blockers, ranitine, bosentan, and rezulin. In accordance with this embodiment, the combination therapies of the invention can be used prior to, concurrently or subsequent to the administration of such cardiovascular drugs. Further, in accordance with this embodiment, such cardiovascular drugs do not encompass agents characterized herein as lymphoid tissue inducers and/or immunomodulatory agents.

### 5.4.6. TREATMENT OF INFLAMMATORY DISORDERS

The present invention provides methods of preventing, treating, or ameliorating one or more symptoms associated with an inflammatory disorder, said method comprising administering to a subject in need of such treatment a prophylactically or therapeutically effective amount of one or more lymphoid tissue inducers and a prophylactically or therapeutically effective amount of one or more immunomodulatory agents. In particular, the present invention provides methods of preventing, treating, or ameliorating one or more symptoms associated with an inflammatory disorder, said method comprising administering to a subject in need of such treatment a prophylactically or therapeutically effective amount of one or more microtubule stabilizing agents and a prophylactically or therapeutically effective amount of one or more immunomodulatory agents. The present invention also provides methods of preventing, treating, or ameliorating one or more symptoms associated with an inflammatory disorder, said method comprising administering to a subject in need of such treatment a prophylactically or therapeutically effective amount of one or more small molecules or one or more TNF-inducing agents and a prophylactically or therapeutically effective amount of one or more immunomodulatory agents. In a specific embodiment, the immunomodulatory agents used in accordance with the methods of the invention to treat an inflammatory disorder shift the Th1 and/or Th2 response, more preferably said agents shift the Th2 response to a Th1 response.

In a specific embodiment, the present invention provides methods of preventing, treating, or ameliorating one or more symptoms associated with an inflammatory disorder, said method comprising administering to a subject in need of such treatment a prophylactically or therapeutically effective amount of a taxane (*e.g.*, taxol) and a prophylactically or therapeutically effective amount of one or more immunomodulatory agents. In another embodiment, the present invention provides methods of preventing, treating, or ameliorating one or more symptoms associated with an inflammatory disorder, said method comprising administering to a subject in need of such treatment a prophylactically or therapeutically effective amount of one or more microtubule stabilizing agents, a prophylactically or therapeutically effective amount of arsenic trioxide, and optionally, a prophylactically or therapeutically effective amount of one or more immunomodulatory agents other than arsenic trioxide.

Any inflammatory disorder can be treated in accordance with the methods of the invention. Examples of inflammatory disorders include, but are not limited to, asthma, encephilitis, inflammatory bowel disease (*e.g.*, Crohn's disease and ulcerative colitis), chronic obstructive pulmonary disease (COPD), inflammatory osteolysis, allergic disorders, septic shock, pulmonary fibrosis (*e.g.,* idiopathic pulmonary fibrosis), inflammatory vaculitides (*e.g.,* polyarteritis nodosa, Wegner's granulomatosis, Takayasu's arteritis, temporal arteritis, and lymphomatoid granulomatosus), post-traumatic vacular angioplasty (*e.g.*, restenosis after angioplasty), undifferentitated spondyloarthropathy, undifferentiated arthropathy, arthritis, inflammatory osteolysis, chronic hepatitis, and chronic inflammation resulting from chronic viral or bacteria infections.

In a specific embodiment, the combination therapies of the invention are administered to a subject with an inflammatory disorder that is refractory to one or more anti-inflammatory agents. In another embodiment, the combination therapies of the invention are used in conjunction with other types of anti-inflammatory agents including, but not limited to, non-steroidal anti-inflammatory drugs (NSAIDs), steroidal anti-inflammatory drugs, beta-agonists, anticholingeric agents, and methyl xanthines. Examples of NSAIDs include, but are not limited to, ibuprofen, celecoxib (CELEBREX), diclofenac (VOLTAREN), etodolac (LODINE™), fenoprofen (NALFON), indomethacin (INDOCIN), ketoralac (TORADOL), oxaprozin (DAYPRO), nabumentone (RELAFEN), sulindac (CLINORIL), tolmentin (TOLECTIN), rofecoxib (VIOXX), naproxen (ALEVE, NAPROSYN), ketoprofen (ACTRON) and nabumetone (RELAFEN). Such NSAIDs function by inhibiting a cyclooxgenase enzyme (*e.g.,* COX-1 and/or COX-2). Examples of steroidal anti-inflammatory drugs include, but are not limited to, glucocorticoids, dexamethasone (DECADRON), cortisone, hydrocortisone, prednisone (DELTASONE), prednisolone, triamcinolone, azulfidine, and eicosanoids such as thromboxanes, and leukotrienes. In accordance with the above embodiment, the combination therapies of the invention can be used prior to, concurrently or subsequent to the administration of such anti-inflammatory agents. Further, such anti-inflammatory agents do not encompass agents characterized herein as lymphoid tissue inducers and/or immunomodulatory agents.

### 5.4.7 TREATMENT OF AUTOIMMUNE DISEASES

The present invention provides methods of preventing, treating, or ameliorating one or more symptoms associated with an autoimmune disorder, said method comprising administering to a subject in need of such treatment a prophylactically or therapeutically effective amount of one or more lymphoid tissue inducers and a prophylactically or therapeutically effective amount of one or more immunomodulatory agents. In particular, the present invention provides methods of preventing, treating, or ameliorating one or more symptoms associated with an autoimmune disorder, said method comprising administering to a subject in need of such treatment a prophylactically or therapeutically effective amount of one or more microtubule stabilizing agents and a prophylactically or therapeutically effective amount of one or more immunomodulatory agents. The present invention also provides methods of preventing, treating, or ameliorating one or more symptoms associated with an autoimmune disorder, said method comprising administering to a subject in need of such treatment a prophylactically or therapeutically effective amount of one or more small molecules or one or more microtubule stabilizing agents and a prophylactically or therapeutically effective amount of one or more immunomodulatory agents. In a specific embodiment, the immunomodulatory agents used in accordance with the invention to treat an autoimmune disorder shift the Th1 and/or Th2 response, preferably said agents shift the Th1 response to a Th2 response.

In a specific embodiment, the present invention provides methods of preventing, treating, or ameliorating one or more symptoms associated with an autoimmune disorder, said method comprising administering to a subject in need of such treatment a prophylactically or therapeutically effective amount of a taxane (*e.g.*, taxol) and a prophylactically or therapeutically effective amount of one or more immunomodulatory agents. In another embodiment, the present invention provides methods of preventing, treating, or ameliorating one or more symptoms associated with an autoimmune disorder, said method comprising administering to a subject in need of such treatment a prophylactically or therapeutically effective amount of one or more microtubule stabilizing agents, a prophylactically or therapeutically effective amount of arsenic trioxide, and optionally, a prophylactically or therapeutically effective amount of one or more immunomodulatory agents other than arsenic trioxide.

Any autoimmune disorder can be treated in accordance with the methods of the invention. Examples of autoimmune disorders include, but not limited to, alopecia areata, ankylosing spondylitis, antiphospholipid syndrome, autoimmune Addison's disease, autoimmune diseases of the adrenal gland, autoimmune hemolytic anemia, autoimmune hepatitis, autoimmune oophoritis and orchitis, autoimmune thrombocytopenia, Behcet's disease, bullous pemphigoid, cardiomyopathy, celiac sprue-dermatitis, chronic fatigue immune dysfunction syndrome (CFIDS), chronic inflammatory demyelinating polyneuropathy, Churg-Strauss syndrome, cicatrical pemphigoid, CREST syndrome, cold agglutinin disease, Crohn's disease, discoid lupus, essential mixed cryoglobulinemia, fibromyalgia-fibromyositis, glomerulonephritis, Graves' disease, Guillain-Barre, Hashimoto's thyroiditis, idiopathic pulmonary fibrosis, idiopathic thrombocytopenia purpura (ITP), IgA neuropathy, juvenile arthritis, lichen planus, Ménière's disease, mixed connective tissue disease, multiple sclerosis, type 1 or immune-mediated diabetes mellitus, myasthenia gravis, pemphigus vulgaris, pernicious anemia, polyarteritis nodosa, polychrondritis, polyglandular syndromes, polymyalgia rheumatica, polymyositis and dermatomyositis, primary agammaglobulinemia, primary biliary cirrhosis, psoriasis, psoriatic arthritis, Raynauld's phenomenon, Reiter's syndrome, Rheumatoid arthritis, sarcoidosis, scleroderma, progressive systemic sclerosis, Sjögren's syndrome, Good pasture's syndrome, stiff-man syndrome, systemic lupus erythematosus, lupus erythematosus, takayasu arteritis, temporal arteristis/ giant cell arteritis, ulcerative colitis, uveitis, vasculitides such as dermatitis herpetiformis vasculitis, vitiligo, and Wegener's granulomatosis.

In a specific embodiment, the combination therapies of the invention are administered to a subject with an autoimmune disorder that is refractory to one or more autoimmune therapies. In another embodiment, the combination therapies of the invention are used in conjunction with other types of autoimmune therapies. In accordance with this embodiment, the combination therapies of the invention can be used prior to, concurrently or subsequent to the administration of such autoimmune therapies. Further, such autoimmune therapies do not encompass agents characterized herein as lymphoid tissue inducers and/or immunomodulatory agents.

### 5.5. COMPOSITIONS AND METHODS OF ADMINISTERING THERAPIES

The present invention provides compositions for the treatment, prophylaxis, and amelioration of one or more symptoms associated with a disorder in which modulation of a subject's immune system is beneficial such as a proliferative disorder, an infectious disease, a cardiovascular disease, an inflammatory disorder, and an autoimmune disorder. In a specific embodiment, a composition comprises one or more lymphoid tissue inducers (*e.g.,* one or more microtubule stabilizing agents, one or more TNF-inducing agents, or one or more small molecules). In another embodiment, a composition comprises one or more immunomodulatory agents (preferably, immune system enhancers). In another embodiment, a composition comprises one or more microtubule stabilizing agents and one or more immunomodulatory agents (preferably, immune system enhancers). In other embodiments, a composition can include a lymphoid tissue inducer and at least two immunomodulatory agents (preferably, immune system enhancers), and as many as three, four or more as desired and depending on the case.

In a preferred embodiment, a composition of the invention is a pharmaceutical composition. Such compositions comprise a prophylactically or therapeutically effective amount of one or more prophylactic or therapeutic agents, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant (*e.g.*, Freund's adjuvant (complete and incomplete)), excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in ''Remington's Pharmaceutical Sciences" by E.W. Martin. Such compositions will contain a prophylactically or therapeutically effective amount of a prophylactic or therapeutic agent preferably in purified form, together with a suitable amount of carrier so as to provide the form for proper administration to the patient. The formulation should suit the mode of administration. In a preferred embodiment, the pharmaceutical compositions are sterile and in suitable form for administration to a subject, preferably an animal subject, more preferably a mammalian subject, and most preferably a human subject.

Various delivery systems are known and can be used to administer one or more prophylactic or therapeutic agents, *e.g.*, formulating with a pharmaceutically acceptable carrier, encapsulation in liposomes, microparticles, microcapsules, recombinant cells capable of expressing the prophylactic or therapeutic agents, receptor-mediated endocytosis (see, e.g., Wu and Wu, J. Biol. Chem. 262:4429-4432 (1987)), construction of a nucleic acid as part of a retroviral or other vector, etc. Methods of administering a prophylactic or therapeutic agent, or pharmaceutical composition comprising a prophylactic or therapeutic agent include, but are not limited to, parenteral administration (*e.g.,* intradermal, intramuscular, intraperitoneal, intravenous and subcutaneous), epidural, topically, mucosal (*e.g*., intranasal and oral routes) and rectal. In a specific embodiment, a lymphoid tissue inducer and/or an immunomodulatory agent (preferably, an immune system enhancer), or a pharmaceutical composition is administered intramuscularly, subcutaneously, orally or intravenously. The compositions may be administered by any convenient route, for example by infusion or bolus injection, by absorption through epithelial or mucocutaneous linings (*e.g.,* oral mucosa, rectal and intestinal mucosa, etc.) and may be administered together with other biologically active agents. Administration can be systemic or local.

In a specific embodiment, it may be desirable to administer the pharmaceutical compositions of the invention locally to the area in need of treatment; this may be achieved by, for example, and not by way of limitation, local infusion, by injection, or by means of an implant, said implant being of a porous, non-porous, or gelatinous material, including membranes, such as sialastic membranes, or fibers. Preferably, when administering a prophylactic or therapeutic agent, care must be taken to use materials to which the prophylactic or therapeutic agent does not absorb.

In another embodiment, the composition can be delivered in a vesicle, in particular a liposome (see Langer, Science 249:1527-1533 (1990); Treat *et al.,* in Liposomes in the Therapy of Infectious Disease and Cancer, Lopez-Berestein and Fidler (eds.), Liss, New York, pp. 353- 365 (1989); Lopez-Berestein, ibid., pp. 3 17-327; see generally ibid.).

In yet another embodiment, the composition can be delivered in a controlled release or sustained release system. In one embodiment, a pump may be used to achieve controlled or sustained release (see Langer, supra; Sefton, 1987, CRC Crit. Ref. Biomed. Eng. 14:20; Buchwald et al., 1980, Surgery 88:507; Saudek et al., 1989, N. Engl. J. Med. 321:574). In another embodiment, polymeric materials can be used to achieve controlled or sustained release of the antibodies of the invention or fragments thereof (see *e.g.,* Medical Applications of Controlled Release, Langer and Wise (eds.), CRC Pres., Boca Raton, Florida (1974); Controlled Drug Bioavailability, Drug Product Design and Performance, Smolen and Ball (eds.), Wiley, New York (1984); Ranger and Peppas, 1983, J., Macromol. Sci. Rev. Macromol. Chem. 23:61; see also Levy et al., 1985, Science 228:190; During et al., 1989, Ann. Neurol. 25:351; Howard et al., 1989, J. Neurosurg. 71:105); U.S. Patent No. 5,679,377; U.S. Patent No. 5,916,597; U.S. Patent No. 5,912,015; U.S. Patent No. 5,989,463; U.S. Patent No. 5,128,326; PCT Publication No. WO 99/15154; and PCT Publication No. WO 99/20253. Examples of polymers used in sustained release formulations include, but are not limited to, poly(2-hydroxy ethyl methacrylate), poly(methyl methacrylate), poly(acrylic acid), poly(ethylene-co-vinyl acetate), poly(methacrylic acid), polyglycolides (PLG), polyanhydrides, poly(N-vinyl pyrrolidone), poly(vinyl alcohol), polyacrylamide, poly(ethylene glycol), polylactides (PLA), poly(lactide-co-glycolides) (PLGA), and polyorthoesters. In a preferred embodiment, the polymer used in a sustained release formulation is inert, free of leachable impurities, stable on storage, sterile, and biodegradable. In yet another embodiment, a controlled or sustained release system can be placed in proximity of the therapeutic target, *e.g.,* a tumor, thus requiring only a fraction of the systemic dose (see, *e.g.,* Goodson, in Medical Applications of Controlled Release, supra, vol. 2, pp. 115-138 (1984)).

Controlled release systems are discussed in the review by Langer (1990, Science 249:1527-1533). Any technique known to one of skill in the art can be used to produce sustained release formulations comprising one or more antibodies of the invention or fragments thereof. See, *e.g.*,.U.S. Patent No. 4,526,938, PCT publication WO 91/05548, PCT publication WO 96/20698, Ning *et al.,* 1996, "Intratumoral Radioimmunotheraphy of a Human Colon Cancer Xenograft Using a Sustained-Release Gel," Radiotherapy & Oncology 39:179-189, Song *et al.,* 1995, "Antibody Mediated Lung Targeting of Long-Circulating Emulsions," PDA Journal of Pharmaceutical Science & Technology 50:372-397, Cleek *et al.,* 1997, "Biodegradable Polymeric Carriers for a bFGF Antibody for Cardiovascular Application," Pro. Int'I. Symp. Control. Rel. Bioact. Mater. 24:853-854, and Lam *et al.,* 1997, "Microencapsulation of Recombinant Humanized Monoclonal Antibody for Local Delivery," Proc. Int'1. Symp. Control Rel. Bioact. Mater. 24:759-760, each of which is incorporated herein by reference in their entirety.

In a specific embodiment where the composition of the invention is a nucleic acid encoding a prophylactic or therapeutic agent, the nucleic acid can be administered *in vivo* to promote expression of its encoded prophylactic or therapeutic agent, by constructing it as part of an appropriate nucleic acid expression vector and administering it so that it becomes intracellular, *e.g.,* by use of a retroviral vector (see U.S. Patent No. 4,980,286), or by direct injection, or by use of microparticle bombardment (e.g., a gene gun; Biolistic, Dupont), or coating with lipids or cell-surface receptors or transfecting agents, or by administering it in linkage to a homeobox-like peptide which is known to enter the nucleus (see e.g., Joliot et at., 1991, Proc. Natl. Acad. Sci. USA 88:1864-1868), etc. Alternatively, a nucleic acid can be introduced intracellularly and incorporated within host cell DNA for expression by homologous recombination.

A pharmaceutical composition of the invention is formulated to be compatible with its intended route of administration. Examples of routes of administration include, but are not limited to, parenteral, *e.g.,* intravenous, intradermal, subcutaneous, oral (*e.g.*, inhalation), intranasal, transdermal (topical), transmucosal, and rectal administration. In a specific embodiment, the composition is formulated in accordance with routine procedures as a pharmaceutical composition adapted for intravenous, subcutaneous, intramuscular, oral, intranasal or topical administration to human beings. Typically, compositions for intravenous administration are solutions in sterile isotonic aqueous buffer. Where necessary, the composition may also include a solubilizing agent and a local anesthetic such as lignocamne to ease pain at the site of the injection.

If the compositions of the invention are to be administered topically, the compositions can be formulated in the form of, *e.g.,* an ointment, cream, transdermal patch, lotion, gel, shampoo, spray, aerosol, solution, emulsion, or other form well-known to one of skill in the art. See, *e.g.,* Remington's Pharmaceutical Sciences and Introduction to Pharmaceutical Dosage Forms, 4^{th} ed., Lea & Febiger, Philadelphia, PA (1985). For non-sprayable topical dosage forms, viscous to semi-solid or solid forms comprising a carrier or one or more excipients compatible with topical application and having a dynamic viscosity preferably greater than water are typically employed. Suitable formulations include, without limitation, solutions, suspensions, emulsions, creams, ointments, powders, liniments, salves, and the like, which are, if desired, sterilized or mixed with auxiliary agents (e.g., preservatives, stabilizers, wetting agents, buffers, or salts) for influencing various properties, such as, for example, osmotic pressure. Other suitable topical dosage forms include sprayable aerosol preparations wherein the active ingredient, preferably in combination with a solid or liquid inert carrier, is packaged in a mixture with a pressurized volatile (*e.g.*, a gaseous propellant, such as freon), or in a squeeze bottle. Moisturizers or humectants can also be added to pharmaceutical compositions and dosage forms if desired. Examples of such additional ingredients are well-known in the art.

If the compositions of the invention are to be administered intranasally, the compositions can be formulated in an aerosol form, spray, mist or in the form of drops. In particular, prophylactic or therapeutic agents for use according to the present invention can be conveniently delivered in the form of an aerosol spray presentation from pressurized packs or a nebuliser, with the use of a suitable propellant, *e.g.,* dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of, *e.g.,* gelatin for use in an inhaler or insufflator may be formulated containing a powder mix of the compound and a suitable powder base such as lactose or starch.

If the compositions of the invention are to be administered orally, the compositions can be formulated orally in the form of, *e.g.,* tablets, capsules, cachets, gelcaps, solutions, suspensions and the like. Tablets or capsules can be prepared by conventional means with pharmaceutically acceptable excipients such as binding agents (*e.g.,* pregelatinised maize starch, polyvinylpyrrolidone or hydroxypropyl methylcellulose); fillers (*e.g.,* lactose, microcrystalline cellulose or calcium hydrogen phosphate); lubricants (*e.g.,* magnesium stearate, talc or silica); disintegrants (*e.g.,* potato starch or sodium starch glycolate); or wetting agents (*e.g.*, sodium lauryl sulphate). The tablets may be coated by methods well-known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (*e.g.*, sorbitol syrup, cellulose derivatives or hydrogenated edible fats); emulsifying agents (*e.g.*, lecithin or acacia); non-aqueous vehicles (*e.g.,* almond oil, oily esters, ethyl alcohol or fractionated vegetable oils); and preservatives (*e.g.,* methyl or propyl-p-hydroxybenzoates or sorbic acid). The preparations may also contain buffer salts, flavoring, coloring and sweetening agents as appropriate. Preparations for oral administration may be suitably formulated for slow release, controlled release or sustained release of a prophylactic or therapeutic agent(s).

The compositions of the invention may be formulated for parenteral administration by injection, *e.g.*, by bolus injection or continuous infusion. Formulations for injection may be presented in unit dosage form, *e.g.,* in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g., sterile pyrogen-free water, before use.

The compositions of the invention may also be formulated in rectal compositions such as suppositories or retention enemas, *e.g.*, containing conventional suppository bases such as cocoa butter or other glycerides.

In addition to the formulations described previously, the compositions of the invention may also be formulated as a depot preparation. Such long acting formulations may be administered by implantation (for example subcutaneously or intramuscularly) or by intramuscular injection. Thus, for example, the compositions may be formulated with suitable polymeric or hydrophobic materials (for example as an emulsion in an acceptable oil) or ion exchange resins, or as sparingly soluble derivatives, for example, as a sparingly soluble salt.

The compositions of the invention can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

Generally, the ingredients of compositions of the invention are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

In particular, the invention provides that one or more of the prophylactic or therapeutic agents, or pharmaceutical compositions of the invention is packaged in a hermetically sealed container such as an ampoule or sachette indicating the quantity of the agent. In one embodiment, one or more of the prophylactic or therapeutic agents, or pharmaceutical compositions of the invention is supplied as a dry sterilized lyophilized powder or water free concentrate in a hermetically sealed container and can be reconstituted, *e.g.*, with water or saline to the appropriate concentration for administration to a subject. Preferably, one or more of the prophylactic or therapeutic agents, or pharmaceutical compositions of the invention is supplied as a dry sterile lyophilized powder in a hermetically sealed container at a unit dosage of at least 5 mg, more preferably at least 10 mg, at least 15 mg, at least 25 mg, at least 35 mg, at least 45 mg, at least 50 mg, at least 75 mg, or at least 100 mg. The lyophilized prophylactic or therapeutic agents, or pharmaceutical compositions of the invention should be stored at between 2 and 8 C in its original container and the prophylactic or therapeutic agents, or pharmaceutical compositions of the invention should be administered within 1 week, preferably within 5 days, within 72 hours, within 48 hours, within 24 hours, within 12 hours, within 6 hours, within 5 hours, within 3 hours, or within 1 hour after being reconstituted. In an alternative embodiment, one or more of the prophylactic or therapeutic agents, or pharmaceutical compositions of the invention is supplied in liquid form in a hermetically sealed container indicating the quantity and concentration of the agent. Preferably, the liquid form of the administered composition is supplied in a hermetically sealed container at least 0.25 mg/ml, more preferably at least 0.5 mg/ml, at least 1 mg/ml, at least 2.5 mg/ml, at least 5 mg/ml, at least 8 mg/ml, at least 10 mg/ml, at least 15 mg/ml, at least 25 mg/ml, at least 50 mg/ml, at least 75 mg/ml or at least 100 mg/ml. The liquid form should be stored at between 2°C and 8°C in its original container.

### 5.5.1. GENE THERAPY

In a specific embodiment, nucleic acids comprising sequences encoding one or more prophylactic or therapeutic agents, are administered to treat, prevent or ameliorate one or more symptoms associated with an inflammatory or autoimmune disease, by way of gene therapy. Gene therapy refers to therapy performed by the administration to a subject of an expressed or expressible nucleic acid. In this embodiment of the invention, the nucleic acids produce their encoded prophylactic or therapeutic agent that mediates a prophylactic or therapeutic effect.

Any of the methods for gene therapy available in the art can be used according to the present invention. Exemplary methods are described below.

For general reviews of the methods of gene therapy, see Goldspiel et al., 1993, Clinical Pharmacy 12:488-505; Wu and Wu, 1991, Biotherapy 3:87-95; Tolstoshev, 1993, Ann. Rev. Pharmacol. Toxicol. 32:573-596; Mulligan, Science 260:926-932 (1993); and Morgan and Anderson, 1993, Ann. Rev. Biochem. 62:191-217; May, 1993, TIBTECH 11(5):155-215. Methods commonly known in the art of recombinant DNA technology which can be used are described in Ausubel et al. (eds.), Current Protocols in Molecular Biology, John Wiley & Sons, NY (1993); and Kriegler, Gene Transfer and Expression, A Laboratory Manual, Stockton Press, NY (1990).

In a preferred aspect, a composition of the invention comprises nucleic acids encoding a prophylactic or therapeutic agent, said nucleic acids being part of an expression vector that expresses the prophylactic or therapeutic agent in a suitable host. In particular, such nucleic acids have promoters, preferably heterologous promoters, operably linked to the antibody coding region, said promoter being inducible or constitutive, and, optionally, tissue-specific. In another particular embodiment, nucleic acid molecules are used in which the prophylactic or therapeutic agent coding sequences and any other desired sequences are flanked by regions that promote homologous recombination at a desired site in the genome, thus providing for intrachromosomal expression of the antibody encoding nucleic acids (Koller and Smithies, 1989, Proc. Natl. Acad. Sci. USA 86:8932-8935; Zijlstra et al., 1989, Nature 342:435-438). In certain embodiments, the prophylactic or therapeutic agent expressed.

Delivery of the nucleic acids into a subject may be either direct, in which case the subject is directly exposed to the nucleic acid or nucleic acid-carrying vectors, or indirect, in which case, cells are first transformed with the nucleic acids in vitro, then transplanted into the subject. These two approaches are known, respectively, as *in vivo* or *ex vivo* gene therapy.

In a specific embodiment, the nucleic acid sequences are directly administered *in vivo,* where it is expressed to produce the encoded product. This can be accomplished by any of numerous methods known in the art, *e.g.,* by constructing them as part of an appropriate nucleic acid expression vector and administering it so that they become intracellular, *e.g.,* by infection using defective or attenuated retrovirals or other viral vectors (see U.S. Patent No. 4,980,286), or by direct injection of naked DNA, or by use of microparticle bombardment (*e.g.,* a gene gun; Biolistic, Dupont), or by a matrix with *in situ* scaffolding in which the nucleic acid sequence is contained (see, *e.g.*, European Patent No. EP 0 741 785 B1 and U.S. Patent No. 5,962,427), or coating with lipids or cell-surface receptors or transfecting agents, encapsulation in liposomes, microparticles, or microcapsules, or by administering them in linkage to a peptide which is known to enter the nucleus, by administering it in linkage to a ligand subject to receptor-mediated endocytosis (see, *e.g.*, Wu and Wu, 1987, J. Biol. Chem. 262:4429-4432) (which can be used to target cell types specifically expressing the receptors), etc. In another embodiment, nucleic acid-ligand complexes can be formed in which the ligand comprises a fusogenic viral peptide to disrupt endosomes, allowing the nucleic acid to avoid lysosomal degradation. In yet another embodiment, the nucleic acid can be targeted *in vivo* for cell specific uptake and expression, by targeting a specific receptor (see, *e.g.,* PCT Publication Nos. WO 92/06180, WO 92/22635, WO 92/20316, WO 93/14188, and WO 93/20221). Alternatively, the nucleic acid can be introduced intracellularly and incorporated within host cell DNA for expression, by homologous recombination (Koller and Smithies, 1989, Proc. Natl. Acad. Sci. USA 86:8932-8935; and Zijlstra et al., 1989, Nature 342:435-438).

In a specific embodiment, viral vectors that contain nucleic acid sequences encoding a prophylactic or therapeutic agent are used. For example, a retroviral vector can be used (*see, e.g.*, Miller et al., 1993, Meth. Enzymol. 217:581-599). These retroviral vectors contain the components necessary for the correct packaging of the viral genome and integration into the host cell DNA. The nucleic acid sequences encoding a prophylactic or therapeutic agent to be used in gene therapy are cloned into one or more vectors, which facilitates delivery of the gene into a subject. More detail about retroviral vectors can be found in Boesen et al., 1994, Biotherapy 6:291-302, which describes the use of a retroviral vector to deliver the mdr I gene to hematopoietic stem cells in order to make the stem cells more resistant to chemotherapy. Other references illustrating the use of retroviral vectors in gene therapy are: Clowes et al., 1994, J. Clin. Invest. 93:644-651; Klein et al., 1994, Blood 83:1467-1473; Salmons and Gunzberg, 1993, Human Gene Therapy 4:129-141; and Grossman and Wilson, 1993, Curr. Opin. in Genetics and Devel. 3:110-114.

Adenoviruses are other viral vectors that can be used in gene therapy. Adenoviruses are especially attractive vehicles for delivering genes to respiratory epithelia. Adenoviruses naturally infect respiratory epithelia where they cause a mild disease. Other targets for adenovirus-based delivery systems are liver, the central nervous system, endothelial cells, and muscle. Adenoviruses have the advantage of being capable of infecting non-dividing cells. Kozarsky and Wilson, 1993, Current Opinion in Genetics and Development 3:499-503 present a review of adenovirus-based gene therapy. Bout et al., 1994, Human Gene Therapy 5:3-10 demonstrated the use of adenovirus vectors to transfer genes to the respiratory epithelia of rhesus monkeys. Other instances of the use of adenoviruses in gene therapy can be found in Rosenfeld et al., 1991, Science 252:431-434; Rosenfeld et al., 1992, Cell 68:143-155; Mastrangeli et al., 1993, J. Clin. Invest. 91:225-234; PCT Publication W094/12649; and Wang et al., 1995, Gene Therapy 2:775-783. In a preferred embodiment, adenovirus vectors are used.

Adeno-associated virus (AAV) has also been proposed for use in gene therapy (Walsh et al., 1993, Proc. Soc. Exp. Biol. Med. 204:289-300; and U.S. Patent No. 5,436,146).

Another approach to gene therapy involves transferring a gene to cells in tissue culture by such methods as electroporation, lipofection, calcium phosphate mediated transfection, or viral infection. Usually, the method of transfer includes the transfer of a selectable marker to the cells. The cells are then placed under selection to isolate those cells that have taken up and are expressing the transferred gene. Those cells are then delivered to a subject.

In this embodiment, the nucleic acid is introduced into a cell prior to administration *in vivo* of the resulting recombinant cell. Such introduction can be carried out by any method known in the art, including but not limited to transfection, electroporation, microinjection, infection with a viral or bacteriophage vector containing the nucleic acid sequences, cell fusion, chromosome-mediated gene transfer, microcellmediated gene transfer, spheroplast fusion, etc. Numerous techniques are known in the art for the introduction of foreign genes into cells (see, *e.g.,* Loeffler and Behr, 1993, Meth. Enzymol. 217:599-618; Cohen et al., 1993, Meth. Enzymol. 217:618-644; Clin. Pharma. Ther. 29:69-92 (1985)) and may be used in accordance with the present invention, provided that the necessary developmental and physiological functions of the recipient cells are not disrupted. The technique should provide for the stable transfer of the nucleic acid to the cell, so that the nucleic acid is expressible by the cell and preferably heritable and expressible by its cell progeny.

The resulting recombinant cells can be delivered to a subject by various methods known in the art. Recombinant blood cells (*e.g.*, hematopoietic stem or progenitor cells) are preferably administered intravenously. The amount of cells envisioned for use depends on the desired effect, patient state, etc., and can be determined by one skilled in the art.

Cells into which a nucleic acid can be introduced for purposes of gene therapy encompass any desired, available cell type, and include but are not limited to epithelial cells, endothelial cells, keratinocytes, fibroblasts, muscle cells, hepatocytes; blood cells such as T lymphocytes, B lymphocytes, natural killer (NK) cells, monocytes, macrophages, neutrophils, eosinophils, megakaryocytes, granulocytes; various stem or progenitor cells, in particular hematopoietic stem or progenitor cells, *e.g.*, as obtained from bone marrow, umbilical cord blood, peripheral blood, fetal liver, etc.

In a preferred embodiment, the cell used for gene therapy is autologous to the subject.

In an embodiment in which recombinant cells are used in gene therapy, nucleic acid sequences encoding a prophylactic or therapeutic agent are introduced into the cells such that they are expressible by the cells or their progeny, and the recombinant cells are then administered *in vivo* for prophylactic or therapeutic effect. In a specific embodiment, stem or progenitor cells are used. Any stem and/or progenitor cells which can be isolated and maintained *in vitro* can potentially be used in accordance with this embodiment of the present invention (see *e.g.,* PCT Publication No. WO 94/08598; Stemple and Anderson, 1992, Cell 7 1:973-985; Rheinwald, 1980, Meth. Cell Bio. 21A:229; and Pittelkow and Scott, 1986, Mayo Clinic Proc. 61:771).

In a specific embodiment, the nucleic acid to be introduced for purposes of gene therapy comprises a constitutive, tissue-specific, or inducible promoter operably linked to the coding region. In a preferred embodiment, the nucleic acid to be introduced for purposes of gene therapy comprises an inducible promoter operably linked to the coding region, such that expression of the nucleic acid is controllable by controlling the presence or absence of the appropriate inducer of transcription.

### 5.6. DOSAGES & FREQUENCY

The prophylactically or therapeutically effective amount of a composition of the invention which will be effective in the treatment, prevention or amelioration of one or more symptoms associated with a disorder (*e.g.*, a proliferative disorder, an infectious disease, a cardiovascular disease, an inflammatory disease or an autoimmune disorder) can be determined by standard clinical techniques. The dose, dose frequency, or both, will depend on the age of the patient, the patient's body weight, the patient's response, the seriousness of the patient's condition, and the past medical history of the patient as well as the route of administration, pharmacokinetic and pharmacodynamic effects of the prophylactic or therapeutic agent, and should be decided according to the judgment of the practitioner and each patient'scircumstances. Effective doses may be extrapolated from dose-response curves derived from *in vitro* or animal model test systems.

Exemplary doses of a small molecule include milligram or microgram amounts of the small molecule per kilogram of subject or sample weight (*e.g*., about 1 microgram per kilogram to about 500 milligrams per kilogram, about 100 micrograms per kilogram to about 5 milligrams per kilogram, or about 1 microgram per kilogram to about 50 micrograms per kilogram).

For antibodies, proteins, polypeptides, peptides and fusion proteins encompassed by the invention, the dosage administered to a patient is typically 0.0001 mg/kg to 100 mg/kg of the patient's body weight. Preferably, the dosage administered to a patient is between 0.0001 mg/kg and 20 mg/kg, 0.0001 mg/kg and 10 mg/kg, 0.0001 mg/kg and 5 mg/kg, 0.0001 and 2 mg/kg, 0.0001 and 1 mg/kg, 0.0001 mg/kg and 0.75 mg/kg, 0.0001 mg/kg and 0.5 mg/kg, 0.0001 mg/kg to 0.25 mg/kg, 0.0001 to 0.15 mg/kg, 0.0001 to 0.10 mg/kg, 0.001 to 0.5 mg/kg, 0.01 to 0.25 mg/kg or 0.01 to 0.10 mg/kg of the patient's body weight. Generally, human antibodies have a longer half-life within the human body than antibodies from other species due to the immune response to the foreign polypeptides. Thus, lower dosages of human antibodies and less frequent administration is often possible. Further, the dosage and frequency of administration of antibodies of the invention or fragments thereof may be reduced by enhancing uptake and tissue penetration of the antibodies by modifications such as, for example, lipidation.

Generally, a lymphoid tissue inducer is present in a composition in an amount that is sufficient or otherwise effective to induce lymphoid tissue, but insufficient to cause, bring about, or otherwise promote cell death by affecting the cell division process in cells that form the diseased tissue. For example, in compositions where the lymphoid tissue inducer is a microtubule-stabilizing agent, the microtubule stabilizing agent is present in an amount ineffective to stabilize the microtubules sufficiently to cause cell death by interfering with one or more stages of mitosis. Although some cell death can occur by interfering with one or more stages of mitosis, the amount of microtubule stabilizing agent is chosen to kill no more than about 50% (*i.e.,* amount of cells killed by microtubule stabilization leading to interference with mitosis divided by the total number of cells killed), preferably no more than about 45%, no more than about 40%, no more than about 35%, no more than about 30%, or no more than about 25%, no more than about 20%, no more than about 15%, no more than about 10%, or no more than about 5% of cells by stabilizing microtubules sufficiently to interfere with one or more stages of mitosis.

Generally, the prophylactically or therapeutically effective amount of a lymphoid tissue inducer is typically, on a weight/volume percentage basis (gram of lymphoid tissue inducer/100 ml composition), about 0.00001% to about 5%, preferably about 0.00001% to about 1%, and most preferably about 0.0001% to about 0.1%. Alternatively, the amount of a lymphoid tissue inducer in a composition is 0.0000001 g/m² to 10 g/m², preferably 0.0000001 g/m² to 5 g/m², and most preferably 0.0000001 g/m² to 1 g/m² of body surface. In a specific embodiment, the prophylactically or therapeutically effective amount of a lymphoid tissue inducer is 0.0000001 g/m², 0.000001 g/m², 0.00001 g/m², 0.0001 g/m², 0.001 g/m², 0.01 g/m², 0.1 g/m², 0.25 g/m², 0.5 g/m², 0.75 g/m², 1 g/m², 2 g/m² or 5 g/m² of body surface. In another embodiment, the prophylactically or therapeutically effective amount of taxol or a taxol analog is typically from about 1 mg/mm² per day to 1000 mg/mm² per day, preferably from about 10 mg/mm² per day to about 500 mg/mm² per day.

Generally, the prophylactically or therapeutically effective amount of an immunomodulatory agent is typically, on a weight/volume percentage basis (gram of immunomodulatory agent/100 ml composition), about 0.00001% to about 5%, preferably about 0.0000 1 % to about 1%, and most preferably about 0.0001% to about 0.1%. Alternatively, the prophylactically or therapeutically effective amount of an immunomodulatory agent is 0.0000001 g/m² to 10 g/m², preferably 0.0000001 g/m² to 5 g/m², and most preferably 0.0000001 g/m² to 1 g/m² of body surface. In a specific embodiment, the amount of an immunomodulatory agent in a composition is 0.0000001 g/m², 0.000001 g/m², 0.00001 g/m², 0.0001 g/m², 0.001 g/m², 0.01 g/m², 0.1 g/m², 0.25 g/m², 0.5 g/m², 0.75 g/m², 1 g/m², 2 g/m² or 5 g/m² of body surface.

The ratio of the lymphoid tissue inducer and immunomodulatory agent in the composition will also depend on the circumstances as delineated above. However, generally, it is preferred that the weight ratio between the lymphoid tissue inducer and the immunomodulatory agent is about 1:1 to about 1:100, more preferably about 1:1 to about 1:10. When more than one immunomodulatory agent is utilized, it is preferred that the weight ratio between the lymphoid tissue inducer and the immunomodulatory agent be about 1:0.1 to about 1:1, more preferably about 1:0.3 to about 1:0.5.

Generally, for various forms of neoplastic diseases, the amount of lymphoid tissue inducer in the composition administered can range, on a weight/kg body weight percentage basis (*i.e.,* g inducer/kg body weight) from about 0.00001% to about 1%, and further preferably about 0.0001 % to about 0.1 %. The amount of immunomodulatory agent, such as an immune system enhancer, in the composition administered can range, on a weight/kg body weight percentage basis (*i.e.,* g modulator/kg body weight) from about 0.00001% to about 1%, and further preferably about 0.0001% to about 0.1 %. The amount of lymphoid tissue utilized is preferably effective to induce formation of lymphoid tissue, but ineffective to cause cellular death by affecting cell division of cells in, or otherwise forming, the diseased tissue. Such formulations are preferably administered over a period of about 0.1 hours to about 48 hours per treatment. The number of treatments, and the intervals between treatments can vary, depending on the case and can further be determined by the skilled artisan. Alternatively, the lymphoid tissue inducer can be administered in an amount of about 0.00001 g/m² to about 0.1 g/m² of body surface, and preferably about 0.001 g/m² to about 0.01 g/m² of body surface. Additionally, the immunomodulatory agent can be administered in an amount of about 0.00001 g/m² to about 0.1 g/m² of body surface, and further preferably about 0.001 g/m² to about 0.01 g/m² of body surface.

For various diseases of bacterial origin, the amount of lymphoid tissue inducer administered can range, on a weight/kg body weight percentage basis (*i.e.*, g inducer per kg body weight), from about 0.00001 % to about 1%, and further preferably about 0.001 % to about 0.1%. The amount of immunomodulatory agent in the composition administered can range, on a weight/kg body weight percentage basis *(i.e.,* g modulator/kg body weight) from about 0.00001 % to about 1%, and further preferably about 0.001 % to about 0.1 %. Such a formulation is preferably administered over a period of about 0.1 hours to about 48 hours per treatment. Alternatively, lymphoid tissue inducer can be administered in an amount of about 0.0001 g/m² to about 0.1 g/m² of body surface, and preferably about 0.001 g/m² to about 0.01 g/m² of body surface. Further alternatively, the immunomodulatory agent can be administered in an amount of about 0.0001 g/ m² to about 0.1 g/m² of body surface, and further preferably about 0.001 g/m² to about 0.1 g/m² of body surface.

For various diseases of viral origin, the amount of lymphoid tissue inducer administered can range, on a weight/kg body weight percentage basis (*i.e.,* g inducer per kg body weight), from about 0.00001 % to about 1 %, and further preferably about 0.000 1 % to about 0.1%. The amount of immunomodulatory agent in the composition administered can range, on a weight/kg body weight percentage basis (*i.e.,* g modulator/kg body weight) from about 0.0001 % to about 1%, and further preferably about 0.001 % to about 0.1 %. Such a formulation is preferably administered over a period of about 0.1 hours to about 48 hours per treatment. Alternatively, the lymphoid tissue inducer can be administered in an amount of about 0.00001 g/ m² to about 0.1 g/m² of body surface, and preferably about 0.001 g/ m² to about 0.01 g/m² of body surface. Further alternatively, the immunomodulatory agent can be administered in an amount of about 0.00001 g/m² to about 0.1 g/m² of body surface, and further preferably about 0.001 g/m² to about 0.1 g/m² of body surface.

For various diseases of fungal origin, the amount of lymphoid tissue inducer administered can range, on a weight/kg body weight percentage basis (i.e., g inducer/kg body weight), from about 0.00001 % to about 1%, and further preferably about 0.001% to about 0.1%. The amount of immunomodulatory agent in the composition administered can range, on a weight/kg body weight percentage basis (i.e., g modulator/kg body weight) from about 0.00001% to about 1%, and further preferably about 0.001% to about 0.1%. Such a formulation is preferably administered over a period of about 0.1 hours to about 48 hours per treatment. Alternatively, the lymphoid tissue inducer can be administered in an amount of about 0.00001 g/ m² to about 0.1 g/m² of body surface, and preferably about 0.001 g/ m² to about 0.01 g/m² of body surface. Further alternatively, the immunomodulatory agent can be administered in an amount ranging from about 0.00001 g/m² to about 0.1 g/m² of body surface, and preferably from about 0.001 g/m² to about 0.1 g/m² of body surface.

In another embodiment, a subject is administered one or more doses of a prophylactically or therapeutically effective amount of a lymphoid tissue inducer, wherein the prophylactically or therapeutically effective amount is not the same for each dose. In another embodiment, a subject is administered one or more doses of a prophylactically or therapeutically effective amount of a lymphoid tissue inducer, wherein the dose of a prophylactically or therapeutically effective amount of the lymphoid tissue inducer administered to said subject is increased by, *e.g.,* 0.01 µg/kg, 0.02 µg/kg, 0.04 µg/kg, 0.05 µg/kg, 0.06 µg/kg, 0.08 µg/kg, 0.1 µg/kg, 0.2 µg/kg, 0.25 µg/kg, 0.5 µg/kg, 0.75 µg/kg, 1 µg/kg, 1.5 µg/kg, 2 µg/kg, 4 µg/kg, 5 µg/kg, 10 µg/kg, 15 µg/kg, 20 µg/kg, 25 µg/kg, 30 µg/kg, 35 µg/kg, 40 µg/kg, 45 µg/kg, or 50 µg/kg, as treatment progresses. In another embodiment, a subject is administered one or more doses of a prophylactically or therapeutically effective amount of a lymphoid tissue inducer, wherein the dose of a prophylactically or therapeutically effective amount of the lymphoid tissue inducer administered to said subject is decreased by, *e.g.,* 0.01 µg/kg, 0.02 µg/kg, 0.04 µg/kg, 0.05 µg/kg, 0.06 µg/kg, 0.08 µg/kg, 0.1 µg/kg, 0.2 µg/kg, 0.25 µg/kg, 0.5 µg/kg, 0.75 µg/kg, 1 µg/kg, 1.5 µg/kg, 2 µg/kg, 4 µg/kg, 5 µg/kg, 10 µg/kg, 15 µg/kg, 20 µg/kg, 25 µg/kg, 30 µg/kg, 35 µg/kg, 40 µg/kg, 45 µg/kg, or 50 µg/kg, as treatment progresses.

In another embodiment, a subject is administered one or more doses of a prophylactically or therapeutically effective amount of an immunomodulatory agent, wherein the prophylactically or therapeutically effective amount is not the same for each dose. In another embodiment, a subject is administered one or more doses of a prophylactically or therapeutically effective amount of an immunomodulatory agent, wherein the dose of a prophylactically or therapeutically effective amount of the immunomodulatory agent administered to said subject is increased by, *e.g.*, 0.01 µg/kg, 0.02 µg/kg, 0.04 µg/kg, 0.05 µg/kg, 0.06 µg/kg, 0.08 µg/kg, 0.1 µg/kg, 0.2 µg/kg, 0.25 µg/kg, 0.5 µg/kg, 0.75 µg/kg, 1 µg/kg, 1.5 µg/kg, 2 µg/kg, 4 µg/kg, 5 µg/kg, 10 µg/kg, 15 µg/kg, 20 µg/kg, 25 µg/kg, 30 µg/kg, 35 µg/kg, 40 µg/kg, 45 µg/kg, or 50 µg/kg, as treatment progresses. In another embodiment, a subject is administered one or more doses of a prophylactically or therapeutically effective amount of an immunomodulatory agent, wherein the dose of a prophylactically or therapeutically effective amount of the immunomodulatory agent administered to said subject is decreased by, *e.g.,* 0.01 µg/kg, 0.02 µg/kg, 0.04 µg/kg, 0.05 µg/kg, 0.06 µg/kg, 0.08 µg/kg, 0.1 µg/kg, 0.2 µg/kg, 0.25 µg/kg, 0.5 µg/kg, 0.75 µg/kg, 1 µg/kg, 1.5 µg/kg, 2 µg/kg, 4 µg/kg, 5 µg/kg, 10 µg/kg, 15 µg/kg, 20 µg/kg, 25 µg/kg, 30 µg/kg, 35 µg/kg, 40 µg/kg, 45 µg/kg, or 50 µg/kg, as treatment progresses.

The following is exemplary only and merely serves to illustrate possible administration regimens designed by a person of ordinary skill in the art. In one example, a lymphoid tissue inducer and/or an immunomodulatory agent are administered every two or three days for the first two weeks of every month for six months. After a six month period of rest, the lymphoid tissue inducer and/or the immunomodulatory agent can be administered under the same or different schedule. In another example, a lymphoid tissue inducer and/or an immunomodulatory agent is administered once a week for three months. After a three month period of rest, the lymphoid tissue inducer and/or the immunomodulatory agent can be administered under the same or different schedule. In another example, the lymphoid tissue inducer and/or the immunomodulatory agent is administered every three weeks for a year. After a two month period of rest, the lymphoid tissue inducer and the immunomodulatory agent can be administered under the same or different schedule. In a preferred example, the lymphoid tissue inducer and/or the immunomodulatory agent is administered once a week for 3 weeks out of each 4 week cycle. After a one week period of rest, the lymphoid tissue inducer and/or the immunomodulatory agent can be administered under the same or different schedule.

In a preferred embodiment, the lymphocyte count, preferably the T lymphocyte count, in a subject is monitored before, during and/or after administration of a certain number of doses of a lymphoid tissue inducer and/or an immunomodulatory agent. In accordance with these embodiments, the dosage of the lymphoid tissue inducer and/or immunomodulatory agent administered to said subject may be adjusted based up the results obtained while monitoring the subject. Further, in accordance with this embodiment, a certain number of doses is 0, 1, 2, 3, 4, 5, 6, 7, 8, 9, 10, 11, 12 or more doses. Techniques for assessing lymphocyte counts are well-known in the art and described herein in Section 5.7.

The amount of composition administered can also be sufficient for positively affecting the prognostic outcome of a particular disease. Endpoints that can be observed to verify a positive outcome will vary depending on the disease. For example, in the case of abnormal cellular proliferation, or cancer, tumor, or other neoplasm, a positive outcome includes a decrease in the amount of the diseased tissue in or otherwise on the patient, including a decrease in the size of the tumor, a decrease in metastasis, a decrease in the symptoms of the disease experienced by the patient and/or characteristic of the disease, or a combination thereof. In the case of viral, bacterial or fungal diseases, a positive outcome includes a decrease in the amount of microorganisms in or otherwise on the patient, a decrease in the symptoms of the disease, a decrease in the spreading of microorganisms to undiseased tissues, a decrease in the amount of diseased tissue or a combination thereof. In the case of an inflammatory disorder, a positive outcome includes a reduction in the swelling of a joint, tissue or organ.

### 5.7. CHARACTERIZATION & DEMONSTRATION OF PROPHYLACTIC OR THERAPEUTIC UTILITY OF THE COMBINATION THERAPY

Several aspects of the pharmaceutical compositions or prophylactic or therapeutic agents of the invention are preferably tested *in vitro,* in a cell culture system, and in an animal model organism, such as a rodent animal model system, for the desired therapeutic activity prior to use in humans. For example, assays which can be used to determine whether administration of a specific pharmaceutical composition or a specific combination of lymphoid tissue inducers and immunomodulatory agents is indicated, include cell culture assays in which a patient tissue sample is grown in culture, and exposed to or otherwise contacted with a pharmaceutical composition, and the effect of such composition upon the tissue sample is observed. The tissue sample can be obtained by biopsy from the patient. This test allows the identification of the therapeutically most effective prophylactic or therapeutic molecule(s) for each individual patient. In various specific embodiments, *in vitro* assays can be carried out with representative cells of cell types involved in a disorder (*e.g.,* immune cells or cancer cells), to determine if a pharmaceutical composition of the invention has a desired effect upon such cell types.

The combination therapies of the invention can be assayed for their ability to modulate the activation of various types of immune cells (including T cells, B cells, NK cells, macrophages, and dendritic cells). Activation of immune cells can be determined by measuring, *e.g.,* changes in the level of expression of cytokines and/or cell surface markers. Techniques known to those of skill in the art, including, but not limited to, immunoprecipitation followed by Western blot analysis, ELISAs, flow cytometry, Northern blot analysis, and RT-PCR can be used to measure the expression of cytokines and cell surface markers indicative of activation of the immune cell.

The combination therapies of the invention can be assayed for their ability to induce signal transduction in immune cells. The induction of signal transduction pathways in immune cells can be assayed by techniques known to those of skill in the art including, *e.g.*, kinase assays and electrophoretic mobility shift assays. The combination therapies of the invention can also be assayed for their ability to modulate immune cell proliferation. Techniques known to those in art, including, but not limited to, ³H-thymidine incorporation, trypan blue cell counts, and fluorescence activated cell sorting ("FACS") analysis. The combination therapies of the invention can also be assayed for their ability to induce cytolysis. Cytolysis can be assessed by techniques known to those in art, including, but not limited to,⁵¹CR-release assays.

The combination therapies of the invention can be tested in suitable animal model systems prior to use in humans. Such animal model systems include, but are not limited to rats, mice, chicken, cows, monkeys, pigs, dogs, rabbits, etc. Any animal system well-known in the art may be used. In a specific embodiment of the invention, the combination therapies of the invention are tested in a mouse model system. Such model systems are widely used and well-known to the skilled artisan. Lymphoid tissue inducers and/or immunomodulatory agents can be administered repeatedly. Several aspects of the procedure may vary. Said aspects include the temporal regime of administering the lymphoid tissue inducers and/or immunomodulatory, and whether such agents are administered separately or as an admixture.

The anti-cancer activity of the combination therapies of the invention can be determined using any suitable animal model, including, but not limited to, SCID mice with a tumor or injected with malignant cells. The anti-inflammatory activity of the combination therapies of the invention can be determined by using various experimental animal models of inflammatory arthritis known in the art and described in Crofford L.J. and Wilder R.L., "Arthritis and Autoimmunity in Animals", in Arthritis and Allied Conditions: A Textbook of Rheumatology, McCarty et *al*.(eds.), Chapter 30 (Lee and Febiger, 1993). Experimental and spontaneous animal models of inflammatory arthritis and autoimmune rheumatic diseases can also be used to assess the anti-inflammatory activity of the combination therapies of invention. The following are some assays provided as examples and not by limitation.

The principle animal models for arthritis or inflammatory disease known in the art and widely used include: adjuvant-induced arthritis rat models, collagen-induced arthritis rat and mouse models and antigen-induced arthritis rat, rabbit and hamster models, all described in Crofford L.J. and Wilder R.L., "Arthritis and Autoimmunity in Animals", in Arthritis and Allied Conditions: A Textbook of Rheumatology, McCarty et al. (eds.), Chapter 30 (Lee and Febiger, 1993), incorporated herein by reference in its entirety.

The anti-inflammatory activity of the combination therapies of invention can be assessed using a carrageenan-induced arthritis rat model. Carrageenan-induced arthritis has also been used in rabbit, dog and pig in studies of chronic arthritis or inflammation. Quantitative histomorphometric assessment is used to determine therapeutic efficacy. The methods for using such a carrageenan-induced arthritis model is described in Hansra P. *et al.,* "Carrageenan-Induced.Arthritis in the Rat," Inflammation, 24(2): 141-155, (2000). Also commonly used are zymosan-induced inflammation animal models as known and described in the art.

The anti-inflammatory activity of the combination therapies of invention can also be assessed by measuring the inhibition of carrageenan-induced paw edema in the rat, using a modification of the method described in Winter C. A. *et al.,* "Carrageenan-Induced Edema in Hind Paw of the Rat as an Assay for Anti-inflammatory Drugs" Proc. Soc. Exp. Biol Med. 111, 544-547, (1962). This assay has been used as a primary *in vivo* screen for the anti-inflammatory activity of most NSAIDs, and is considered predictive of human efficacy. The anti-inflammatory activity of the test prophylactic or therapeutic agents is expressed as the percent inhibition of the increase in hind paw weight of the test group relative to the vehicle dosed control group.

In a specific embodiment of the invention where the experimental animal model used is adjuvant-induced arthritis rat model, body weight can be measured relative to a control group to determine the anti-inflammatory activity of the combination therapies of invention. Alternatively, the efficacy of the combination therapies of invention can be assessed using assays that determine bone loss. Animal models such as ovariectomy-induced bone resorption mice, rat and rabbit models are known in the art for obtaining dynamic parameters for bone formation. Using methods such as those described by *Yositake et al.* or Yamamoto *et al.,* bone volume is measured *in vivo* by microcomputed tomography analysis and bone histomorphometry analysis. Yoshitake *et al.,* "Osteopontin-Deficient Mice Are Resistant to Ovariectomy-Induced Bone Resorption," Proc. Natl. Acad. Sci. 96:8156-8160, (1999); Yamamoto *et al.,* "The Integrin Ligand Echistatin Prevents Bone Loss in Ovariectomized Mice and Rats," Endocrinology 139(3):1411-1419, (1998), both incorporated herein by reference in their entirety.

Additionally, animal models for inflammatory bowel disease can also be used to assess the efficacy of the combination therapies of invention (Kim et al., 1992, Scand. J. Gastroentrol. 27:529-537; Strober, 1985, Dig. Dis. Sci. 30(12 Suppl):3S-10S). Ulcerative cholitis and Crohn's disease are human inflammatory bowel diseases that can be induced in animals. Sulfated polysaccharides including, but not limited to amylopectin, carrageen, amylopectin sulfate, and dextran sulfate or chemical irritants including but not limited to trinitrobenzenesulphonic acid (TNBS) and acetic acid can be administered to animals orally to induce inflammatory bowel diseases.

Animal models for asthma can also be used to assess the efficacy of the combination therapies of invention. An example of one such model is the murine adoptive transfer model in which aeroallergen provocation of TH1 or TH2 recipient mice results in TH effector cell migration to the airways and is associated with an intense neutrophilic (TH1) and eosinophilic (TH2) lung mucosal inflammatory response (Cohn et al., 1997, J. Exp. Med. 1861737-1747).

Animal models for autoimmune disorders can also be used to assess the efficacy of the combination therapies of invention. Animal models for autoimmune disorders such as type 1 diabetes, thyroid autoimmunity, sytemic lupus eruthematosus, and glomerulonephritis have been developed (see, *e.g.*, Flanders et al., 1999, Autoimmunity 29:235-246; Krogh et al., 1999, Biochimie 81:511-515; Foster, 1999, Semin. Nephrol. 19:12-24).

Animal models for psoriasis can also be used to assess the efficacy of the combination therapies of invention. Animal models for psoriasis have been developed (see, *e.g.*, Schon, 1999, J. Invest. Dermatol. 112:405-410).

Further, any assays known to those skilled in the art can be used to evaluate the prophylactic and/or therapeutic utility of the combination therapies of invention for the disorders disclosed herein.

The effect of the combination therapies of the invention on peripheral blood lymphocyte counts can be monitored/assessed using standard techniques known to one of skill in the art. Peripheral blood lymphocytes counts in a subject can be determined by, *e.g.*, obtaining a sample of peripheral blood from said subject, separating the lymphocytes from other components of peripheral blood such as plasma using, *e.g.*, Ficoll-Hypaque (Pharmacia) gradient centrifugation, and counting the lymphocytes using trypan blue. Peripheral blood T-cell counts in subject can be determined by, *e.g.*, separating the lymphocytes from other components of peripheral blood such as plasma using, *e.g.*, a use of Ficoll-Hypaque (Pharmacia) gradient centrifugation, labeling the T-cells with an antibody directed to a T-cell antigen such as CD3, CD4, and CD8 which is conjugated to FITC or phycoerythrin, and measuring the number of T-cells by FACS.

The toxicity and/or efficacy of the prophylactic and/or therapeutic protocols of the instant invention can be determined by standard pharmaceutical procedures in cell cultures or experimental animals, *e.g.*, for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Lymphoid tissue inducers and immunomodulatory agents that exhibit large therapeutic indices are preferred. While lymphoid tissue inducers and immunomodulatory agents that exhibit toxic side effects may be used, care should be taken to design a delivery system that targets such agents to the site of affected tissue in order to minimize potential damage to uninfected cells and, thereby, reduce side effects.

The data obtained from the cell culture assays and animal studies can be used in formulating a range of dosage of the lymphoid tissue inducers and immunomodulatory agents for use in humans. The dosage of such agents lies preferably within a range of circulating concentrations that include the ED₅₀ with little or no toxicity. The dosage may vary within this range depending upon the dosage form employed and the route of administration utilized. For any agent used in the method of the invention, the therapeutically effective dose can be estimated initially from cell culture assays. A dose may be formulated in animal models to achieve a circulating plasma concentration range that includes the IC₅₀ (*i.e.,* the concentration of the test compound that achieves a half-maximal inhibition of symptoms) as determined in cell culture. Such information can be used to more accurately determine useful doses in humans. Levels in plasma may be measured, for example, by high performance liquid chromatography (HPLC) and radioimmunasssay (RIA). The pharmacokinetics of a prophylactic or therapeutic can be determined, *e.g.*, by measuring parameters such as peak plasma level (Cₘₐₓ), area under the curve (AUC, which is measured by plotting plasma concentration of the agent versus time, and reflects bioavailability), half-life of the compound (t_{1/2}), and time at maximum concentration.

Efficacy in preventing or treating a proliferative disorder such as cancer may be demonstrated, *e.g.,* by detecting the ability of the combination therapies of the invention to reduce one or more symptoms of the proliferative disorder, to reduce the proliferation of cancerous cells, to reduce the spread of cancerous cells, or to reduce the size of a tumor. Efficacy in preventing or treating an infectious disease may be demonstrated, *e.g.*, by detecting the ability of the combination therapies of the invention to reduce one or more symptoms of the infectious disease, a reduction in the replication of the infectious agent, or a reduction in the spread of the infectious agent. Efficacy in preventing or treating a cardiovascular disease may be demonstrated, *e.g.*, by detecting the ability of the combination therapies of the invention to reduce one or more symptoms of the cardiovascular disease, a reduction in the blockage of blood vessels, or improved breathing.

Efficacy in preventing or treating an autoimmune disorder may be demonstrated, *e.g.*, by detecting the ability of the combination therapies of the invention to reduce one or more symptoms of the autoimmune disorder, to alter mean absolute lymphocyte counts, to decrease T cell proliferation, to decrease autoantibodies, or to modulate one or more particular cytokine profiles. Efficacy in preventing or treating an inflammatory disorder may be demonstrated, *e.g.*, by detecting the ability of the combination therapies of the invention to reduce one or more symptoms of the inflammatory disorder, to decrease T cell activation, to decrease T cell proliferation, to modulate one or more cytokine profiles, to reduce cytokine production, to reduce inflammation of a joint, organ or tissue or to improve quality of life. Changes in inflammatory disease activity may be assessed through tender and swollen joint counts, patient and physician global scores for pain and disease activity, and the ESR/CRP. Progression of structural joint damage may be assessed by quantitative scoring of X-rays of hands, wrists, and feet (Sharp method). Changes in functional status in humans with inflammatory disorders may be evaluated using the Health Assessment Questionnaire (HAQ), and quality of life changes are assessed with the SF-36.

### 5.8. METHODS OF PRODUCING ANTIBODIES

Antibodies that immunospecifically bind to an antigen can be produced by any method known in the art for the synthesis of antibodies, in particular, by chemical synthesis or preferably, by recombinant expression techniques.

Polyclonal antibodies immunospecific for an antigen can be produced by various procedures well-known in the art. For example, a human antigen can be administered to various host animals including, but not limited to, rabbits, mice, rats, etc. to induce the production of sera containing polyclonal antibodies specific for the human antigen. Various adjuvants may be used to increase the immunological response, depending on the host species, and include but are not limited to, Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (bacille Calmette-Guerin) and corynebacterium parvum. Such adjuvants are also well known in the art.

Monoclonal antibodies can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow *et al., Antibodies: A Laboratory Manual,* (Cold Spring Harbor Laboratory Press, 2nd ed. 1988); Hammerling, *et al.,* in: *Monoclonal Antibodies and T-Cell Hybridomas* 563-681 (Elsevier, N.Y., 1981) (said references incorporated by reference in their entireties). The term "monoclonal antibody'' as used herein is not limited to antibodies produced through hybridoma technology. The term "monoclonal antibody" refers to an antibody that is derived from a single clone, including any eukaryotic, prokaryotic, or phage clone, and not the method by which it is produced.

Methods for producing and screening for specific antibodies using hybridoma technology are routine and well known in the art. Briefly, mice can be immunized with a non-murine antigen and once an immune response is detected, *e.g.*, antibodies specific for the antigen are detected in the mouse serum, the mouse spleen is harvested and splenocytes isolated. The splenocytes are then fused by well known techniques to any suitable myeloma cells, for example cells from cell line SP20 available from the ATCC. Hybridomas are selected and cloned by limited dilution. The hybridoma clones are then assayed by methods known in the art for cells that secrete antibodies capable of binding a polypeptide of the invention. Ascites fluid, which generally contains high levels of antibodies, can be generated by immunizing mice with positive hybridoma clones.

Accordingly, the present invention provides methods of generating monoclonal antibodies as well as antibodies produced by the method comprising culturing a hybridoma cell secreting an antibody disclosed herein wherein, preferably, the hybridoma is generated by fusing splenocytes isolated from a mouse immunized with a non-murine antigen with myeloma cells and then screening the hybridomas resulting from the fusion for hybridoma clones that secrete an antibody able to bind to the antigen.

Antibody fragments which recognize specific particular epitopes may be generated by any technique known to those of skill in the art. For example, Fab and F(ab')2 fragments of the invention may be produced by proteolytic cleavage of immunoglobulin molecules, using enzymes such as papain (to produce Fab fragments) or pepsin (to produce F(ab')2 fragments). F(ab')2 fragments contain the variable region, the light chain constant region and the CH1 domain of the heavy chain. Further, antibodies can also be generated using various phage display methods known in the art.

In phage display methods, functional antibody domains are displayed on the surface of phage particles which carry the polynucleotide sequences encoding them. In particular, DNA sequences encoding VH and VL domains are amplified from animal cDNA libraries (*e.g.*, human or murine cDNA libraries of affected tissues). The DNA encoding the VH and VL domains are recombined together with an scFv linker by PCR and cloned into a phagemid vector. The vector is electroporated in *E. coli* and the *E. coli* is infected with helper phage. Phage used in these methods are typically filamentous phage including fd and M13 and the VH and VL domains are usually recombinantly fused to either the phage gene III or gene VIII. Phage expressing an antigen binding domain that binds to a particular antigen can be selected or identified with antigen, *e.g.,* using labeled antigen or antigen bound or captured to a solid surface or bead. Examples of phage display methods that can be used to make the antibodies of the present invention include those disclosed in Brinkman et al., 1995, J. Immunol. Methods 182:41-50; Ames et al., 1995, J. Immunol. Methods 184:177-186; Kettleborough et al., 1994, Eur. J. Immunol. 24:952-958; Persic et al., 1997, Gene 187:9-18; Burton et al., 1994, Advances in Immunology 57:191-280; PCT application No. PCT/GB91/O1 134; PCT publication Nos. WO 90/02809, WO 91/10737, WO 92/01047, WO 92/18619, WO 93/1 1236, WO 95/15982, WO 95/20401, and WO97/13844; and U.S. Patent Nos. 5,698,426, 5,223,409, 5,403,484, 5,580,717, 5,427,908, 5,750,753, 5,821,047, 5,571,698, 5,427,908, 5,516,637, 5,780,225, 5,658,727, 5,733,743 and 5,969,108; each of which is incorporated herein by reference in its entirety.

As described in the above references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria, *e.g.,* as described below. Techniques to recombinantly produce Fab, Fab's and F(ab')2 fragments can also be employed using methods known in the art such as those disclosed in PCT publication No. WO 92/22324; Mullinax et al., 1992, BioTechniques 12(6):864-869; Sawai et al., 1995, AJRI 34:26-34; and Better et al., 1988, Science 240:1041-1043 (said references incorporated by reference in their entireties).

To generate whole antibodies, PCR primers including VH or VL nucleotide sequences, a restriction site, and a flanking sequence to protect the restriction site can be used to amplify the VH or VL sequences in scFv clones. Utilizing cloning techniques known to those of skill in the art, the PCR amplified VH domains can be cloned into vectors expressing a VH constant region, *e.g.*, the human gamma 4 constant region, and the PCR amplified VL domains can be cloned into vectors expressing a VL constant region, *e.g.*, human kappa or lamba constant regions. Preferably, the vectors for expressing the VH or VL domains comprise an EF-1α promoter, a secretion signal, a cloning site for the variable domain, constant domains, and a selection marker such as neomycin. The VH and VL domains may also cloned into one vector expressing the necessary constant regions. The heavy chain conversion vectors and light chain conversion vectors are then co-transfected into cell lines to generate stable or transient cell lines that express full-length antibodies, *e.g.*, IgG, using techniques known to those of skill in the art.

For some uses, including *in vivo* use of antibodies in humans and *in vitro* detection assays, it may be preferable to use human or chimeric antibodies. Completely human antibodies are particularly desirable for therapeutic treatment of human subjects. Human antibodies can be made by a variety of methods known in the art including phage display methods described above using antibody libraries derived from human immunoglobulin sequences. See also U.S. Patent Nos. 4,444,887 and 4,716,111; and PCT publication Nos. WO 98/46645, WO 98/50433, WO 98/24893, WO98/16654, WO 96/34096, WO 96/33735, and WO 91/10741; each of which is incorporated herein by reference in its entirety.

Human antibodies can also be produced using transgenic mice which are incapable of expressing functional endogenous immunoglobulins, but which can express human immunoglobulin genes. For example, the human heavy and light chain immunoglobulin gene complexes may be introduced randomly or by homologous recombination into mouse embryonic stem cells. Alternatively, the human variable region, constant region, and diversity region may be introduced into mouse embryonic stem cells in addition to the human heavy and light chain genes. The mouse heavy and light chain immunoglobulin genes may be rendered non-functional separately or simultaneously with the introduction of human immunoglobulin loci by homologous recombination. In particular, homozygous deletion of the J_{H} region prevents endogenous antibody production. The modified embryonic stem cells are expanded and microinjected into blastocysts to produce chimeric mice. The chimeric mice are then be bred to produce homozygous offspring which express human antibodies. The transgenic mice are immunized in the normal fashion with a selected antigen, *e.g.*, all or a portion of a polypeptide of the invention. Monoclonal antibodies directed against the antigen can be obtained from the immunized, transgenic mice using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg and Huszar (1995, *Int. Rev. Immunol.* 13:65-93). For a detailed discussion of this technology for producing human antibodies and human monoclonal antibodies and protocols for producing such antibodies, *see, e.g.,* PCT publication Nos. WO 98/24893, WO 96/34096, and WO 96/33735; and U.S. Patent Nos. 5,413,923, 5,625,126, 5,633,425, 5,569,825, 5,661,016, 5,545,806, 5,814,318, and 5,939,598, which are incorporated by reference herein in their entirety. In addition, companies such as Abgenix, Inc. (Freemont, CA) and Genpharm (San Jose, CA) can be engaged to provide human antibodies directed against a selected antigen using technology similar to that described above.

A chimeric antibody is a molecule in which different portions of the antibody are derived from different immunoglobulin molecules such as antibodies having a variable region derived from a human antibody and a non-human immunoglobulin constant region. Methods for producing chimeric antibodies are known in the art. See *e.g.*, Morrison, 1985, Science 229:1202; Oi et al., 1986, BioTechniques 4:214; Gillies et al., 1989, J. Immunol. Methods 125:191-202; and U.S. Patent Nos. 5,807,715, 4,816,567, and 4,8 16397, which are incorporated herein by reference in their entirety. Chimeric antibodies comprising one or more CDRs from human species and framework regions from a non-human immunoglobulin molecule can be produced using a variety of techniques known in the art including, for example, CDR-grafting (EP 23.9,400; PCT publication No. WO 91/09967; and U.S. Patent Nos. 5,225,539, 5,530,101, and 5,585,089), veneering or resurfacing (EP 592,106; EP. 519,596; Padlan, 1991, Molecular Immunology 28(4/5):489-498; Studnicka et al., 1994, Protein Engineering 7(6):805-814; and Roguska et al., 1994, PNAS 91:969-973), and chain shuffling (U.S. Patent No. 5,565,332). Often, framework residues in the framework regions will be substituted with the corresponding residue from the CDR donor antibody to alter, preferably improve, antigen binding. These framework substitutions are identified by methods well known in the art, *e.g.,* by modeling of the interactions of the CDR and framework residues to identify framework residues important for antigen binding and sequence comparison to identify unusual framework residues at particular positions. (See, *e.g.,* Queen et al., U.S. Patent No. 5,585,089; and Riechmann et al., 1988, Nature 332:323, which are incorporated herein by reference in their entireties.)

Further, the antibodies that immunospecifically bind to an antigen can, in turn, be utilized to generate anti-idiotype antibodies that "mimic" an antigen using techniques well known to those skilled in the art. (See, *e.g.*, Greenspan & Bona, 1989, FASEB J. 7(5):437-444; and Nissinoff, 1991, J. Immunol. 147(8):2429-2438).

### 5.8.1. POLYNUCLEOTIDE SEQUENCES ENCODING ANTIBODIES

The invention provides polynucleotides comprising a nucleotide sequence encoding an antibody or fragment thereof that immunospecifically binds to an antigen. The invention also encompasses polynucleotides that hybridize under high stringency, intermediate or lower stringency hybridization conditions, *e.g.*, as defined *supra,* to polynucleotides that encode an antibody of the invention.

The polynucleotides may be obtained, and the nucleotide sequence of the polynucleotides determined, by any method known in the art. The nucleotide sequence of antibodies immunospecific for an antigen can be obtained, *e.g.*, from the literature or a database such as GenBank. Nucleotide codons known to encode particular amino acids may be assembled in such a way to generate a nucleic acid that encodes the antibody. Such a polynucleotide encoding the antibody may be assembled from chemically synthesized oligonucleotides (*e.g.*, as described in Kutmeier et al., 1994, BioTechniques 17:242), which, briefly, involves the synthesis of overlapping oligonucleotides containing portions of the sequence encoding the antibody, annealing and ligating of those oligonucleotides, and then amplification of the ligated oligonucleotides by PCR.

Alternatively, a polynucleotide encoding an antibody may be generated from a nucleic acid from a suitable source. If a clone containing a nucleic acid encoding a particular antibody is not available, but the sequence of the antibody molecule is known, a nucleic acid encoding the immunoglobulin may be chemically synthesized or obtained from a suitable source (*e.g.*, an antibody cDNA library, or a cDNA library generated from, or nucleic acid, preferably poly A+ RNA, isolated from, any tissue or cells expressing the antibody, such as hybridoma cells selected to express an antibody of the invention) by PCR amplification using synthetic primers hybridizable to the 3' and 5' ends of the sequence or by cloning using an oligonucleotide probe specific for the particular gene sequence to identify, *e.g.*, a cDNA clone from a cDNA library that encodes the antibody. Amplified nucleic acids generated by PCR may then be cloned into replicable cloning vectors using any method well known in the art.

Once the nucleotide sequence of the antibody is determined, the nucleotide sequence of the antibody may be manipulated using methods well known in the art for the manipulation of nucleotide sequences, *e.g.*, recombinant DNA techniques, site directed mutagenesis, PCR, etc. (see, for example, the techniques described in Sambrook et al., 1990, Molecular Cloning, A Laboratory Manual, 2d Ed., Cold Spring Harbor Laboratory, Cold Spring Harbor, NY and Ausubel *et al.,* eds., 1998, Current Protocols in Molecular Biology, John Wiley & Sons, NY, which are both incorporated by reference herein in their entireties), to generate antibodies having a different amino acid sequence, for example to create amino acid substitutions, deletions, and/or insertions.

In a specific embodiment, one or more of the CDRs is inserted within framework regions using routine recombinant DNA techniques. The framework regions may be naturally occurring or consensus framework regions, and preferably human framework regions (see, *e.g*., Chothia et al., 1998, J. Mol. Biol. 278: 457-479 for a listing of human framework regions). Preferably, the polynucleotide generated by the combination of the framework regions and CDRs encodes an antibody that specifically binds to a particular antigen (*e.g*., a cancer cell antigen). Preferably, as discussed *supra,* one or more amino acid substitutions may be made within the framework regions, and, preferably, the amino acid substitutions improve binding of the antibody to its antigen. Additionally, such methods may be used to make amino acid substitutions or deletions of one or more variable region cysteine residues participating in an intrachain disulfide bond to generate antibody molecules lacking one or more intrachain disulfide bonds. Other alterations to the polynucleotide are encompassed by the present invention and within the skill of the art.

### 5.8.2. RECOMBINANT EXPRESSION OF ANTIBODIES

Recombinant expression of an antibody that immunospecifically binds to an antigen requires construction of an expression vector containing a polynucleotide that encodes the antibody. Once a polynucleotide encoding an antibody molecule of the invention has been obtained, the vector for the production of the antibody molecule may be produced by recombinant DNA technology using techniques well-known in the art. See, *e.g.,* U.S. Patent No. 6,331,415, which is incorporated herein by reference in its entirety. Thus, methods for preparing a protein by expressing a polynucleotide containing an antibody encoding nucleotide sequence are described herein. Methods which are well known to those skilled in the art can be used to construct expression vectors containing antibody coding sequences and appropriate transcriptional and translational control signals. These methods include, for example, *in vitro* recombinant DNA techniques, synthetic techniques, and *in vivo* genetic recombination. The invention, thus, provides replicable vectors comprising a nucleotide sequence encoding an antibody molecule disclosed herein, a heavy or light chain of an antibody, a heavy or light chain variable domain of an antibody or a portion thereof, or a heavy or light chain CDR, operably linked to a promoter. Such vectors may include the nucleotide sequence encoding the constant region of the antibody molecule (see, e.g., PCT Publication No. WO 86/05807; PCT Publication No. WO 89/01036; and U.S. Patent No. 5,122,464) and the variable domain of the antibody may be cloned into such a vector for expression of the entire heavy, the entire light chain, or both the entire heavy and light chains.

The expression vector is transferred to a host cell by conventional techniques and the transfected cells are then cultured by conventional techniques to produce an antibody of the invention. Thus, the invention includes host cells containing a polynucleotide encoding an antibody or fragments thereof, or a heavy or light chain thereof, or portion thereof, or a single chain antibody, operably linked to a heterologous promoter. In preferred embodiments for the expression of double-chained antibodies, vectors encoding both the heavy and light chains may be co-expressed in the host cell for expression of the entire immunoglobulin molecule, as detailed below.

A variety of host-expression vector systems may be utilized to express the antibody molecules (see, *e.g.*, U.S. Patent No. 5,807,715). Such host-expression systems represent vehicles by which the coding sequences of interest may be produced and subsequently purified, but also represent cells which may, when transformed or transfected with the appropriate nucleotide coding sequences, express an antibody molecule of the invention in *situ.* These include but are not limited to microorganisms such as bacteria (*e.g*., *E. coli* and *B. subtilis*) transformed with recombinant bacteriophage DNA, plasmid DNA or cosmid DNA expression vectors containing antibody coding sequences; yeast (*e.g., Saccharomyces Pichia*) transformed with recombinant yeast expression vectors containing antibody coding sequences; insect cell systems infected with recombinant virus expression vectors (*e.g.*, baculovirus) containing antibody coding sequences; plant cell systems infected with recombinant virus expression vectors (*e.g.*, cauliflower mosaic virus, CaMV; tobacco mosaic virus, TMV) or transformed with recombinant plasmid expression vectors (*e.g.*, Ti plasmid) containing antibody coding sequences; or mammalian cell systems (*e.g.*, COS, CHO, BHK, 293, NS0, and 3T3 cells) harboring recombinant expression constructs containing promoters derived from the genome of mammalian cells (*e.g.*, metallothionein promoter) or from mammalian viruses (*e.g.*, the adenovirus late promoter; the vaccinia virus 7.5K promoter). Preferably, bacterial cells such as *Escherichia coli,* and more preferably, eukaryotic cells, especially for the expression of whole recombinant antibody molecule, are used for the expression of a recombinant antibody molecule. For example, mammalian cells such as Chinese hamster ovary cells (CHO), in conjunction with a vector such as the major intermediate early gene promoter element from human cytomegalovirus is an effective expression system for antibodies (Foecking et al., 1986, Gene 45:101; and Cockett et al., 1990, Bio/Technology 8:2). In a specific embodiment, the expression of nucleotide sequences encoding antibodies which immunospecifically bind to one or more antigens is regulated by a constitutive promoter, inducible promoter or tissue specific promoter.

In bacterial systems, a number of expression vectors may be advantageously selected depending upon the use intended for the antibody molecule being expressed. For example, when a large quantity of such a protein is to be produced, for the generation of pharmaceutical compositions of an antibody molecule, vectors which direct the expression of high levels of fusion protein products that are readily purified may be desirable. Such vectors include, but are not limited to, the *E. coli* expression vector pUR278 (Ruther et al., 1983, EMBO 12:1791), in which the antibody coding sequence may be ligated individually into the vector in frame with the lac Z coding region so that a fusion protein is produced; pIN vectors (Inouye & Inouye, 1985, Nucleic Acids Res. 13:3101-3109; Van Heeke & Schuster, 1989, J. Biol. Chem. 24:5503-5509); and the like. pGEX vectors may also be used to express foreign polypeptides as fusion proteins with glutathione 5-transferase (GST). In general, such fusion proteins are soluble and can easily be purified from lysed cells by adsorption and binding to matrix glutathione agarose beads followed by elution in the presence of free glutathione. The pGEX vectors are designed to include thrombin or factor Xa protease cleavage sites so that the cloned target gene product can be released from the GST moiety.

In an insect system, Autographa californica nuclear polyhedrosis virus (AcNPV) is used as a vector to express foreign genes. The virus grows in *Spodoptera frugiperda* cells. The antibody coding sequence may be cloned individually into non-essential regions (for example the polyhedrin gene) of the virus and placed under control of an AcNPV promoter (for example the polyhedrin promoter).

In mammalian host cells, a number of viral-based expression systems may be utilized. In cases where an adenovirus is used as an expression vector, the antibody coding sequence of interest may be ligated to an adenovirus transcription/translation control complex, *e.g.*, the late promoter and tripartite leader sequence. This chimeric gene may then be inserted in the adenovirus genome by *in vitro* or *in vivo* recombination. Insertion in a non-essential region of the viral genome (*e.g.*, region E1 or E3) will result in a recombinant virus that is viable and capable of expressing the antibody molecule in infected hosts (*e.g.*, see Logan & Shenk, 1984, Proc. Natl. Acad. Sci. USA 8 1:355-359). Specific initiation signals may also be required for efficient translation of inserted antibody coding sequences. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (see, *e.g.*, Bittner et al., 1987, Methods in Enzymol. 153:51-544).

In addition, a host cell strain may be chosen which modulates the expression of the inserted sequences, or modifies and processes the gene product in the specific fashion desired. Such modifications (*e.g.*, glycosylation) and processing (*e.g.*, cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include but are not limited to CHO, VERY, BHK, Hela, COS, MDCK, 293, 3T3, W138, BT483, Hs578T, HTB2, BT2O and T47D, NS0 (a murine myeloma cell line that does not endogenously produce any immunoglobulin chains), CRL7O3O and HsS78Bst cells.

For long-term, high-yield production of recombinant proteins, stable expression is preferred. For example, cell lines which stably express the antibody molecule may be engineered. Rather than using expression vectors which contain viral origins of replication, host cells can be transformed with DNA controlled by appropriate expression control elements (*e.g.*, promoter, enhancer, sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the antibody molecule. Such engineered cell lines may be particularly useful in screening and evaluation of compositions that interact directly or indirectly with the antibody molecule.

A number of selection systems may be used, including but not limited to, the herpes simplex virus thymidine kinase (Wigler et al., 1977, Cell **11**:223), hypoxanthineguanine phosphoribosyltransferase (Szybalska & Szybalski, 1992, Proc. Natl. Acad. Sci. USA 48:202), and adenine phosphoribosyltransferase (Lowy et al., 1980, Cell 22:8-17) genes can be employed in tk-, hgprt- or aprt- cells, respectively. Also, antimetabolite resistance can be used as the basis of selection for the following genes: *dhfr,* which confers resistance to methotrexate (Wigler et al., 1980, Natl. Acad. Sci. USA 77:357; O'Hare et al., 1981, Proc. Natl. Acad. Sci. USA 78:1527); *gpt,* which confers resistance to mycophenolic acid (Mulligan & Berg, 1981, Proc. Natl. Acad. Sci. USA 78:2072); neo, which confers resistance to the aminoglycoside G-418 (Wu and Wu, 1991, Biotherapy 3:87-95; Tolstoshev, 1993, Ann. Rev. Pharmacol. Toxicol. 32:573-596; Mulligan, 1993, Science 260:926-932; and Morgan and Anderson, 1993, Ann. Rev. Biochem. 62: 191-217; May, 1993, TIB TECH 11(5):155-2 15); and *hygro,* which confers resistance to hygromycin (Santerre et al., 1984, Gene 30:147). Methods commonly known in the art of recombinant DNA technology may be routinely applied to select the desired recombinant clone, and such methods are described, for example, in Ausubel *et al.* (eds.), Current Protocols in Molecular Biology, John Wiley & Sons, NY (1993); Kriegler, Gene Transfer and Expression, A Laboratory Manual, Stockton Press, NY (1990); and in Chapters 12 and 13, Dracopoli *et al.* (eds), Current Protocols in Human Genetics, John Wiley & Sons, NY (1994); Colberre-Garapin et al., 1981, J. Mol. Biol. 150:1, which are incorporated by reference herein in their entireties.

The expression levels of an antibody molecule can be increased by vector amplification (for a review, see Bebbington and Hentschel, The use of vectors based on gene amplification for the expression of cloned genes in mammalian cells in DNA cloning, Vol.3. (Academic Press, New York, 1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., 1983, Mol. Cell. Biol. 3:257).

The host cell may be co-transfected with two expression vectors of the invention, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes, and is capable of expressing, both heavy and light chain polypeptides. In such situations, the light chain should be placed before the heavy chain to avoid an excess of toxic free heavy chain (Proudfoot, 1986, Nature 322:52; and Kohler, 1980, Proc. Natl. Acad. Sci. USA 77:2 197). The coding sequences for the heavy and light chains may comprise cDNA or genomic DNA.

Once an antibody has been produced by recombinant expression, it may be purified by any method known in the art for purification of an immunoglobulin molecule, for example, by chromatography (*e.g.*, ion exchange, affinity, particularly by affinity for the specific antigen after Protein A, and sizing column chromatography), centrifugation, differential solubility, or by any other standard technique for the purification of proteins. Further, antibodies or fragments thereof may be fused to heterologous polypeptide sequences described herein or otherwise known in the art to facilitate purification.

### 5.8.3. ANTIBODIES WITH INCREASED HALF-LIVES

The present invention encompasses antibodies that immunospecifically bind to an antigen which have a extended half-life *in vivo.* In particular, the present invention encompasses antibodies that immunospecifically bind to an antigen which have a half-life in an animal, preferably a mammal and most preferably a human, of greater than 3 days, greater than 7 days, greater than 10 days, preferably greater than 15 days, greater than 25 days, greater than 30 days, greater than 35 days, greater than 40 days, greater than 45 days, greater than 2 months, greater than 3 months, greater than 4 months, or greater than 5 months.

To prolong the serum circulation of antibodies (*e.g.,* monoclonal antibodies, single chain antibodies and Fab fragments) *in vivo,* for example, inert polymer molecules such as high molecular weight polyethyleneglycol (PEG) can be attached to the antibodies with or without a multifunctional linker either through site-specific conjugation of the PEG to the Nor C-terminus of the antibodies or via epsilon-amino groups present on lysine residues. Linear or branched polymer derivatization that results in minimal loss of biological activity will be used. The degree of conjugation can be closely monitored by SDS-PAGE and mass spectrometry to ensure proper conjugation of PEG molecules to the antibodies. Unreacted PEG can be separated from antibody-PEG conjugates by size-exclusion or by ion-exchange chromatography. PEG-derivatized antibodies can be tested for binding activity as well as for *in vivo* efficacy using methods well-known to those of skill in the art, for example, by immunoassays described herein.

Antibodies having an increased half-life *in vivo* can also be generated introducing one or more amino acid modifications (*i.e.,* substitutions, insertions or deletions) into an IgG constant domain, or FcRn binding fragment thereof (preferably a Fc or hinge-Fc domain fragment). See, *e.g.*, International Publication No. WO 98/23289; International Publication No. WO 97/34631; and U.S. Patent No. 6,277,375, each of which is incorporated herein by reference in its entirety.

### 5.8.4. ANTIBODY CONJUGATES

The present invention encompasses antibodies or antigen-binding fragments thereof that immunospecifically bind to an antigen recombinantly fused or chemically conjugated (including both covalently and non-covalently conjugations) to a heterologous polypeptide (or a fragment thereof, preferably at least 5, at least 10, at least 20, at least 30, at least 40, at least 5 0, at least 60, at least 70, at least 80, at least 90 or at least 100 contiguous amino acids of the polypeptide) to generate fusion proteins. The fusion does not necessarily need to be direct, but may occur through linker sequences. For example, antibodies may be used to target heterologous polypeptides to particular cell types (*e.g.*, T-cells), either *in vitro* or *in vivo*, by fusing or conjugating the antibodies to antibodies specific for particular cell surface receptors such as, *e.g.*, CD4 and CD8.

The present invention also encompasses antibodies or antigen-binding fragments thereof that immunospecifically bind to an antigen fused to marker sequences, such as a peptide to facilitate purification. In preferred embodiments, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described in Gentz et al., 1989, Proc. Natl. Acad. Sci. USA 86:821-824, for instance, hexa-histidine provides for convenient purification of the fusion protein. Other peptide tags useful for purification include, but are not limited to, the hemagglutinin"HA" tag, which corresponds to an epitope derived from the influenza hemagglutinin protein (Wilson et al., 1984, Cell 37:767) and the "flag" tag.

The present invention further encompasses antibodies or antigen-binding fragments thereof that immunospecifically bind to an antigen conjugated to an agent which has a potential therapeutic benefit. An antibody or an antigen-binding fragment thereof that immunospecifically binds to an antigen may be conjugated to a therapeutic moiety such as a cytotoxin, *e.g.*, a cytostatic or cytocidal agent, an agent which has a potential therapeutic benefit, or a radioactive metal ion, *e.g.*, alpha-emitters. A cytotoxin or cytotoxic agent includes any agent that is detrimental to cells. Examples of a cytotoxin or cytotoxic agent include, but are not limited to, paclitaxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Agents which have a potential therapeutic benefit include, but are not limited to, antimetabolites (*e.g.*, methotrexate, 6-mercaptopurine, 6-thioguanine, cytarabine, 5-fluorouracil decarbazine), alkylating agents (*e.g.*, mechlorethamine, thioepa chlorambucil, melphalan, carmustine (BSNU) and lomustine (CCNU), cyclothosphamide, busulfan, dibromomannitol, streptozotocin, mitomycin C, and cisdichlorodiamine platinum (II) (DDP) cisplatin), anthracyclines (*e.g.,* daunorubicin (formerly daunomycin) and doxorubicin), antibiotics (*e.g.*, dactinomycin (formerly actinomycin), bleomycin, mithramycin, and anthramycin (AMC)), and anti-mitotic agents (*e.g.*, vincristine and vinblastine).

Further, an antibody or an antigen-binding fragment thereof that immunospecifically binds to an antigen may be conjugated to a therapeutic agent or drug moiety that modifies a given biological response. Agents which have a potential therapeutic benefit or drug moieties are not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumor necrosis factor, interferon-alpha, interferon-beta, interferon-gamma nerve growth factor (NGF), platelet derived growth factor (PDGF), tissue plasminogen activator (TPA), an apoptotic agent, *e.g.,* TNF-alpha, TNF-beta, AIM I (see, International Publication No. WO 97/33899), AIM II (see, International Publication No. WO 97/34911), Fas Ligand (Takahashi et al., 1994, J. Immunol., 6:1567-1574), and VEGF (see, International Publication No. WO 99/23105), a thrombotic agent or an anti-angiogenic agent, *e.g.*, angiostatin or endostatin; or, a biological response modifier such as, for example, a lymphokine (*e.g*., IL-1, IL-2, IL-6, IL-10, granulocyte macrophage colony stimulating factor (GM-CSF), and granulocyte colony stimulating factor (G-CSF), or a growth factor (*e.g.*, growth hormone (GH)).

Techniques for conjugating such therapeutic moieties to antibodies are well known, see, *e.g.,* Amon *et al.,* "Monoclonal Antibodies For Immunotargeting Of Drugs In Cancer Therapy", in Monoclonal Antibodies And Cancer Therapy, Reisfeld *et al.* (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985); and Thorpe et al., 1982, Immunol. Rev. 62:119-58.

An antibody or an antigen-binding fragment thereof that immunospecifically binds to an antigen can be conjugated to a second antibody to form an antibody heteroconjugate as described by Segal in U.S. Patent No. 4,676,980, which is incorporated herein by reference in its entirety.

Antibodies or antigen-binding fragments thereof that immunospecifically bind to an antigen may be attached to solid supports, which are particularly useful for the purification of immune cells such as T-cells. Such solid supports include, but are not limited to, glass, cellulose, polyacrylamide, nylon, polystyrene, polyvinyl chloride or polypropylene.

### 5.9. METHODS OF PRODUCING POLYPEPTIDES OR FUSION PROTEINS

Polypeptides and fusion proteins can be produced by standard recombinant DNA techniques or by protein synthetic techniques, *e.g.*, by use of a peptide synthesizer. For example, a nucleic acid molecule encoding a polypeptide or a fusion protein can be synthesized by conventional techniques including automated DNA synthesizers. Alternatively, PCR amplification of gene fragments can be carried out using anchor primers which give rise to complementary overhangs between two consecutive gene fragments which can subsequently be annealed and reamplified to generate a chimeric gene sequence (*see, e.g., Current Protocols in Molecular Biology,* Ausubel et al., eds., John Wiley & Sons, 1992). Moreover, a nucleic acid encoding a first polypeptide can be cloned into an expression vector containing a second polypeptide such that the first polypeptide is linked in-frame to the second polypeptide.

Methods for fusing or conjugating polypeptides to the constant regions of antibodies are known in the art. See, *e.g.*, U.S. Patent Nos. 5,336,603, 5,622,929, 5,359,046, 5,349,053, 5,447,851, 5,723,125, 5,783,181, 5,908,626, 5,844,095, and 5,112,946; EP 307,434; EP 367,166; EP 394,827; PCT publication Nos. WO 91/06570, WO 96/04388, WO 96/22024, WO 97/34631, and WO 99/04813; Ashkenazi et al., 1991, Proc. Natl. Acad. Sci. USA 88: 10535-10539; Traunecker et al., 1988, Nature, 331:84-86; Zheng et al., 1995, J. Immunol. 154:5590-5600; and Vil et al., 1992, Proc. Natl. Acad. Sci. USA 89:11337- 11341, which are incorporated herein by reference in their entireties.

The nucleotide sequences encoding a polypeptide may be obtained from any information available to those of skill in the art (*i.e.*, from Genbank, the literature, or by routine cloning). The nucleotide sequence coding for a polypeptide a fusion protein can be inserted into an appropriate expression vector, *i.e.*, a vector which contains the necessary elements for the transcription and translation of the inserted protein-coding sequence. A variety of host-vector systems may be utilized in the present invention to express the protein-coding sequence. These include but are not limited to mammalian cell systems infected with virus (*e.g*., vaccinia virus, adenovirus, etc.); insect cell systems infected with virus *(e.g.,* baculovirus); microorganisms such as yeast containing yeast vectors; or bacteria transformed with bacteriophage, DNA, plasmid DNA, or cosmid DNA. The expression elements of vectors vary in their strengths and specificities. Depending on the host-vector system utilized, any one of a number of suitable transcription and translation elements may be used.

The expression of a polypeptide or a fusion protein may be controlled by any promoter or enhancer element known in the art. Promoters which may be used to control the expression of the gene encoding fusion protein include, but are not limited to, the SV40 early promoter region (Bemoist and Chambon, 1981, Nature 290:304-310), the promoter contained in the 3' long terminal repeat of Rous sarcoma virus (Yamamoto, et al., 1980, Cell 22:787-797), the herpes thymidine kinase promoter (Wagner et al., 1981, Proc. Natl. Acad. Sci. U.S.A. 78:1441-1445), the regulatory sequences of the metallothionein gene (Brinster et al., 1982, Nature 296:39-42), the tetracycline (Tet) promoter (Gossen et al., 1995, Proc. Nat. Acad. Sci. USA 89:5547-5551); prokaryotic expression vectors such as the β-lactamase promoter (Villa-Kamaroff, et al., 1978, Proc. Natl. Acad. Sci. U.S.A. 75:3727-3731), or the *tac* promoter (DeBoer, et al., 1983, Proc. Natl. Acad. Sci. U.S.A. 80:21-25; see also "Useful proteins from recombinant bacteria" in Scientific American, 1980, 242:74-94); plant expression vectors comprising the nopaline synthetase promoter region (Herrera-Estrella et al., Nature 303:209-213) or the cauliflower mosaic virus 35S RNA promoter (Gardner, et al., 1981, Nucl. Acids Res. 9:2871), and the promoter of the photosynthetic enzyme ribulose biphosphate carboxylase (Herrera-Estrella et al., 1984, Nature 310:115-120); promoter elements from yeast or other fungi such as the Gal 4 promoter, the ADC (alcohol dehydrogenase) promoter, PGK (phosphoglycerol kinase) promoter, alkaline phosphatase promoter, and the following animal transcriptional control regions, which exhibit tissue specificity and have been utilized in transgenic animals: elastase I gene control region which is active in pancreatic acinar cells (Swift et al., 1984, Cell 38:639-646; Omitz et al., 1986, Cold Spring Harbor Symp. Quant. Biol. 50:399-409; MacDonald, 1987, Hepatology 7:425-515); insulin gene control region which is active in pancreatic beta cells (Hanahan, 1985, Nature 315:115-122), immunoglobulin gene control region which is active in lymphoid cells (Grosschedl et al., 1984, Cell 38:647-658; Adames et al., 1985, Nature 318:533-538; Alexander et al., 1987, Mol. Cell. Biol. 7:1436-1444), mouse mammary tumor virus control region which is active in testicular, breast, lymphoid and mast cells (Leder et al., 1986, Cell 45:485-495), albumin gene control region which is active in liver (Pinkert et al., 1987, Genes and Devel. 1:268-276), alpha-fetoprotein gene control region which is active in liver (Krumlauf et al., 1985, Mol. Cell. Biol. 5:1639-1648; Hammer et al., 1987, Science 235:53-58; alpha 1-antitrypsin gene control region which is active in the liver (Kelsey et al., 1987, Genes and Devel. 1:161-171), beta-globin gene control region which is active in myeloid cells (Mogram et al., 1985, Nature 315:338-340; Kollias et al., 1986, Cell 46:89-94; myelin basic protein gene control region which is active in oligodendrocyte cells in the brain (Readhead et al., 1987, Cell 48:703-712); myosin light chain-2 gene control region which is active in skeletal muscle (Sani, 1985, Nature 314:283-286); neuronal-specific enolase (NSE) which is active in neuronal cells (Morelli et al., 1999, Gen. Virol. 80:571-83); brain-derived neurotrophic factor (BDNF) gene control region which is active in neuronal cells (Tabuchi et al., 1998, Biochem. Biophysic. Res. Com. 253:818-823); glial fibrillary acidic protein (GFAP) promoter which is active in astrocytes (Gomes et al., 1999, Braz J Med Biol Res 32(5):619-631; Morelli et al., 1999, Gen. Virol. 80:571-83) and gonadotropic releasing hormone gene control region which is active in the hypothalamus (Mason et al., 1986, Science 234:1372-1378).

In a specific embodiment, the expression of a polypeptide or a fusion protein is regulated by a constitutive promoter. In another embodiment, the expression of a polypeptide or a fusion protein is regulated by an inducible promoter. In another embodiment, the expression of a polypeptide or a fusion protein is regulated by a tissue-specific promoter.

In a specific embodiment, a vector is used that comprises a promoter operably linked to a polypeptide- or a fusion protein-encoding nucleic acid, one or more origins of replication, and, optionally, one or more selectable markers (*e.g.*, an antibiotic resistance gene).

In mammalian host cells, a number of viral-based expression systems may be utilized. In cases where an adenovirus is used as an expression vector, the polypeptide or fusion protein coding sequence may be ligated to an adenovirus transcription/translation control complex, *e.g.,* the late promoter and tripartite leader sequence. This chimeric gene may then be inserted in the adenovirus genome by *in vitro* or *in vivo* recombination. Insertion in a non-essential region of the viral genome (*e.g.*, region E1 or E3) will result in a recombinant virus that is viable and capable of expressing the antibody molecule in infected hosts (*e.g.*, see Logan & Shenk, 1984, Proc. Natl. Acad. Sci. USA 81:355-359). Specific initiation signals may also be required for efficient translation of inserted fusion protein coding sequences. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (see Bittner et al., 1987, Methods in Enzymol. 153:51-544).

Expression vectors containing inserts of a gene encoding a polypeptide or a fusion protein can be identified by three general approaches: (a) nucleic acid hybridization, (b) presence or absence of "marker" gene functions, and (c) expression of inserted sequences. In the first approach, the presence of a gene encoding a polypeptide or a fusion protein in an expression vector can be detected by nucleic acid hybridization using probes comprising sequences that are homologous to an inserted gene encoding the polypeptide or the fusion protein, respectively. In the second approach, the recombinant vector/host system can be identified and selected based upon the presence or absence of certain "marker" gene functions (*e.g.*, thymidine kinase activity, resistance to antibiotics, transformation phenotype, occlusion body formation in baculovirus, etc.) caused by the insertion of a nucleotide sequence encoding a polypeptide or a fusion protein in the vector. For example, if the nucleotide sequence encoding the fusion protein is inserted within the marker gene sequence of the vector, recombinants containing the gene encoding the fusion protein insert can be identified by the absence of the marker gene function. In the third approach, recombinant expression vectors can be identified by assaying the gene product (*e.g.*, fusion protein) expressed by the recombinant. Such assays can be based, for example, on the physical or functional properties of the fusion protein in *in vitro* assay systems, *e.g.,* binding to an antibody.

In addition, a host cell strain may be chosen which modulates the expression of the inserted sequences, or modifies and processes the gene product in the specific fashion desired. Expression from certain promoters can be elevated in the presence of certain inducers; thus, expression of the genetically engineered fusion protein may be controlled. Furthermore, different host cells have characteristic and specific mechanisms for the translational and post-translational processing and modification (*e.g.*, glycosylation, phosphorylation of proteins). Appropriate cell lines or host systems can be chosen to ensure the desired modification and processing of the foreign protein expressed. For example, expression in a bacterial system will produce an unglycosylated product and expression in yeast will produce a glycosylated product. Eukaryotic host cells which possess the cellular machinery for proper processing of the primary transcript, glycosylation, and phosphorylation of the gene product may be used. Such mammalian host cells include, but are not limited to, CHO, VERY, BHK, Hela, COS, MDCK, 293, 3T3, WI38, NS0, and in particular, neuronal cell lines such as, for example, SK-N-AS, SK-N-FI, SK-N-DZ human neuroblastomas (Sugimoto et al., 1984, J. Natl. Cancer Inst. 73: 51-57), SK-N-SH human neuroblastoma (Biochim. Biophys. Acta, 1982, 704: 450-460), Daoy human cerebellar medulloblastoma (He et al., 1992, Cancer Res. 52: 1144-1148) DBTRG-05MG glioblastoma cells (Kruse et al., 1992, In Vitro Cell. Dev. Biol. 28A: 609-614), IMR-32 human neuroblastoma (Cancer Res., 1970, 30: 2110-2118), 1321N1 human astrocytoma (Proc. Natl Acad. Sci. USA ,1977, 74: 4816), MOG-G-CCM human astrocytoma (Br. J. Cancer, 1984, 49: 269), U87MG human glioblastoma-astrocytoma (Acta Pathol. Microbiol. Scand., 1968, 74: 465-486), A172 human glioblastoma (Olopade et al., 1992, Cancer Res. 52: 2523-2529), C6 rat glioma cells (Benda et al., 1968, Science 161: 370-371), Neuro-2a mouse neuroblastoma (Proc. Natl. Acad. Sci. USA, 1970, 65: 129-136), NB41A3 mouse neuroblastoma (Proc. Natl. Acad. Sci. USA, 1962, 48: 1184-1190), SCP sheep choroid plexus (Bolin et al., 1994, J. Virol. Methods 48: 211-221), G355-5, PG-4 Cat normal astrocyte (Haapala et al., 1985, J. Virol. 53: 827-833), Mpf ferret brain (Trowbridge et al., 1982, In Vitro 18: 952-960), and normal cell lines such as, for example, CTX TNA2 rat normal cortex brain (Radany et al., 1992, Proc. Natl. Acad. Sci. USA 89: 6467-6471) such as, for example, CRL7030 and Hs578Bst. Furthermore, different vector/host expression systems may effect processing reactions to different extents.

For long-term, high-yield production of recombinant proteins, stable expression is preferred. For example, cell lines which stably express a polypeptide or a fusion protein may be engineered. Rather than using expression vectors which contain viral origins of replication, host cells can be transformed with DNA controlled by appropriate expression control elements (*e.g.*, promoter, enhancer, sequences, transcription termina-tors, polyadenylation sites, etc.), and a selectable marker. Following the introduction of the foreign DNA, engineered cells may be allowed to grow for 1-2 days in an enriched medium, and then are switched to a selective medium. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express a polypeptide or a fusion protein. Such engineered cell lines may be particularly useful in screening and evaluation of compounds that affect the activity of a polypeptide or a fusion protein.

A number of selection systems may be used, including but not limited to the herpes simplex virus thymidine kinase (Wigler, et al., 1977, Cell 11:223), hypoxanthine-guanine phosphoribosyltransferase (Szybalska & Szybalski, 1962, Proc. Natl. Acad. Sci. USA 48:2026), and adenine phosphoribosyltransferase (Lowy, et al., 1980, Cell 22:817) genes can be employed in tk-, hgprt- or aprt- cells, respectively. Also, antimetabolite resistance can be used as the basis of selection for dhfr, which confers resistance to methotrexate (Wigler, et al., 1980, Natl. Acad. Sci. USA 77:3567; O'Hare, et al., 1981, Proc. Natl. Acad. Sci. USA 78:1527); gpt, which confers resistance to mycophenolic acid (Mulligan & Berg, 1981, Proc. Natl. Acad. Sci. USA 78:2072); neo, which confers resistance to the aminoglycoside G-418 (Colberre-Garapin, et al., 1981, J. Mol. Biol. 150:1); and hygro, which confers resistance to hygromycin (Santerre, et al., 1984, Gene 30:147) genes.

Once a polypeptide or a fusion protein has been produced by recombinant expression, it may be purified by any method known in the art for purification of a protein, for example, by chromatography (*e.g.*, ion exchange, affinity, particularly by affinity for the specific antigen after Protein A, and sizing column chromatography), centrifugation, differential solubility, or by any other standard technique for the purification of proteins.

### 5.10. ARTICLES OF MANUFACTURE

The present invention also encompasses a finished packaged and labeled pharmaceutical product. This article of manufacture includes the appropriate unit dosage form in an appropriate vessel or container such as a glass vial or other container that is hermetically sealed. In the case of dosage forms suitable for parenteral administration a prophylactic or therapeutic agent(s) (*e.g.*,lymphoid tissue inducer and/or immunomodulator (preferably, an immune system enhancer)), is sterile and suitable for administration as a particulate free solution. In other words, the invention encompasses both parenteral solutions and lyophilized powders, each being sterile, and the latter being suitable for reconstitution prior to injection. Alternatively, the unit dosage form may be a solid suitable for oral, transdermal, topical or mucosal delivery.

In a preferred embodiment, the unit dosage form is suitable for intravenous, intramuscular, oral or subcutaneous delivery. Thus, the invention encompasses solutions, preferably sterile, suitable for each delivery route.

As with any pharmaceutical product, the packaging material and container are designed to protect the stability of the product during storage and shipment. Further, the products of the invention include instructions for use or other informational material that advise the physician, technician or patient on how to appropriately prevent or treat the disease or disorder in question. In other words, the article of manufacture includes instruction means indicating or suggesting a dosing regimen including, but not limited to, actual doses, monitoring procedures, total lymphocyte and T-cell counts and other monitoring information.

Specifically, the invention provides an article of manufacture comprising packaging material, such as a box, bottle, tube, vial, container, sprayer, insufflator, intravenous (i.v.) bag, envelope and the like; and at least one unit dosage form of each pharmaceutical agent contained within said packaging material, wherein one pharmaceutical agent comprises a lymphoid tissue inducer and the other pharmaceutical agent comprises an immunomodulatory agent (preferably, an immune enhancer), and wherein said packaging material includes instruction means which indicate that said agents can be used to treat, prevent or ameliorate one or more symptoms of a disorder disclosed herein by administering specific doses and using specific dosing regimens as described herein.

The invention also provides an article of manufacture comprising packaging material, such as a box, bottle, tube, vial, container, sprayer, insufflator, i.v. bag, envelope and the like; and at least one unit dosage form of a pharmaceutical agent contained within said packaging material, wherein said pahrmaceutical agent comprises a lymphoid tissue inducer and an immunomodulatory agent (preferably, an immune system enhancer), and wherein said packaging material includes instruction means which indicate that such pharmaceutical agent can be used to treat, prevent or ameliorate one or more symptoms of a disorder disclosed herein by administering specific doses and using specific dosing regimens as described herein.

In specific embodiment, the instruction means enclosed in an article of manufacture indicates that lymphocyte or T-cell counts be monitored one or more times before and/or after a dose. For example, the instruction means enclosed in an article of manufacture can indicate that a lymphocyte count be taken before the first dose and after one or more subsequent doses. Suitable instruction means include printed labels, printed package inserts, tags, cassette tapes, and the like.

The present invention provides that the adverse effects that may be reduced or avoided by the methods of the invention are indicated in informational material enclosed in an article of manufacture for use in preventing, treating or ameliorating one or more symptoms associated with a disorder such as a proliferative disorder, an infectious disease, a cardiovascular disorder, an autoimmune disorder, or an inflammatory disorder. Adverse effects that may be reduced or avoided by the methods of the invention include but are not limited to vital sign abnormalities (*e.g*., fever, tachycardia, bardycardia, hypertension, and hypotension), hypercalcemia, hematological events (*e.g*., anemia, lymphopenia, leukopenia, and thrombocytopenia), headache, chills, dizziness, nausea, asthenia, back pain, chest pain (*e.g*., chest pressure), diarrhea, myalgia, pain, pruritus, psoriasis, rhinitis, sweating, injection site reaction, and vasodilatation.

Further, the information material enclosed in an article of manufacture for use in preventing, treating or ameliorating one or more symptoms associated with a disorder can indicate that foreign proteins may also result in allergic reactions, including anaphylaxis, or cytosine release syndrome. The information material should indicate that allergic reactions may exhibit only as mild pruritic rashes or they may be severe such as erythroderma, Stevens-Johnson syndrome, vasculitis, or anaphylaxis. The information material should also indicate that anaphylactic reactions (anaphylaxis) are serious and occasionally fatal hypersensitivity reactions. Allergic reactions including anaphylaxis may occur when any foreign protein is injected into the body. They may range from mild manifestations such as urticaria or rash to lethal systemic reactions. Anaphylactic reactions occur soon after exposure, usually within 10 minutes. Patients may experience paresthesia, hypotension, laryngeal edema, mental status changes, facial or pharyngeal angioedema, airway obstruction, bronchospasm, urticaria and pruritus, serum sickness, arthritis, allergic nephritis, glomerulonephritis, temporal arthritis, or eosinophilia.

The information material can also indicate that cytokine release syndrome is an acute clinical syndrome, temporally associated with the administration of certain activating anti-T cell antibodies. Cytokine release syndrome has been attributed to the release of cytokines by activated lymphocytes or monocytes. The clinical manifestations for cytokine release syndrome have ranged from a more frequently reported mild, self-limited, "flu-like" illness to a less frequently reported severe, life-threatening, shock-like reaction, which may include serious cardiovascular, pulmonary and central nervous system manifestations. The syndrome typically begins approximately 30 to 60 minutes after administration (but may occur later) and may persist for several hours. The frequency and severity of this symptom complex is usually greatest with the first dose. With each successive dose, both the incidence and severity of the syndrome tend to diminish. Increasing the amount of a dose or resuming treatment after a hiatus may result in a reappearance of the syndrome. As mentioned above, the invention encompasses methods of treatment and prevention that avoid or reduce one or more of the adverse effects discussed herein.

### 5.11. SPECIFIC EMBODIMENTS

In a specific embodiment, the therapeutic regimen for treating breast cancer comprises administering to a subject in need thereof taxol (80-160 mg/m², weekly, biweekly or monthly for 2-6 cycles); 4783 (50-500 mg/m², weekly, biweekly or monthly for 2-6 cycles); Herceptin antibody (2-100 mg/m², weekly, biweekly or monthly for 2-6 cycles); and OK-432 (0.5-5 mg/m², weekly, biweekly or monthly for 2-6 cycles).

In a specific embodiment, the therapeutic regimen for treating prostate cancer comprises administering to a subject in need thereof taxol (80-160 mg/m², weekly, biweekly or monthly for 2-6 cycles); 4783 (50-500 mg/m², weekly, biweekly or monthly for 2-6 cycles); PSMA antibody (2-100 mg/m², weekly, biweekly or monthly for 2-6 cycles); and CpG (0.5-5 mg/m², weekly, biweekly or monthly for 2-6 cycles).

In a specific embodiment, the therapeutic regimen for treating colorectal cancer comprises administering to a subject in need thereof taxotere (80-160 mg/m², weekly, biweekly or monthly for 2-6 cycles); prostaglandin J2 (5-50 mg/m², weekly, biweekly or monthly for 2-6 cycles); EpCAM antibody (2-100 mg/m², weekly, biweekly or monthly for 2-6 cycles); and BCG (0.5-5 mg/m², weekly, biweekly or monthly for 2-6 cycles).

In a specific embodiment, the therapeutic regimen for treating non-small lung cancer comprises administering to a subject in need thereof epothilone A, B,C, or D (80-160 mg/m², weekly, biweekly or monthly for 2-6 cycles); geranyl-geranyl-acetone (50-500 mg/m², weekly, biweekly or monthly for 2-6 cycles); EGF receptor antibody (2-100 mg/m², weekly, biweekly or monthly for 2-6 cycles); and lipoteichoic acid (0.5-5 mg/m², weekly, biweekly or monthly for 2-6 cycles).

In a specific embodiment, the therapeutic regimen for treating small lung cancer comprises administering to a subject in need thereof taxol (80-160 mg/m², weekly, biweekly or monthly for 2-6 cycles); 4783 (50-500 mg/m², weekly, biweekly or monthly for 2-6 cycles); Carboplatin (50-500 mg/m², weekly, biweekly or monthly for 2-6 cycles); and OK-432 (0.5-5 mg/m², weekly, biweekly or monthly for 2-6 cycles).

In a specific embodiment, the therapeutic regimen for treating genital warts comprises administering to a subject in need thereof taxol (8-16 mg/m², weekly, biweekly or monthly for 2-6 cycles); prostaglandin J2 (50-500 mg/m², weekly, biweekly or monthly for 2-6 cycles); and OK-432 (0.5-5 mg/m², weekly, biweekly or monthly for 2-6 cycles).

In a specific embodiment, the therapeutic regimen for treating shingles comprises administering to a subject in need thereof taxol (80-160 mg/m², weekly, biweekly or monthly for 2-6 cycles); geranyl-geranyl acetone (50-500 mg/m², weekly, biweekly or monthly for 2-6 cycles); and CpG (0.5-5 mg/m², weekly, biweekly or monthly for 2-6 cycles).

In a specific embodiment, the therapeutic regimen for treating uterine fibroids comprises administering to a subject in need thereof taxotere (80-160 mg/m², weekly, biweekly or monthly for 2-6 cycles); 4783 (50-500 mg/m², weekly, biweekly or monthly for 2-6 cycles); EpCAM antibody (2-100 mg/m², weekly, biweekly or monthly for 2-6 cycles); and OK-432 (0.5-5 mg/m², weekly, biweekly or monthly for 2-6 cycles).

In a specific embodiment, the therapeutic regimen for treating ovarian cysts comprises administering to a subject in need thereof taxol (80-160 mg/m², weekly, biweekly or monthly for 2-6 cycles); 4783 (50-500 mg/m², weekly, biweekly or monthly for 2-6 cycles); EGF receptor antibody (2-100 mg/m², weekly, biweekly or monthly for 2-6 cycles); and CpG (0.5-5 mg/m², weekly, biweekly or monthly for 2-6 cycles).

In a specific embodiment, the therapeutic regimen for treating endometrosis comprises administering to a subject in need thereof taxol (80-160 mg/m², weekly, biweekly or monthly for 2-6 cycles); prostaglandin J2 (50-500 mg/m², weekly, biweekly or monthly for 2-6 cycles); EpCAM antibody (2-100 mg/m², weekly, biweekly or monthly for 2-6 cycles); and lipoteichoic acid (0.5-5 mg/m², weekly, biweekly or monthly for 2-6 cycles).

In a specific embodiment, the therapeutic regimen for treating benign prostate hyperplasia comprises administering to a subject in need thereof taxol (60-100 mg/m², weekly, biweekly or monthly for 2-6 cycles); jeranyl-geranyl acetone (50-500 mg/m², weekly, biweekly or monthly for 2-6 cycles); PSMA antibody (2-100 mg/m², weekly, biweekly or monthly for 2-6 cycles); and CpG (0.5-5 mg/m², weekly, biweekly or monthly for 2-6 cycles).

In a specific embodiment, the therapeutic regimen for treating atherosclerosis comprises administering to a subject in need thereof taxotere (8-16 mg/m², weekly, biweekly or monthly for 2-6 cycles); 4783 (5-50 mg/m², weekly, biweekly or monthly for 2-6 cycles); foam cell antibody (2-50 mg/m², weekly, biweekly or monthly for 2-6 cycles); and OK-432 (0.5-2 mg/m², weekly, biweekly or monthly for 2-6 cycles).

In a specific embodiment, the therapeutic regimen for treating persistent bacterial infection comprises administering to a subject in need thereof taxol (40-100 mg/m², weekly, biweekly or monthly for 2-6 cycles); 4783 (50-500 mg/m², weekly, biweekly or monthly for 2-6 cycles); anti-bacterial antibody (2-100 mg/m², weekly, biweekly or monthly for 2-6 cycles); and CpG (0.5-5 mg/m², weekly, biweekly or monthly for 2-6 cycles).

In a specific embodiment, the therapeutic regimen for treating persistent viral infection comprises administering to a subject in need thereof taxol (40-100 mg/m², weekly, biweekly or monthly for 2-6 cycles); 4783 (50-500 mg/m², weekly, biweekly or monthly for 2-6 cycles); viral antigens antibody (2-100 mg/m², weekly, biweekly or monthly for 2-6 cycles); and OK-432 (0.5-5 mg/m², weekly, biweekly or monthly for 2-6 cycles).

### 6. EXAMPLES:

### EXAMPLE 1: METHODS FOR IDENTIFYING LYMPHOID TISSUE INDUCERS

The present example describes methods for identifying inducers of lymphoid tissue. The method can be applied to identify a wide variety of lymphoid tissue inducers, especially those that induce expression of lymphotoxin-α, B lymphocyte chemokine (BLC) and secondary lymphoid organ chemokine (SLC). As one example, it describes methods that can be used to identify small molecule inducers of lymphotoxin-α, BLC and SLC by *in vitro* screening. To date, the only two small molecules reported to induce lymphotoxin expression are pentoxifylline (LT-X, Clerici *et al., J. Infect. Dis.* 175(5):1210 - 1215 (1997)) and cimetidine (LT-β, Takahashi et al., Biochem. Biophys. Res. comm. 281(5):1113-1119 (2001)). LT-α expression has been shown to induce expression of BLC and SLC (Hjelmstrom *et al., Am. J. Pathol.,* 156(4):1133-1138 (2000)).

### Cells and Assay System

In one form of a method, chemical libraries can be screened utilizing the cancer cell lines described herein. Cells can be maintained as monolayers and exposed to compounds of interest. After 24 hours in culture, the supernatants can be collected and assayed by ELISA. ELISA plates will be prepared with capture antibodies for LT-α (human, BD Biosciences), BLC or SLC (human, R&D Systems). The culture supernatants can be added at 150 µL per well. Standard curves can be generated using recombinant LT-α, BLC and SLC such that protein values can be measured as pg/mL.

### Test for in vivo induction with small molecules

Once the small molecules have been identified, they can be screened to either verify they induce production of the molecules, such as proteins, that induce production of lymphoid tissue or verify that they induce production of lymphoid tissue. In this example, the screening is performed to verify production of the proteins encoding genes that the genes LT-α, BLC, and/or SLC. Therefore, tumor-bearing mice will be treated with small molecules that induce LT-α, BLC, and/or SLC in vitro. Tumor tissue, spleen, and lymph nodes will be analyzed for expression of these genes by quantitative *in situ* hybridization and immunohistochemistry.

### In vivo tumor formation

6-week-old C57BL/6J mice (Charles River Laboratories) can be housed under pathogen-free conditions with free access to food and water. Only normal, healthy mice should be used. All experiments with mice should be performed according to NIH/ICOC guidelines for animal care. Tumors can be induced by subcutaneous injection of 2.5 x 10⁶ B78-D14 melanoma cells, which results in 40 µL tumors within 14 days (Schrama, et al., Immunity 14(2):111-121 (2001)).

### Small molecule injection

Candidate small molecules (0.2 mL/20 g of mouse body weight, 45% encapsin HPB (hydroxypropyl β-cyclodextrin; American Maise-Products Co.) in water) or vehicle (45% HPB) can be administered intraperitoneally to tumor-bearing mice at three-day intervals at appropriately determined doses.

### In situ hybridization and immunohistochemistry

Analysis of gene expression levels can be performed following the death of the animal. B78-D14 melanoma-bearing mice have a median survival time of 47 days (Schrama, D. et al., Immunity 14(2):111-121, (2001)). Although this survival time can lengthen in treated mice, analysis can be performed the same day for treated and control mice. For *in situ* hybridization, replicate 10 µm frozen sections (stored at -80 C) of tumor tissue can be hybridized with digoxigenin-labelled anti-sense probes for LT-α, BLC and SLC, as described (Ansel, K.M., et al., Nature 406(6793):309-314 (2000)). Following incubation with alkaline-phosphatase conjugated anti-digoxigenin, the sections can be counter-stained with Nuclear Fast Red and mounted. The frequency of cells expressing mRNA for LT-α, BLC and SLC can be determined in a blinded manner using a grid counting system and a Zeiss microscope. Immunohistochemistry can also be performed using mouse monocolonal antibodies against LT-α₁β₂ (rat monoclonal anti-mouse, Pharmingen), and goat anti-mouse BLC and SLC (R&D Systems, Minneapolis, MN - used in Luther et al). The frequency of positive cells can be determined in a blinded manner.

### EXAMPLE 2: METHODS FOR EVALUATING TUMOR-ASSOCIATED LYMPHOID NEOGENESIS AND TUMOR REGRESSION

Once candidate small molecule or other inducers of lymphoid tissue have been identified, they can be administered to animals utilizing, for example, a subject model of tumorigenesis and tumor-associated lymphoid neogenesis and/or tumor regression can be observed as described in this example.

### Assaying tumor-associated lymphoid neogenesis

Tumor sections can be analyzed immunohistochemically for hallmark features of lymphoid tissue. Tumor infiltration by T cells can be assayed using CD4, CD8, and L-selectin (CD62L) antibodies (Pharmingen, San Diego, CA). The presence of B cells and their segregation can be assessed using the CD45R/B220 monoclonal antibody (Pharmingen). For those tumors displaying T and B cell infiltration, further analysis can be performed.

Infiltration by antigen-presenting cells can be analyzed using dendritic cell-specific, macrophage-specific or B cell-specific antibodies (ATCC, Rockville, MD). The formation of high endothelial venules can be checked using antibodies for peripheral lymph node addressin (PNAd) (Pharmingen) and, in PNAd⁺ tumors, sections can also be stained with SLC antibodies (R&D Systems).

### EXAMPLE 3: IN VIVO ANTI-TUMOR EFFICACY OF COMBINATION TREATMENT OF PACLITAXEL ANDARSENIC TRIOXIDE ON MDA-435 HUMAN BREAST CANCER NUDE MOUSE MODEL

This example demonstrates the *in vivo* anti-cancer efficacy of the combination of paclitaxel and arsenic trioxide in tumor bearing mice using a tumor growth inhibition assay.

### MATERIALS & METHODS

### Human Breast Cancer Nude Mouse Model

A supplemented media was prepared from 50% DMEM/Dulbecco Modified Eagle Medium (High Glucose), 50% RPMI 1640, 10% FBS/Fetal Bovine Serum (Hybridoma Tested; Sterile Filtered), 1% L-Glutamine, 1% Penicillin-Streptomycin, 1% MEM Sodium Pyruvate and 1% MEM Non-Essential Amino Acids. FBS was obtained from Sigma Chemical Co. and other ingredients were obtained from Invitrogen Life Technologies, USA). The supplemental media was warmed to 37 °C and 50 ml of media was added to a 175 cm² tissue culture flask.

The cells used in the assay were MDA-435 Human Breast Carcinoma from the American Type Culture Collection. 1 vial of MDA-435 cells from the liquid nitrogen frozen cell stock was removed. The frozen vial of cells was immediately placed into a 37°C water bath and gently swirled until thawed. The freeze-vial was wiped with 70% ethanol and cells were immediately pipetted into the 175 cm² tissue culture flask containing supplemented media. The cells were incubated overnight and the media was removed and replaced with fresh supplemented media the next day. The flask was incubated until flask became approximately 90% confluent. This took anywhere from 5-7 days.

The flask was washed with 10 ml of sterile room temperature phosphate buffered saline (PBS). The cells were trypsinized by adding 5 ml of warmed Trypsin-EDTA (Invitrogen) to the flask of cells. The cells were then incubated for 2-3 minutes at 37°C until cells begun to detach from the surface of the flask. An equal volume of supplemented media (5 ml) was added to the flask. All the cells were collected into 50 ml tube, and centrifuged at 1000 RPM for 5 minutes at 20° C. The supernatant was aspirated and the cell pellet was resuspended in 10 ml of supplemented media and the cells were counted. 1-3 million cells/flask were seeded into 5-7 tissue culture flasks (175 cm²). Each flask contained 50 ml of supplemented media. The flasks were incubated until about 90% confluent. The passaging of the cells was repeated until enough cells have been grown for tumor implantation.

The above procedure for trypsinizing and centrifuging the cells was followed. The supernatant was aspirated and the cell pellet was resuspended in 10 ml of sterile PBS and the cells were counted. The cells were centrifuged and then resuspended with appropriate volume of sterile PBS for injection of correct number of cells needed for tumor implantation. In the case of MDA-435, 100 million cells were suspended with 2.0 ml of sterile PBS to a final concentration of 50 million cells/ml in order to inject 5 million cells in 0.1 ml/mouse.

Mice (CD-1 nu/nu) were obtained from Charles River Laboratories: nomenclature Cr1:CD-1-nuBR, Age: 6-8 weeks. The mice were allowed to acclimate for 1 week prior to the being used in an experimental procedure.

Implantation of the MDA-435 tumor cell suspension took place into the corpus adiposum of the female CD-1 nu/nu mouse. This fat body is located in the ventral abdominal viscera of the mouse. Tumor cells were implanted subcutaneously into the fat body located in the right quadrant of the abdomen at the juncture of the os coxae (pelvic bone) and the os femoris (femur). 5 million MDA-435 cells in 0.1 ml of sterile PBS were injected using 27 G (1/2 inch) needle. MDA-43 tumors developed 2-3 weeks after implantation.

### Preparation of Paclitaxel & Arsenic Trioxide

Preparation of Dosing Solution for Paclitaxel Administration: The Paclitaxel DMSO stock solution was diluted 1:10 with 20% Cremophor RH40. The final formulation for the Paclitaxel dosing solution was 10% DMSO, 18% Cremophor RH40 and 72% water.

The Dosing Solution (Dosing Volume: 0.01 ml/gram = 10 ml/ kg) was injected intravenously into the mice bearing MDA-435 human breast tumor.

Arsenic Trioxide (As₂O₃) was prepared in 0.9% Sodium Chloride (Abbott Laboratories, USA). Mice received a dose of 5 mg/kg. This compound was given by the intraperitoneal (IP) route of administration. Intraperitoneal injections are made into the caudal left abdominal quadrant of the mouse. This is done in order to avoid the cecum in the caudal right abdominal quadrant. A dosing solution of 0.5 mg/ml of As₂O₃ prepared in 0.9% NaCL, and the dosing volume per injection was 10 mL/kg.

### Protocol

Formulation: Paclitaxell0%DMSO, 18%CrRH40, 72%H₂O for Paclitaxel
   0.9% Sodium Chloride for As₂O₃
Administration route: Intravenous Bolus Injection (Paclitaxel)
   Intraperitoneal Injection (As₂O₃)
Dosing schedule: 3x/week x 3 weeks

| **Group** | **Drug Treatment (Dose)** |
|---|---|
| 1 | Vehicle Only |
| 2 | Paclitaxel (5 mg/kg) |
| 3 | As₂O₃ (5 mg/kg) |
| 4 | As₂O₃ (5 mg/kg) + Paclitaxel (5 mg/kg) |

Treatment of the tumor with arsenic trioxide and Paclitaxel began after the tumor had been established (volume was about 200 mm³). Animals then underwent a multiple injection schedule whereby the compounds were given by the IV or IP route of administration. Tumors were measured two times a week. During the course of this assay, animals were monitored daily for signs of toxicity including body weight loss.

### RESULTS

Figure 1 shows the anti-tumor efficacy of treatment of combination of As₂O₃ and Paclitaxel as compared to As₂O₃ and Paclitaxel alone treatments. As can be seen in Figure 1, the combination treatment of As₂O₃ and Paclitaxel shows significantly higher anti-tumor efficacy than either As₂O₃ alone or Paclitaxel alone in this tumor model.

### Equivalents

Those skilled in the art will recognize, or be able to ascertain using no more than routine experimentation, many equivalents to the specific embodiments of the invention described herein. Such equivalents are intended to be encompassed by the following claims.

All publications, patents and patent applications mentioned in this specification are herein incorporated by reference into the specification to the same extent as if each individual publication, patent or patent application was specifically and individually indicated to be incorporated herein by reference.

## Claims

1. A composition comprising a therapeutically effective amount of one or more microtubule stabilizing agents and a therapeutically effective amount of one or more heat shock protein (HSP)-inducing agents.

2. A composition comprising a therapeutically effective amount of one or more microtubule stabilizing agents and a therapeutically effective amount of one or more chemokine-receptor inducing agents.

3. A composition comprising a therapeutically effective amount of one or more microtubule stabilizing agents and a therapeutically effective amount of one or more ICAM-inducing agents.

4. The composition of claim 1, 2 or 3, wherein at least one of the microtubule stabilizing agents is a taxane, an epothilone, a discodermolide, an eleutherobin, a taccalonolide, a sarcodictyin, or a derivative or analog thereof.

5. The composition of claim 4, wherein the taxane is paclitaxel or doxotaxel.

6. The composition of claim 1, wherein at least one of the HSP-inducing agents is prostaglandin J2, geranyl-geranyl-acetone, 5-fluorouracil, cyclosporine A, sodium butyrate, aspirin, herbimycin A, arsenite, arsenic trioxide or geldanamycin.

7. The composition of claim 1, wherein at least one of the HSP-inducing agents induces or increases the expression of HSP60, HSP70, HSP72, HSP80 or HSP90.

8. The composition of claim 2, wherein at least one of the chemokine receptor-inducing agents is lymphotoxin-α, CpG7909, CpG8916 or CpG8954.

9. The composition of claim 3, wherein at least one of the ICAM-inducing agents is tributyrin, OK-432, retionic acid, sodium buytrate or lymphotoxin-α.

10. The composition of claim 3, wherein at least one of the ICAM-inducing agents induces or increases the expression of ICAM-1.

11. A method of treating a hyperproliferative disorder or one or more symptoms thereof comprising administering to a subject in need in thereof a dose of a therapeutically effective amount of the composition of claim 1, 2 or 3.

12. A method of treating an infectious disease, an autoimmune disorder or an inflammatory disorder, or ameliorating one or more symptoms thereof, said method comprising administering to a subject in need thereof a dose of a therapeutically effective amount of the composition of claim 1, 2 or 3.

13. A method of treating a proliferative disorder or ameliorating one or more symptoms thereof, said method comprising administering to a subject in need thereof a dose of a therapeutically effective amount of one or more microtubule stabilizing agents and a dose of a therapeutically effective amount of one or more heat shock protein (HSP)-inducing agents, wherein said HSP-inducing agents are not 5-fluorouracil, cyclosporine A, aspirin, glutamine, or herbimycin A.

14. A method of treating a proliferative disorder or ameliorating one or more symptoms thereof, said method comprising administering to a subject in need thereof a dose of a therapeutically effective amount of one or more microtubule stabilizing agents and a dose of a therapeutically effective amount of one or more chemokine receptor-inducing agents.

15. A method of treating a proliferative disorder or ameliorating one or more symptoms thereof, said method comprising administering to a subject in need thereof a dose of a therapeutically effective amount of one or more microtubule stabilizing agents and a dose of a therapeutically effective amount of one or more ICAM-inducing agents.

16. A method of treating an infectious disease, an autoimmune disorder or an inflammatory disorder, or ameliorating one or more symptoms thereof, said method comprising administering to a subject in need thereof a dose of a therapeutically effective amount of one or more microtubule stabilizing agents and a dose of a therapeutically effective amount of one or more heat shock protein (HSP)-inducing agents, one or more chemokine receptor-inducing agents, or one or more ICAM-inducing agents.

17. The method of claim 13, 14, 15 or 16, wherein at least one of the microtubule stabilizing agents is a taxane, an epothilone, a discodermolide, an eleutherobin, a taccalonolide, a sarcodictyin, or a derivative or analog thereof.

18. The method of claim 17, wherein the taxane is paclitaxel or doxotaxel.

19. The method of claim 13, wherein at least one of the HSP-inducing agents is prostaglandin J2, geranyl-geranyl-acetone, sodium butyrate, herbimycin A, arsenite, arsenic trioxide or geldanamycin.

20. The method of claim 16, wherein at least one of the HSP-inducing agents is prostaglandin J2, geranyl-geranyl-acetone, 5-fluorouracil, cyclosporine A, sodium butyrate, aspirin, herbimycin A, arsenite, arsenic trioxide or geldanamycin.

21. The method of claim 13 or 16, wherein at least one of the HSP-inducing agents induces or increases the expression of HSP60, HSP70, HSP72, HSP80 or HSP90.

22. The method of claim 14 or 16, wherein at least one of the chemokine receptor-inducing agents is lymphotoxin-α, CpG7909, CpG8916 or CpG8954.

23. The method of claim 15 or 16, wherein at least one of the ICAM-inducing agents is tributyrin, OK-432, retionic acid, sodium buytrate or lymphotoxin-α.

24. The method of claim 15 or 16, wherein at least one of the ICAM-inducing agents induces or increases the expression of ICAM-1.

25. The method of claim 13 further comprising administering to said subject a dose of a therapeutically effective amount of one or more immunomodulatory agents other than HSP-inducing agents.

26. The method of claim 25, wherein at least one of the immunomodulatory agents is a chemokine receptor-inducing agent or ICAM-inducing agent.

27. The method of claim 14 further comprising administering to said subject a dose of a therapeutically effective amount of one or more immunomodulatory agents other than chemokine receptor-inducing agents.

28. The method of claim 27, wherein at least one of the immunomodulatory agents is a HSP-inducing agent or ICAM-inducing agent.

29. The method of claim 15 further comprising administering to said subject a dose of a therapeutically effective amount of one or more immunomodulatory agents other than ICAM-inducing agents.

30. The method of claim 29, wherein at least one of the immunomodulatory agents is a HSP-inducing agent or chemokine receptor-inducing agent.

31. The method of claim 13 further comprising administering to said subject a dose of a therapeutically effective amount of one or more ICAM-inducing agents, wherein said ICAM-inducing agents are different than said HSP-inducing agents.

32. The method of claim 14 further comprising administering to said subject a dose of a therapeutically effective amount of one or more ICAM-inducing agents.

33. The method of claim 13, 14 or 15 further comprising administering to said subject radiation therapy.

34. The method of claim 13, 14, 15 or 16, wherein the therapeutically effective amount of the microtubule stabilizing agent ranges from about 0.000001 g/m² to 10 g/m².

35. The method of claim 13, wherein the therapeutically effective amount of the HSP-inducing agent ranges from about 0.000001 g/m² to 10 g/m².

36. The method of claim 14, wherein the therapeutically effective amount of the chemokine receptor-inducing agent ranges from about 0.000001 g/m² to 1 g/m².

37. The method of claim 15, wherein the therapeutically effective amount of the ICAM-inducing agent ranges from about 0.000001 g/m² to 1 g/m².

38. The method of claim 13, 14 or 15, wherein the dose of the therapeutically effective amount of one or more microtubule stabilizing agents is administered intravenously, intramuscularly, subcutaneously, intraperitoneally, orally or intratumorally.

39. The method of claim 16, wherein the dose of the therapeutically effective amount of one or more microtubule stabilizing agents is administered intravenously, intramuscularly, subcutaneously, intraperitoneally or orally.

40. The method of claim 13, wherein the dose of the therapeutically effective amount of one or more HSP-inducing agents is administered intravenously, intramuscularly, subcutaneously, intraperitoneally, orally or intratumorally.

41. The method of claim 16, wherein the dose of the therapeutically effective amount of one or more HSP-inducing agents is administered intravenously, intramuscularly, subcutaneously, intraperitoneally or orally.

42. The method of claim 14, wherein the dose of the therapeutically effective amount of one or more chemokine receptor-inducing agents is administered intravenously, intramuscularly, subcutaneously, intraperitoneally, orally or intratumorally.

43. The method of claim 16, wherein the dose of the therapeutically effective amount of one or more chemokine receptor-inducing agents is administered intravenously, intramuscularly, subcutaneously, intraperitoneally or orally.

44. The method of claim 15, wherein the dose of the therapeutically effective amount of one or more ICAM-inducing agents is administered intravenously, intramuscularly, subcutaneously, intraperitoneally, orally or intratumorally.

45. The method of claim 16, wherein the dose of the therapeutically effective amount of one or more ICAM-inducing agents is administered intravenously, intramuscularly, subcutaneously, intraperitoneally or orally.

46. The method of claim 13, 14 or 15, wherein the hyperproliferative disorder is cancer or psorasis.

47. The method of claim 46, wherein the cancer is leukemia, fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, or retinoblastoma.

48. The method of claim 16, wherein the infectious disease is a viral infectious disease, bacterial infectious disease or a fungal infectious disease.

49. The method of claim 13, 14, 15 or 16, wherein said subject is a human.
